# EUROPEAN PATENT APPLICATION

(11) **EP 3 954 765 A1**
(43) Date of publication of application: **16.02.2022**
(21) Application number: 19821431.4
(22) Date of filing: 12.11.2019
(51) Int. Cl.: C12N 5/16

(54) **ARTIFICIAL RECOMBINANT CHROMOSOME AND USE THEREOF**

(30) Priority: 12.04.2019 US 201962833489 P
(71) Applicant: HUMAB CO. LTD., Seongdong-gu Seoul 04782 (KR)
(72) Inventor: OH, Chang Kyu, Jongno-gu Seoul 03012 (KR); PARK, Soon Ik, Yongsan-gu, Seoul 04385 (KR); KANG, Ho Jin, Uiwang-si Gyeonggi-do 16039 (KR); CHOI, Ae Jin, Namyangju-si Gyeonggi-do 12181 (KR)
(74) Representative: dompatent von Kreisler Selting Werner - Partnerschaft von Patent- und Rechtsanwälten mbB
(86) International application number: PCT/KR2019/015351
(87) International publication number: WO 2020/209458

(57) **Abstract**

The disclosure in the specification relates to an artificial recombinant chromosome and a use thereof, and more particularly to an artificial recombinant chromosome produced by recombination of two or more chromosomes and the production of a transgenic animal using a cell including the same.

## Description

### [Technical Field]

The disclosure in the specification relates to an artificial recombinant chromosome and the use thereof, and more particularly to an artificial recombinant chromosome generated by the recombination of two or more chromosomes and a production of a transgenic animal using a cell including the same.

### [Background Art]

Transgenic animals may contribute to genetic engineering development by expressing of a DNA encoding an exogenous protein or inactivating of an endogenous gene.

To produce a transgenic animal, generally, DNA encoding an exogenous protein is inserted into the genome of an animal cell, and an animal is produced using a cell generated thereby. Here, to insert DNA encoding an exogenous protein into the genome of an animal cell, a vector containing DNA encoding an exogenous protein is used, and to produce the vector, DNA encoding an exogenous protein is cloned.

### [Disclosure]

### [Technical Problem]

To produce a transgenic animal, generally, DNA encoding an exogenous protein is inserted into the genome of an animal cell, and an animal is produced using a cell generated thereby. Here, to insert DNA encoding an exogenous protein into the genome of an animal cell, a vector containing DNA encoding an exogenous protein is used, and to produce the vector, DNA encoding an exogenous protein is cloned. This method uses one or two or more vectors according to the size of DNA encoding an exogenous protein. For example, when the size of DNA encoding an exogenous protein is several tens of kilobases or more, the DNA encoding an exogenous protein is fragmented and then inserted using multiple vectors. When the DNA is inserted using a plurality of vectors, instead of one vector, there is a problem in which the yield efficiency of cells into which the full-length DNA encoding an exogenous protein is inserted is reduced.

To solve the above-mentioned problem, the present invention is directed to providing a method of effectively inserting a gene to be inserted into the genome of an animal cell regardless of the size of the gene to be inserted.

The present invention is also directed to providing an artificial recombinant chromosome and a method of producing the same.

The present invention is also directed to providing a cell including an artificial recombinant chromosome and a method of producing the same.

The present invention is also directed to providing a method of producing a transgenic animal using a cell including an artificial recombinant chromosome.

### [Technical Solution]

To solve the technical problems, one aspect of the disclosure in the specification provides a method of inserting a full-length gene (a coding region, a non-coding region, etc.) to be inserted into the genome of an animal cell without separate cloning. Another aspect of the disclosure in the specification provides a method of producing a transgenic animal using the transgenic animal cell generated as described above.

According to an aspect of the disclosure in the specification, the present invention provides a method of producing a cell including one or more artificial recombinant chromosomes.

In one embodiment, a method of producing a cell including one or more artificial recombinant chromosomes may comprise:
i) preparing a first targeted cell and a second targeted cell;
ii) producing one or more microcells using the second targeted cell;
iii) producing a fusion cell using the first targeted cell and the one or more microcells; and
iv) producing a cell including an artificial recombinant chromosome by treating the fusion cell with a site specific recombinase (SSR).

The first targeted cell may comprise a first targeted chromosome.

The second targeted cell may comprise a second targeted chromosome.

Here, the first targeted chromosome may include a first part, a first recombinase recognition sequence (a first RRS) and a first fragment. The first RRS may be located between the first part and the first fragment.

Here, the second targeted chromosome may include a second part, a second recombinase recognition sequence (a second RRS) and a second fragment. The second RRS may be located between the second part and the second fragment.

The one or more microcells may comprise the second targeted chromosome or a fragment thereof.

Here, the fragment of the second targeted chromosome may include the second RRS and the second fragment.

The fusion cell may comprise the first targeted chromosome and the second targeted chromosome, or the first targeted chromosome and the fragment of the second targeted chromosome.

The SSR may induce a recombination by recognizing a pairing of the first RRS present in the first targeted chromosome and the second RRS present in the second targeted chromosome.

Here, the first fragment present in the first targeted chromosome may be exchanged for the second fragment present in the second targeted chromosome by the recombination

Thereby, a first artificial recombinant chromosome with the first part and the second fragment may be produced.

Here, the cell including the first artificial recombinant chromosome may not include the second targeted chromosome.

The cell including one or more artificial recombinant chromosomes may further comprise a second artificial recombinant chromosome. Here, the second artificial recombinant chromosome may include the second part and the first fragment.

The first part may include one of telomere ends located on both sides of the first targeted chromosome. Here, the first fragment may include the other one of telomere ends located on both sides of the first targeted chromosome.

The second part may include one of telomere ends located on both sides of the second targeted chromosome. Here, the second fragment may include the other one of telomere ends located on both sides of the second targeted chromosome.

The first RRS may be one selected from a loxP and a loxP variant, and the second RRS may be one selected from a loxP and a loxP variant. Here, the first RRS may be capable of pairing with the second RRS. Here, the SSR may be a Cre recombinase, and the SSR may be capable of recognizing the first RRS and the second RRS.

Alternatively, the first RRS may be one selected from FRT, attP, attB, ITR and variants thereof, and the second RRS may be one selected from FRT, attP, attB, ITR and variants thereof. Here, the first RRS may be capable of pairing with the second RRS. Here, the SSR may be one selected from a flippase (FLP), an integrase and a transposase, and the SSR may be capable of recognizing the first RRS and the second RRS.

The cell including one or more artificial recombinant chromosomes may undergo somatic cell division (mitosis) or meiosis.

In another embodiment, a method for producing a cell including one or more artificial recombinant chromosomes may comprise:
i) preparing a first targeted cell and a second targeted cell;
ii) producing one or more microcells using the second targeted cell;
iii) producing a fusion cell using the first targeted cell and the one or more microcells; and
iv) producing a cell including an artificial recombinant chromosome by treating the fusion cell with site specific recombinase (SSR).

The first targeted cell may comprise a first targeted chromosome.

Here, the first targeted chromosome may include a first part, a first recombinase recognition sequence (a first RRS), a first fragment, a second recombinase recognition sequence (a second RRS) and a second part.

Here, the first part may include one of telomere ends located on both sides of the first targeted chromosome, and the second part may include the other one of telomere ends located on both sides of the first targeted chromosome.

Here, the first fragment may be located between the first RRS and the second RRS

The second targeted cell may comprise a second targeted chromosome.

Here, the second targeted chromosome may include a third part, a third recombinase recognition sequence (a third RRS), a second fragment, a fourth recombinase recognition sequence (a fourth RRS) and a fourth part.

Here, the third part may include one of telomere ends located on both sides of the second targeted chromosome, and the fourth part may include the other one of telomere ends located on both sides of the second targeted chromosome.

Here, the second fragment may be located between the third RRS and the fourth RRS.

The one or more microcells may comprise the second targeted chromosome or a fragment thereof.

Here, the fragment of the second targeted chromosome may include the third RRS, the second fragment and the fourth RRS

The fusion cell may comprise the first targeted chromosome and the second targeted chromosome, or the first targeted chromosome and the fragment of the second targeted chromosome.

The SSR may induce a recombination by recognizing a pairing of the first RRS present in the first targeted chromosome and the third RRS present in the second targeted chromosome, and a pairing of the second RRS present in the first targeted chromosome and the fourth RRS present in the second targeted chromosome.

Here, the first fragment present in the first targeted chromosome may be exchanged for the second fragment present in the second targeted chromosome by the recombination.

Thereby, a first artificial recombinant chromosome including the first part, the second fragment and the second part may be produced.

Here, the cell including the first artificial recombinant chromosome may not include the second targeted chromosome.

The cell including one or more artificial recombinant chromosomes may further comprise a second artificial recombinant chromosome. Here, the second artificial recombinant chromosome may include the third part, the first fragment and the fourth part.

The first part may include a centromere of the first targeted chromosome.

Alternatively, the first fragment may include a centromere of the first targeted chromosome.

The third part may include a centromere of the second targeted chromosome. The second fragment may include a centromere of the second targeted chromosome.

The first RRS may be one selected from a loxP and a loxP variant, and the third RRS may be one selected from a loxP and a loxP variant. Here, the first RRS may be capable of pairing with the third RRS.

The second RRS may be one selected from a loxP and a loxP variant, and the fourth RRS may be one selected from a loxP and a loxP variant. Here, the second RRS may be capable of pairing with the fourth RRS.

Here, the SSR may be a Cre recombinase.

Alternatively, the first RRS may be one selected from FRT, attP, attB, ITR and variants thereof, and the third RRS may be one selected from FRT, attP, attB, ITR and variants thereof. Here, the first RRS may be capable of pairing with the third RRS.

The second RRS may be one selected from FRT, attP, attB, ITR and variants thereof, and the fourth RRS may be one selected from FRT, attP, attB, ITR and variants thereof. Here, the second RRS may be capable of pairing with the fourth RRS.

Here, the SSR may be one selected from a flippase (FLP), an integrase and a transposase.

The cell including one or more artificial recombinant chromosome may undergo somatic cell division (mitosis) or meiosis.

According to another aspect of the disclosure in the specification, the present invention provides a method of making a transgenic non-human animal using a cell including one or more artificial recombinant chromosomes.

In one embodiment, a method for making a transgenic non-human animal using a cell including one or more artificial recombinant chromosome may comprise:
i) preparing a first targeted cell and a second targeted cell;
ii) producing one or more microcells using the second targeted cell;
iii) producing a fusion cell using the first targeted cell and the one or more microcells;
iv) producing a cell including an artificial recombinant chromosome by treating the fusion cell with a site specific recombinase (SSR); and
v) implanting a chimeric blastocyst comprising the first artificial recombinant chromosome in a surrogate mother's uterus to produce an offspring.

The first targeted cell may be an embryonic stem cell.

The first targeted cell may comprise a first targeted chromosome. The second targeted cell may comprise a second targeted chromosome.

Here, the first targeted chromosome may include a first part, a first recombinase recognition sequence (a first RRS) and a first fragment. The first RRS may be located between the first part and the first fragment.

Here, the second targeted chromosome may include a second part, a second recombinase recognition sequence (a second RRS) and a second fragment. The second RRS may be located between the second part and the second fragment.

The one or more microcells may comprise the second targeted chromosome or a fragment thereof. Here, the fragment of the second targeted chromosome may include the second RRS and the second fragment.

The fusion cell may comprise the first targeted chromosome and the second targeted chromosome, or the first targeted chromosome and the fragment of the second targeted chromosome.

The SSR may induce a recombination by recognizing a pairing of the first RRS present in the first targeted chromosome and the second RRS present in the second targeted chromosome.

Here, the first fragment present in the first targeted chromosome may be exchanged for the second fragment present in the second targeted chromosome.

Thereby, a first artificial recombinant chromosome including the first part and the second fragment may be produced.

Here, the cell including the first artificial recombinant chromosome may not include the second targeted chromosome.

The chimeric blastocyst may be produced by injecting the cell including the first artificial chromosome into a blastocyst.

In another embodiment, a method for making a transgenic non-human animal using a cell including one or more artificial recombinant chromosome may comprise:
i) preparing a first targeted cell and a second targeted cell;
ii) producing one or more microcells using the second targeted cell;
iii) producing a fusion cell using the first targeted cell and the one or more microcells;
iv) producing a cell including an artificial recombinant chromosome by treating the fusion cell with a site specific recombinase (SSR); and
v) implanting a chimeric blastocyst comprising the first artificial recombinant chromosome in a surrogate mother's uterus to produce an offspring.

The first targeted cell may be an embryonic stem cell.

The first targeted cell may comprise a first targeted chromosome. The second targeted cell may comprise a second targeted chromosome.

The first targeted chromosome may include a first part, a first recombinase recognition sequence (a first RRS), a first fragment, a second recombinase recognition sequence (a second RRS) and a second part.

Here, the first part may include one of telomere ends located on both sides of the first targeted chromosome, and the second part may include the other one of telomere ends located on both sides of the first targeted chromosome.

Here, the first fragment may be located between the first RRS and the second RRS.

The second targeted chromosome may include a third part, a third recombinase recognition sequence (a third RRS), a second fragment, a fourth recombinase recognition sequence (a fourth RRS) and a fourth part.

Here, the third part may include one of telomere ends located on both sides of the second targeted chromosome, and the fourth part may include the other one of the telomere ends located on both sides of the second targeted chromosome.

Here, the second fragment may be located between the third RRS and the fourth RRS.

The one or more microcells comprises the second targeted chromosome or a fragment thereof. Here, the fragment of the second targeted chromosome may include the third RRS, the second fragment and the fourth RRS.

The fusion cell may comprise the first targeted chromosome and the second targeted chromosome, or the first targeted chromosome and the fragment of the second targeted chromosome.

The SSR may induce a recombination by recognizing a pairing of the first RRS present in the first targeted chromosome and the third RRS present in the second targeted chromosome, and a pairing of the second RRS present in the first targeted chromosome and the fourth RRS present in the second targeted chromosome.

Here, the first fragment present in the first targeted chromosome may be exchanged for the second fragment present in the second targeted chromosome.

Thereby, a first artificial recombinant chromosome including the first part, the second fragment and the second part may be produced.

Here, the cell including the first artificial recombinant chromosome may not include the second targeted chromosome.

The chimeric blastocyst may be produced by injecting the cell including the first artificial chromosome into a blastocyst.

### [Advantageous Effects]

According to the technology disclosed by the specification, the following effects are exhibited.

First, an artificial recombinant chromosome and a method of producing the same can be provided. Further, an artificial recombinant chromosome containing a larger exogenous DNA segment can be provided.

Second, a cell including an artificial recombinant chromosome and a method of producing the same can be provided. Further, a cell including an artificial recombinant chromosome and a method of preparing the same can be provided by providing a larger exogenous DNA segment to a target chromosome.

Third, a method of producing a transgenic animal using a cell including an artificial recombinant chromosome can be provided. Further, a method of making a transgenic animal using a cell including an artificial recombinant chromosome can be provided by providing a larger exogenous DNA segment to a target chromosome.

### [Description of Drawings]

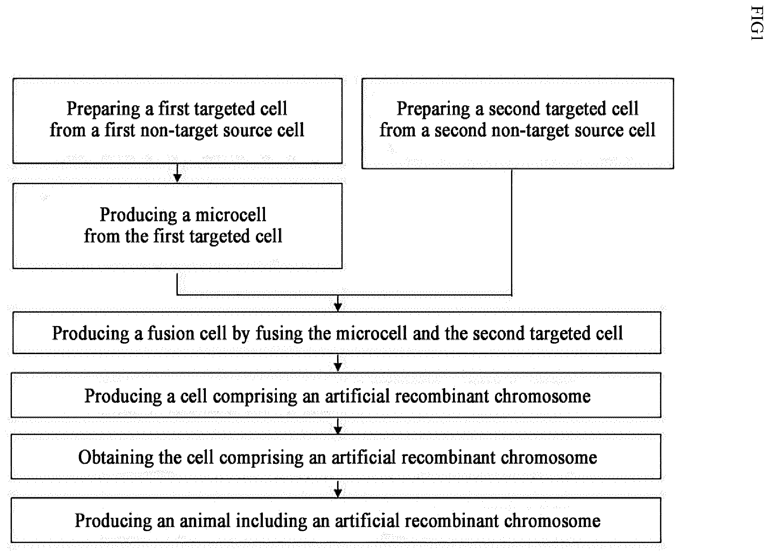

FIG. 1 is a flow chart according to an exemplary embodiment.
FIGS. 2 to 10 are schematic diagrams illustrating the production of an artificial recombinant chromosome from a targeted chromosome, respectively.

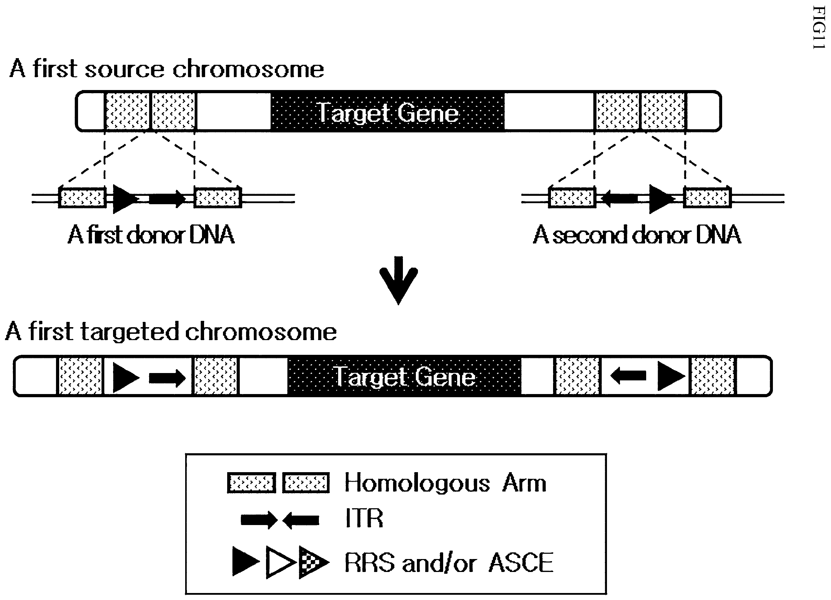

FIG. 11 is a schematic diagram illustrating the production of a first targeted chromosome by providing a first donor DNA and a second donor DNA to a first non-target source chromosome.

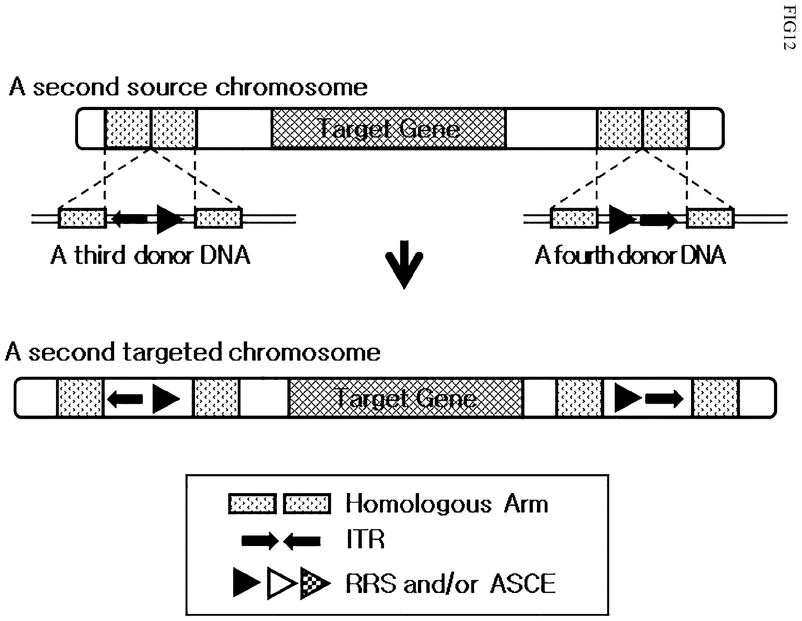

FIG. 12 is a schematic diagram illustrating the production of a second targeted chromosome by providing a third donor DNA and a fourth donor DNA to a second non-target source chromosome.
FIG. 13 is a schematic diagram illustrating the production of a first artificial recombinant chromosome and a second artificial recombinant chromosome from a first targeted chromosome and a second targeted chromosome.
FIG. 14 is a schematic diagram illustrating the production of a final artificial recombinant chromosome from a first artificial recombinant chromosome.
FIG. 15 is a schematic diagram illustrating the production of a first targeted chromosome by providing a first donor DNA and a second donor DNA to a first non-target source chromosome.
FIG. 16 is a schematic diagram illustrating the inversion of a target gene of a first targeted chromosome.
FIG. 17 is a schematic diagram illustrating the production of a second targeted chromosome by providing a third donor DNA and a fourth donor DNA to a second non-target source chromosome.
FIG. 18 is a schematic diagram illustrating the inversion of a target gene of a second targeted chromosome.
FIG. 19 is a schematic diagram illustrating the production of a first artificial recombinant chromosome and a second artificial recombinant chromosome from a first targeted chromosome and a second targeted chromosome.
FIG. 20 is a schematic diagram illustrating the production of a final artificial recombinant chromosome from a first artificial recombinant chromosome.
FIGS. 21 to 24 are schematic diagrams for a DNA structure of a targeted chromosome according to an exemplary embodiment, respectively.
FIGS. 25 and 26 illustrate the results of selecting a targeted cell according to an exemplary embodiment, respectively.
FIG. 27 illustrates a microcell according to an exemplary embodiment.
FIG. 28 illustrates the process of preparing a fusion cell according to an exemplary embodiment.
FIG. 29 illustrates a fusion cell including a targeted chromosome according to an exemplary embodiment.
FIG. 30 illustrates a fusion cell including an artificial recombinant chromosome according to an exemplary embodiment.
FIGS. 31 to 33 illustrate the comparison of a fusion cell including an artificial recombinant chromosome with a fusion cell including a targeted chromosome according to an exemplary embodiment.
FIGS. 34 and 35 illustrate the results of selecting a fusion cell including an artificial recombinant chromosome and confirming an artificial recombinant chromosome according to an exemplary embodiment.

### [Modes of the Invention]

Unless defined otherwise, all technical and scientific terms used in the specification have the same meanings as commonly understood by one of ordinary skill in the art to which the present invention belongs. Although methods and materials similar or equivalent to those described in the specification can be used in the practice or experiments of the present invention, suitable methods and materials are described below. All publications, patent applications, patents and other references mentioned in the specification are incorporated by reference in their entirety. In addition, the materials, methods and examples are merely illustrative and not intended to be limited.

Hereinafter, the present invention will be described.

### The disclosure in the specification relates to production of an artificial recombinant chromosome and cell including the same.

A transgenic animal is an animal into which an artificial trait is introduced, and is used for the study of various diseases and mechanisms and the development of a therapeutic agent. To produce a transgenic animal, a method of producing a transgenic animal includes a process of introducing a desired trait into an animal cell. To this end, currently, a method using a cloning vector is used.

The method using a cloning vector is a method of cloning a desired trait, that is, a target gene, which is desired to be expressed, in a transgenic animal and delivering an artificially produced vector to an animal cell to insert the gene into the genome. Such a method uses a plasmid, a bacterial artificial chromosome (BAC) or a yeast artificial chromosome (YAC). The BAC or YAC, compared to a plasmid, is a DNA construct that can carry a larger fragment (150~350 kbp), and is widely used for transformation. Particularly, due to the advantage in that the BAC or YAC, compared to a plasmid, can carry a relatively larger fragment, it is used for transduction of a large target gene.

However, for a large target gene, a plurality of BACs or YACs are needed. For example, when a mouse producing a human antibody is produced, to produce the mouse, a mouse cell into which a human immunoglobulin (Ig) gene is introduced needs to be produced. To this end, it is necessary to produce a transformation vector cloning a human immunoglobulin heavy (IGH) gene with a size of 1250 kilobases (kb). When the transformation vector is a BAC, at least 4 to 9 BACs respectively having different DNA fragments are made. The BACs produced thereby are sequentially introduced into a mouse cell to be inserted into the genome. That is, a first BAC is introduced into a mouse cell to be inserted into the genome and the first BAC-inserted mouse cell is selected. A second BAC is introduced into the selected mouse cell to be inserted into the genome, and again the second BAC-inserted mouse cell is selected. To select such a mouse cell in which a target gene, that is, the full-length human IGH gene (total DNA), is inserted into the genome, the above-described process needs to be repeated. Such a repeated process is a factor for reducing a yield of mouse cells into which a full-length target gene is inserted. In addition, problems such as time consumption and cost consumption caused by the repetition of the introduction of a trait and selection occur. Moreover, the time and cost of creating multiple BACs are significant.

To solve these problems, the present invention developed transformation technology using recombination between chromosomes.

The disclosure in the specification shows that conventional systems using transformation vectors such as a BAC and a YAC can be replaced by producing an artificial recombinant chromosome through recombination between chromosomes.

The method disclosed in the specification describes a transduction (transformation) method using a chromosome, rather than a BAC or YAC. The transformation method using a chromosome, instead of a BAC or YAC that has been used in the conventional method, is to insert a target gene into the genome of an animal through introduction of one chromosome into an animal cell and recombination, and create a transformed (transduced) animal cell.

The transformation method using a chromosome disclosed in the specification may be largely divided into three steps.

A first step is to artificially manipulate a chromosome containing a target gene, that is, a gene for transduction (transformation) and a chromosome into which the target gene will be inserted in order to include an element required for recombination. This process may be performed in a donor cell having a chromosome containing a target gene and a recipient cell having a chromosome into which a target gene will be inserted. An element required for recombination may be a factor that enables recombination using a recombinase or homologous recombination. For example, when a recombinase is used, a site recognized by the recombinase may be considered an element required for recombination. In one example, when a Cre recombinase is used, an element required for recombination may be a loxP. In another example, when a flippase (FLP) recombinase is used, an element required for recombination may be an FRT. The purpose of this process is to provide a site that can be recognized by a recombinase or a homologous site for homologous recombination in recombination between chromosomes. The process should be designed in consideration of the positions and pairing of an element required for recombination, which is included in a chromosome containing a target gene, and an element required for recombination, which is included in a chromosome into which a target gene will be inserted. The positions may be highly associated with an insertion position of the target gene, and the pairing may determine the success of recombination and the type of recombination. Through the above-described process, a cell (donor cell) having a chromosome containing a target gene into which an element required for recombination is inserted and a cell (recipient cell) having a chromosome into which an element required for recombination is inserted are produced. Here, an element required for recombination, which is included in a chromosome of the donor cell, is paired with an element required for recombination, which is included in a chromosome of the recipient cell.

A second step is for production of a microcell and cell fusion using the same. This process uses the cell (donor cell) produced in the previous process, and the microcell produced by this process has the chromosome containing a target gene into which an element required for recombination is inserted. Alternatively, the microcell produced by this process has a fragment of the chromosome containing a target gene into which an element required for recombination is inserted, wherein the fragment includes the target gene into which an element required for recombination is inserted. This process may be performed using Microcell-Mediated Chromosome Transfer (MMCT), which is conventionally known in the art. MMCT is a technology generally used to transfer the chromosome from the donor cell to the recipient cell (Thorfinn Ege et al., 1974; Thorfinn Ege et al., 1977). The microcell produced through the process includes a chromosome or a chromosomal fragment, which is not a cloning vector such as a plasmid replicated by artificial cloning. In addition, the chromosome or chromosome fragment includes an element required for recombination, which is paired with an element required for recombination included in the recipient cell. The produced microcell is fused with the recipient cell. Through this process, the chromosome of the donor cell, containing a target gene into which the element required for recombination is inserted, is introduced (transferred) into the recipient cell through the fusion of a microcell.

A third step is to produce a cell having an artificial recombinant chromosome using a recombinase or homologous recombination. This process is to induce recombination between chromosomes by treating a cell produced in the previous process, that is, a fusion cell produced through cell fusion with a recombinase or a factor induced by a homologous recombination. In this process, when a recombinase is treated, recombination between chromosomes having a site recognized by the recombinase, that is, the element required for recombination, is induced. In other words, recombination between the chromosome having a target gene containing an element required for recombination and the chromosome into which a target gene will be inserted, having an element required for recombination is induced. As a result, a novel artificial recombinant chromosome is generated by translocation of the target gene due to the recombination between the two chromosomes. Here, the generated artificial recombinant chromosome is a chromosome in which a target gene is inserted into a chromosome into which a desired trait (target gene) will be inserted. In other words, the generated artificial recombinant chromosome is a chromosome generated by inserting a part of the chromosome of the donor cell (i.e., the target gene) into the chromosome of the recipient cell (i.e., the chromosome into which the target gene will be inserted) through recombination between the chromosomes. A transgenic animal may be produced using a cell having the artificial recombinant chromosome.

As described using the above-described examples, when a mouse producing a human antibody is produced, a human IGH gene with a size of 1250 kb should be introduced into a mouse cell. When using a transformation method using the chromosome disclosed in the specification, a chromosome containing a human IGH gene, that is, human chromosome 14, is introduced into a mouse cell. Here, the chromosome containing the human IGH gene is a chromosome artificially manipulated to include an element required for recombination at both ends of a target gene, that is, a human IGH gene. To introduce or deliver the human chromosome 14 into a mouse cell, Microcell-Mediated Chromosome Transfer (MMCT) may be used. A fusion cell in which a microcell and a mouse cell are fused is produced by MMCT, and includes whole chromosomes of a mouse cell and the human chromosome 14. Recombination between the introduced human chromosome 14 and the chromosome into which a target gene is desired to be inserted (e.g., mouse chromosome 12 containing a mouse IGH gene) is induced by treatment the fusion cell with a recombinant enzyme. Here, the chromosome into which a target gene is desired to be inserted (e.g., mouse chromosome 12 containing a mouse IGH gene) is a chromosome artificially manipulated to include an element required for recombination at a locus into which a target gene is desired to be inserted (e.g., both termini of the mouse IGH gene) like the human chromosome 14. Through the recombination induction process, the human IGH gene of the human chromosome 14 is inserted into or replaced with a locus into which the target gene is desired to be inserted (e.g., the mouse IGH locus). For insertion, the human IGH gene may be inserted upstream or downstream of the mouse IGH gene locus. For replacement, the mouse IGH gene located at the mouse IGH locus may be replaced with a human IGH gene. Recombination (insertion or replacement) may vary according to the design of an element required for recombination. The embodiment described above is merely an example, and a target gene can be selectively modified and diversified.

The transformation method using a chromosome, which is disclosed in the specification, uses a chromosome present in a cell without an artificial cloning step, and has a technical difference from a conventional system using transformation vectors such as a BAC and a YAC. In addition, the transformation method using a chromosome, which is disclosed in the specification, is a novel technology that can solve problems (efficiency, time, cost, etc.) of the conventional art by significantly reducing the number of sequential introductions using transformation vectors such as a BAC and a YAC, when a target gene, particularly, a large target gene is introduced.

According to the transformation method using a chromosome, which is disclosed in the specification, a gene to be introduced, that is, the total sequence of the target gene is not cloned. To introduce one target gene into a genome, according to the transformation method using a chromosome, which is disclosed in the specification, following cell fusion, the fusion cell is treated with a recombinase (or a factor induced by homologous recombination) for recombination of chromosomes and not additionally fused with a microcell. However, when there are two or more target genes, and each target gene is located on a different chromosome, the transformation method using a chromosome is carried out by introduction of each target gene into the genome, and at this time, a step of fusing a microcell to introduce a second target gene into a cell into which a first target gene generated by the transformation method using a chromosome is introduced is further performed.

Hereinafter, the transformaiton method using a chromosome, which is disclosed in the specification, that is, a method of producing an artificial recombinant chromosome, will be described in detail.

### One aspect of the disclosure in the specification relates to an artificial recombinant chromosome.

The "artificial recombinant chromosome" refers to a chromosome in which two or more chromosomes provided from two or more source cells are partially recombined. In addition, the artificial recombinant chromosome also includes a chromosome generated by replication of the chromosome in which two or more chromosomes provided from two or more source cells are partially recombined. In one example, the artificial recombinant chromosome may be a chromosome in which a chromosome provided from a first source cell and a chromosome provided from a second source cell are partially recombined. Here, the chromosome provided from the first source cell may be a first source chromosome, and the first source chromosome may be included in the first source cell. Here, the chromosome provided from the second source cell may be a second source chromosome, and the second source chromosome may be included in the second source cell.

A cell including at least one or more artificial recombinant chromosomes is referred to as a "recombinant cell." Here, the recombinant cell includes at least one or more artificial recombinant chromosomes and at least one or more source chromosomes.

The "source chromosome" refers to a chromosome provided to produce an artificial recombinant chromosome. The source chromosome includes both of a natural chromosome and an artificially manipulated chromosome. The natural chromosome is a naturally-occurring chromosome, which is an intact chromosome without any artificial modification. For example, a human nerve cell has 46 naturally-occurring chromosomes. The artificially manipulated chromosome refers to a chromosome produced by artificial modification of the natural chromosome. Here, the artificial modification includes deletion, insertion or substitution of one or more nucleotides constituting the natural chromosome, or a combination thereof. The artificially manipulated chromosome includes all of a targeted chromosome that will be described below, a chromosome generated in the process of producing the same, and chromosomes including an artificial modification, other than the purpose of producing the targeted chromosome. For example, other than the purpose of producing the targeted chromosome, a chromosome including an artificial modification may be a chromosome into which an exogenous nucleic acid encoding an exogenous protein for expression thereof is inserted.

The "source cell" refers to a cell including the source chromosome. The source cell may include both of a cell including a natural chromosome and a cell including an artificially manipulated chromosome. Here, the cell including an artificially manipulated chromosome includes all of a target cell including a targeted chromosome, a cell generated in the process of producing the same, and a cell including a chromosome with an artificial modification, other than the purpose of producing a targeted chromosome. In addition, a cell including a chromosome, other than an artificial recombinant chromosome, that is, a cell not including an artificial recombinant chromosome may also be referred to as a source cell in the present invention.

### Artificial recombinant chromosome

The artificial recombinant chromosome may be a chromosome produced by recombining a part of a chromosome sequence provided from the first source chromosome and the entire chromosome sequence provided from the second source chromosome.

The artificial recombinant chromosome may be a chromosome produced by recombining the entire chromosome sequence provided from a first source chromosome and a part of a chromosome sequence provided from a second source chromosome.

The artificial recombinant chromosome may be a chromosome produced by recombining the entire chromosome sequence provided from a first source chromosome and the entire chromosome sequence provided from a second source chromosome.

The artificial recombinant chromosome may be a chromosome produced by recombining a part of a chromosome sequence provided from a first source chromosome and a part of a chromosome sequence of a second source chromosome.

The first source chromosome may be included in a first source cell.

The second source chromosome may be included in a second source cell.

The first source chromosome may be derived from a first source cell.

The second source chromosome may be derived from a second source cell.

The first source chromosome is included in a first source cell, and the second source chromosome may be included in a second source cell. In this case, the first source cell and the second cell may be the same type of cells. For example, the first source cell and the second cell may be mouse fibroblasts, and present as individual cells. The first chromosome may be different from the second source chromosome. Alternatively, the first source chromosome and the second source chromosome may be homologous chromosomes.

The first source cell and the second source cell may be derived from the same individual.

The first source cell and the second source cell may be derived from different individuals. Here, the different individuals may include homologous and heterologous individuals.

The source cell may be derived from a human cell.

The source cell may be derived from a non-human cell. For example, the non-human cell may be derived from a mouse cell, a rat cell, a rodent cell, a goral cell, a cattle cell or an ungulate cell, but the present invention is not limited thereto.

The source cell may be derived from a somatic cell. For example, the somatic cell may be, for example, a fibroblast (fibroblast cell), but the present invention is not limited thereto.

The source cell may be derived from an immune cell. For example, the immune cell may be a B-cell, a T-cell, an NK cell, a macrophage, a neutrophil, a basophil or eosinophil, but the present invention is not limited thereto.

The source cell may be derived from a germ cell. For example, the germ cell may be a sperm, a spermatocyte, a spermatogonial stem cell, an egg, an oocyte, an oogonial stem cell or a fertilized egg, but the present invention is not limited thereto.

The source cell may be derived from a stem cell. For example, the stem cell may be derived from an embryonic stem cell (ES cell), an adult stem cell, an umbilical cord blood stem cell, a spermatogonial stem cell or an oogonial stem cell, but the present invention is not limited thereto.

In one exemplary embodiment, the artificial recombinant chromosome may include a first fragment and a second fragment.

The first fragment may be a part of the first source chromosome of the first source cell.

Here, the first fragment may include a first telomere. The first telomere may be one of both telomeres of the first source chromosome.

Here, the first source cell may be a human cell, a mouse cell, a rat cell, a rodent cell, a goral cell, a cattle cell or an ungulate cell.

The second fragment may be a part of the second source chromosome of the second source cell.

Here, the second fragment may include a centromere and a second telomere. The centromere may be a centromere of the second source chromosome. The second telomere may be one of both telomeres of the second source chromosome.

Here, the second source cell may be a human cell, a mouse cell, a rat cell, a rodent cell, a goral cell, a cattle cell or an ungulate cell.

Here, the second source cell and the first source cell may be derived from heterologous individuals. For example, when the first source cell is a human cell, the second source cell may be a mouse cell.

Alternatively, the second source cell and the first source cell may be derived from homologous individuals. For example, when the first source cell is a human cell, the second source cell may be a human cell.

The first fragment and the second fragment may be connected by a phosphodiester bond.

The artificial recombinant chromosome may have two telomeres derived from heterologous individuals.

The artificial recombinant chromosome may have two telomeres having different lengths.

Here, the artificial recombinant chromosome may not be the same as the first source chromosome, and the artificial recombinant chromosome may not be the same as the second source chromosome.

In another exemplary embodiment, the artificial recombinant chromosome may include a first fragment, a second fragment and a third fragment.

The first fragment may be a part of a first source chromosome of a first source cell.

Here, the first source cell may be a human cell, a mouse cell, a rat cell, a rodent cell, a goral cell, a cattle cell or an ungulate cell.

The second fragment may be a part of a second source chromosome of a second source cell.

Here, the second fragment may include a centromere and a first telomere. The centromere may be a centromere of the second source chromosome. The first telomere may be one of both telomeres of the second source chromosome.

The third fragment may be a part of the second source chromosome of the second source cell.

Here, the third fragment may include a second telomere. The second telomere may be one of both telomeres of the second source chromosome.

Here, the second source cell may be a human cell, a mouse cell, a rat cell, a rodent cell, a goral cell, a cattle cell or an ungulate cell.

Here, the second source cell and the first source cell may be derived from heterologous individuals. For example, when the first source cell is a human cell, the second source cell may be a mouse cell.

Alternatively, the second source cell and the first source cell may be derived from homologous individuals. For example, when the first source cell is a human cell, the second source cell may be a human cell.

The first fragment and the second fragment may be connected by a phosphodiester bond.

The first fragment and the third fragment may be connected by a phosphodiester bond.

The artificial recombinant chromosome may consist of the sequence of [second fragment]-[first fragment]-[third fragment].

Here, the first fragment may have an inverted form. Here, the inverted form may be the inversion of the first fragment present in the first source chromosome. In this case, in a cell including the artificial recombinant chromosome, a gene included in the first fragment may not be expressed as a protein. Alternatively, a cell including the artificial recombinant chromosome may have a different expression pattern of the gene included in the first fragment, compared to the first source cell including the first source chromosome.

The artificial recombinant chromosome may have both telomeres derived from the same individual.

Here, the artificial recombinant chromosome may not be the same as the first source chromosome, and the artificial recombinant chromosome may not be the same as the second source chromosome.

In still another exemplary embodiment, the artificial recombinant chromosome may include a first fragment, a second fragment and a third fragment.

The first fragment may be a part of a first source chromosome of a first source cell.

Here, the first fragment may include a centromere. The centromere may be a centromere of the first source chromosome.

Here, the first source cell may be a human cell, a mouse cell, a rat cell, a rodent cell, a goral cell, a cattle cell or an ungulate cell.

The second fragment may be a part of a second source chromosome of a second source cell.

Here, the second fragment may include a first telomere. The first telomere may be one of both telomeres of the second source chromosome.

The third fragment may be a part of the second source chromosome of the second source cell.

Here, the third fragment may include a second telomere. The second telomere may be one of both telomeres of the second source chromosome.

Here, the second source cell may be a human cell, a mouse cell, a rat cell, a rodent cell, a goral cell, a cattle cell or an ungulate cell.

Here, the second source cell and the first source cell may be derived from heterologous individuals. For example, when the first source cell is a human cell, the second source cell may be a mouse cell.

Alternatively, the second source cell and the first source cell may be derived from homologous individuals. For example, when the first source cell is a human cell, the second source cell may be a human cell.

The first fragment and the second fragment may be connected by a phosphodiester bond.

The first fragment and the third fragment may be connected by a phosphodiester bond.

The artificial recombinant chromosome may consist of the sequence of [second fragment]-[first fragment]-[third fragment].

Here, the first fragment may have an inverted form. Here, the inverted form may be the inversion of the first fragment present in the first source chromosome. In this case, in a cell including the artificial recombinant chromosome, a gene included in the first fragment may not be expressed as a protein. Alternatively, a cell including the artificial recombinant chromosome may have a different expression pattern of the gene included in the first fragment, compared to the first source cell including the first source chromosome.

The artificial recombinant chromosome may have both telomeres derived from the same individual.

Here, the artificial recombinant chromosome may not be the same as the first source chromosome, and the artificial recombinant chromosome may not be the same as the second source chromosome.

In yet another exemplary embodiment, the artificial recombinant chromosome may include a first fragment, a second fragment and a third fragment.

The first fragment may be a part of a first source chromosome of a first source cell.

Here, the first fragment may include a first telomere. The first telomere may be one of both telomeres of the first source chromosome.

Here, the first source cell may be a human cell, a mouse cell, a rat cell, a rodent cell, a goral cell, a cattle cell or an ungulate cell.

The second fragment may be a part of a second source chromosome of a second source cell.

Here, the second fragment may include a centromere. The centromere may be a centromere of the second source chromosome.

Here, the second source cell may be a human cell, a mouse cell, a rat cell, a rodent cell, a goral cell, a cattle cell or an ungulate cell.

The third fragment may be a part of a third source chromosome of a third source cell.

Here, the third fragment may include a second telomere. The second telomere may be one of both telomeres of the third source chromosome.

Here, the third source cell may be a human cell, a mouse cell, a rat cell, a rodent cell, a goral cell, a cattle cell or an ungulate cell.

Here, the third source cell may be derived from a heterologous individual with respect to the first source cell and the second source cell. For example, when the first source cell and the second source cell are human cells, the third source cell may be a mouse cell. Alternatively, for example, when the first source cell is a human cell, and the second source cell is a mouse cell, the third source cell may be a rat cell.

Alternatively, the third source cell and the first source cell are derived from heterologous individuals and the third source cell and the second source cell are derived from homologous individuals. For example, when the first source cell is a human cell, and the second source cell is a mouse cell, the third source cell may be a mouse cell.

Alternatively, the third source cell and the first source cell are derived from homologous individuals and the third source cell and the second source cell are derived from heterologous individuals. For example, when the first source cell is a mouse cell, and the second source cell is a rat cell, the third source cell may be a mouse cell.

The first fragment and the second fragment may be connected by a phosphodiester bond.

The second fragment and the third fragment may be connected by a phosphodiester bond.

The artificial recombinant chromosome may consist of the sequence of [first fragment]-[second fragment]-[third fragment].

Here, the second fragment may have an inverted form. Here, the inverted form may be the inversion of the second fragment present in the second source chromosome. In this case, in a cell including the artificial recombinant chromosome, a gene included in the second fragment may not be expressed as a protein. Alternatively, a cell including the artificial recombinant chromosome may have a different expression pattern of the gene included in the second fragment, compared to the second source cell including the second source chromosome.

The artificial recombinant chromosome may have both ends of a telomere derived from the same individual.

Alternatively, the artificial recombinant chromosome may have two telomeres derived from heterologous individuals. The artificial recombinant chromosome may have two telomeres with different lengths.

Here, the artificial recombinant chromosome is not the same as the first source chromosome, the second source chromosome or the third source chromosome.

### Another aspect of the disclosure in the specification relates to a method of producing an artificial recombinant chromosome.

The artificial recombinant chromosome may be produced from a source chromosome.

The source chromosome may be an artificially manipulated chromosome.

The artificially manipulated chromosome may be a targeted chromosome.

The "targeted chromosome" means a chromosome further including one or a plurality of constituent elements on a natural chromosome for recombination. In one example, the targeted chromosome may be a chromosome further including one or a plurality of recombinase recognition sites (RRSs) on a natural chromosome. In another example, the targeted chromosome may be a chromosome further including one or a plurality of artificial sequences for chromosome exchange (ASCEs) on a natural chromosome.

The targeted chromosome may be produced from a natural chromosome.

Descriptions relating to the source chromosome are the same as described above.

In one example, a first targeted chromosome may be produced from a first natural chromosome. The first targeted chromosome may include one or a plurality of RRSs on the first natural chromosome. The first targeted chromosome may include one or a plurality of ASCEs on the first natural chromosome.

In another example, a second targeted chromosome may be produced from a second natural chromosome. The second targeted chromosome may include one or a plurality of RRSs on the second natural chromosome. The second targeted chromosome may include one or a plurality of ASCEs on the second natural chromosome.

The "recombinase recognition site (RRS)" means a nucleic acid sequence that can provide a recombination site by a site-specific recombinase. In one example, the RRS may be a loxP site or a variant thereof (Table 1). In another example, the RRS may be an FRT site or a variant thereof. In one example, the RRS may be attP/attB or a variant thereof. In another example, the RRS may be an inverted terminal repeat (ITR) sequence or a variant thereof, which is recognized by one or more transposases. However, the RRS is not limited thereto.

To construct the targeted chromosome from a natural chromosome, a site-specific recombination system may be used. The site-specific recombination system is a system using an SSR acting on an RRS, and is known in the art. The site-specific recombination system may include Cre-lox. The site-specific recombination system may include FLP/FRT. The site-specific recombination system may include φC31 integrase-attP/attB. The site-specific recombination system may include transposon-ITR. However, the RRS and SSR mediated site-specific recombination system is not limited thereto, and various types of recombinases, integrases, resolvases or trasposases are used as SSRs, and depending on the SSR, an RRS can be modified in various forms and designed.

The RRS may be a known sequence. In one example, the RRS may be loxP or a variant thereof.

For example, the loxP variant may be one or more of Lox m2/71, Lox m2/66, Lox71 and Lox66. The DNA sequences of the loxP variants are disclosed in Table 1 below. Hereinafter, a sequence number is listed as SEQ ID NO:.

**[Table 1]**

| | DNA sequences of loxP variants | | |
|---|---|---|---|
| No. | Lox variant | DNA sequence | SEQ ID NO: |
| 1 | Lox m2/71 | 5'-taccgTTCGTATAtggTttcTTATACGAAGTTAT-3' | 23 |
| 2 | Lox m2/66 | 5'-ATAACTTCGTATAtggTttcTTATACGAAcggta-3' | 24 |
| 3 | Lox 71 | 5'-taccgTTCGTATAGCATACATTATACGAAGTTAT-3' | 25 |
| 4 | Lox 66 | 5'-ATAACTTCGTATAGCATACATTATACGAAcggta-3' | 26 |

The RRS may be a known sequence. In another example, the RRS may be an FRT site or a variant thereof.

In still another example, the RRS may be attP/attB or a variant thereof. In yet another example, the RRS may be an ITR sequence or a variant thereof, which is recognized by a transposase. Here, the ITR may be a transposon ITR, which may include a transposon terminal repeat (TR) sequence. For example, the transposon ITR sequence may include a piggyBac terminal repeat (PB-TR).

The DNA sequences of the RRS and variants thereof are listed in Table 2 below.

**[Table 2]**

| | DNA sequences of RRS | | |
|---|---|---|---|
| No. | RRS | DNA sequence | ` SEQ ID NO: |
| 1 | FRT | 5'-gaagttcctatactttctagagaataggaacttcggaataggaacttc-3' | 27 |
| 2 | ϕC31-attP | 5'- | 28 |
| | | cccaggtcagaagcggttttcgggagtagtgccccaactggggtaacctttgagttctct cagttgggggcgtagggtcgccgaca tgacacaaggggtt-3' | |
| 3 | ϕC31-attB | 5'- ctcgaagccgcggtgcgggtgccagggcgtgcccttgggctccccgggcgcgtactc cacctcacccatc-3' | 29 |
| 4 | PiggyBac right (3') ITR | 5'- ccctagaaagataatcatattgtgacgtacgttaaagataatcatgcgtaaaattgacgca tg-3' | 30 |
| 5 | PiggyBac left (5') ITR | 5'-catgcgtcaattttacgcagactatctttctaggg-3' | 31 |

The "artificial sequence for chromosome exchange (ASCE)" means a nucleic acid sequence that provides a recombination site by homologous recombination (HR). The ASCE may be an artificial sequence. The ASCE may be an artificial sequence included in a targeted chromosome. For example, the first targeted chromosome may include a first ASCE. The second targeted chromosome may include a second ASCE. Here, the first ASCE included in the first targeted chromosome and the second ASCE included in the second targeted chromosome may be used in subsequent homologous recombination.

To construct the targeted chromosome from a natural chromosome, homologous recombination may be used. The homologous recombination may be performed by double strand breaking (DSB) and/or single strand breaking (SSB) of a chromosome. The SSB and/or DSB may naturally occur. The SSB and/or DSB may be generated by a clastogen (a substance that cause an abnormality in a chromosome). The clastogen may be ionizing radiation, UV, X-rays, γ-rays, reactive oxygen species or a specific chemical. The specific chemical may be, for example, bleomycin, hydroxyurea, camptothecin, 4-nitroquinoline 1-oxide (4-NQO), cisplatin, or a methylating agent such as EMS or MMS, but the present invention is not limited thereto. The SSB and/or DSB may be generated by engineered nucleases. For example, the SSB and/or DSB may be generated by any one or more of zinc-finger nucleases (ZFN), transcription activator-like effector nucleases (TALEN) and clustered regularly interspaced short palindromic repeats/CRISPR associated protein (CRISPR/Cas).

In one exemplary embodiment, the artificial recombinant chromosome may be produced by at least two or more targeted chromosomes.

The at least two or more targeted chromosomes may include a first targeted chromosome and a second targeted chromosome.

Here, the first targeted chromosome may include one or more RRSs.

Here, the second targeted chromosome may include one or more RRSs.

Here, the RRS included in the first targeted chromosome and the RRS included in the second targeted chromosome may be recognized by a site-specific recombinase (SSR). Here, the RRS included in the first targeted chromosome and the RRS included in the second targeted chromosome may be paired with each other.

The artificial recombinant chromosome may include a part of the first targeted chromosome and a part of the second targeted chromosome.

Here, the artificial recombinant chromosome may be generated by site-specific recombination using the pairing of the RRS included in the first targeted chromosome and the RRS included in the second targeted chromosome.

In one example, when the first targeted chromosome consists of [first fragment]-[first RRS]-[second fragment], and the second targeted chromosome consists of [third fragment]-[second RRS]-[fourth fragment],
the artificial recombinant chromosome may consist of [first fragment]-[third fragment], [first fragment]-[fourth fragment], [third fragment]-[second fragment] or [second fragment]-[fourth fragment].

Alternatively, the artificial recombinant chromosome may consist of [first fragment]-[first RRS]-[third fragment], [first fragment]-[first RRS]-[fourth fragment], [third fragment]-[first RRS]-[second fragment] or [second fragment]-[first RRS]-[fourth fragment].

Alternatively, the artificial recombinant chromosome may consist of [first fragment]-[second RRS]-[third fragment], [first fragment]-[second RRS]-[fourth fragment], [third fragment]-[second RRS]-[second fragment] or [second fragment]-[second RRS]-[fourth fragment].

Alternatively, the artificial recombinant chromosome may consist of [first fragment]-[third RRS]-[third fragment], [first fragment]-[third RRS]-[fourth fragment], [third fragment]-[third RRS]-[second fragment] or [second fragment]-[third RRS]-[fourth fragment]. Here, the third RRS may be RRS generated by recombination of the first RRS and the second RRS, and may not be the same as the first RRS and the second RRS.

In another example, when the first targeted chromosome consists of [first fragment]-[first RRS]-[second fragment]-[second RRS]-[third fragment], and the second targeted chromosome consists of [fourth fragment]-[third RRS]-[fifth fragment]-[fourth RRS]-[sixth fragment],
the artificial recombinant chromosome may consist of [first fragment]-[fifth fragment]-[third fragment] or [fourth fragment]-[second fragment]-[sixth fragment].

Alternatively, the artificial recombinant chromosome may consist of [first fragment]-[first RRS]-[fifth fragment]-[second RRS]-[third fragment], [first fragment]-[first RRS]-[fifth fragment]-[fourth RRS]-[third fragment], [first fragment]-[third RRS]-[fifth fragment]-[second RRS]-[third fragment], [first fragment]-[third RRS]-[fifth fragment]-[fourth RRS]-[third fragment], [fourth fragment]-[first RRS]-[second fragment]-[second RRS]-[sixth fragment], [fourth fragment]-[first RRS]-[second fragment]-[fourth RRS]-[sixth fragment], [fourth fragment]-[third RRS]-[second fragment]-[second RRS]-[sixth fragment] or [fourth fragment]-[third RRS]-[second fragment]-[fourth RRS]-[sixth fragment].

Alternatively, the artificial recombinant chromosome may consist of [first fragment]-[fifth RRS]-[fifth fragment]-[second RRS]-[third fragment], [first fragment]-[fifth RRS]-[fifth fragment]-[fourth RRS]-[third fragment], [fourth fragment]-[fifth RRS]-[second fragment]-[second RRS]-[sixth fragment] or [fourth fragment]-[fifth RRS]-[second fragment]-[fourth RRS]-[sixth fragment]. Here, the fifth RRS may be RRS generated by recombination of the first RRS and the third RRS, and may not be the same as the first RRS and the third RRS.

Alternatively, the artificial recombinant chromosome may consist of [first fragment]-[first RRS]-[fifth fragment]-[sixth RRS]-[third fragment], [first fragment]-[third RRS]-[fifth fragment]-[sixth RRS]-[third fragment], [fourth fragment]-[first RRS]-[second fragment]-[sixth RRS]-[sixth fragment] or [fourth fragment]-[third RRS]-[second fragment]-[sixth RRS]-[sixth fragment]. Here, the sixth RRS may be RRS generated by recombination of the second RRS and the fourth RRS, and may not be the same as the second RRS and the fourth RRS.

Alternatively, the artificial recombinant chromosome may consist of [first fragment]-[fifth RRS]-[fifth fragment]-[sixth RRS]-[third fragment] or [fourth fragment]-[fifth RRS]-[second fragment]-[sixth RRS]-[sixth fragment]. Here, the fifth RRS may be RRS generated by recombination of the first RRS and the third RRS, and may not be the same as the first RRS and the third RRS. Here, the sixth RRS may be RRS generated by recombination of the second RRS and the fourth RRS, and may not be the same as the second RRS and the fourth RRS.

In another exemplary embodiment, the artificial recombinant chromosome may be produced by at least two or more targeted chromosomes.

The at least two or more targeted chromosomes may include a first targeted chromosome and a second targeted chromosome.

Here, the first targeted chromosome may include one or more ASCEs.

Here, the second targeted chromosome may include one or more ASCEs.

Here, the ASCEs included in the first targeted chromosome may be sequences homologous to those included in the second targeted chromosome.

The artificial recombinant chromosome may include a part of the first targeted chromosome and a part of the second targeted chromosome.

Here, the artificial recombinant chromosome may be generated by homologous recombination using homology of the ASCE included in the first targeted chromosome and the ASCE included in the second targeted chromosome.

In one example, when the first targeted chromosome consists of [first fragment]-[first ASCE]-[second fragment]-[second ASCE]-[third fragment], and the second targeted chromosome consists of [fourth fragment]-[third ASCE]-[fifth fragment]-[fourth ASCE]-[sixth fragment],
the artificial recombinant chromosome may consist of [first fragment]-[fifth fragment]-[third fragment] or [fourth fragment]-[second fragment]-[sixth fragment].

Alternatively, the artificial recombinant chromosome may consist of [first fragment]-[first ASCE]-[fifth fragment]-[second ASCE]-[third fragment], [first fragment]-[first ASCE]-[fifth fragment]-[fourth ASCE]-[third fragment], [first fragment]-[third ASCE]-[fifth fragment]-[second ASCE]-[third fragment], [first fragment]-[third ASCE]-[fifth fragment]-[fourth ASCE]-[third fragment], [fourth fragment]-[first ASCE]-[second fragment]-[second ASCE]-[sixth fragment], [fourth fragment]-[first ASCE]-[second fragment]-[fourth ASCE]-[sixth fragment], [fourth fragment]-[third ASCE]-[second fragment]-[second ASCE]-[sixth fragment] or [fourth fragment]-[third ASCE]-[second fragment]-[fourth ASCE]-[sixth fragment].

### Still another aspect of the disclosure in the specification relates to a method of producing cell including one or more artificial recombinant chromosomes.

The method of producing a cell including one or more artificial recombinant chromosomes may use cell fusion.

The "cell fusion" means the fusion between two or more cells; and/or fusion between one or more cells and one or more cell analogs. Here, the fusion may be production of one cell through combining (or mixing) between two or more cells; and/or production of one cell through combining (or mixing) between one or more cells and one or more cell analogs. Here, the cell analogs may be cells or cell-derived materials which include a part of the genome or a part of the whole chromosomes, but do not undergo normal somatic division (mitosis) or meiosis.

The cell fusion may produce a fusion cell.

The "fusion cell" means a cell produced such that a source cell has one or more chromosomes or chromosome fragments. Here, the one or more chromosomes or chromosome fragments are chromosomes or chromosome fragments further added, not naturally occurring in the source cell. Here, the one or more chromosomes or chromosome fragments may be a source chromosome, a fragment of a source chromosome, an artificial recombinant chromosome, or a fragment of an artificial recombinant chromosome.

The fusion cell may be a cell in which one or more source chromosomes or source chromosome fragments are included in a source cell. Here, the one or more source chromosomes or source chromosome fragments may be chromosomes or chromosome fragments which are further added to the source cell, not naturally occurring in the source cell.

For example, when a human fibroblast is a source cell, the fusion cell may be a human fibroblast fusion cell including one or more mouse fibroblast-derived chromosomes. Here, the human fibroblast fusion cell may have a different genome from the human fibroblast, that is, the source cell, and may also have a different genome from the mouse fibroblast. Here, the human fibroblast fusion cell may include 2n (46) human fibroblast-derived chromosomes and one mouse fibroblast-derived chromosome. Alternatively, the human fibroblast fusion cell may include 2n-1 (45) human fibroblast-derived chromosomes and one mouse fibroblast-derived chromosome.

The fusion cell may be a cell in which one or more recombinant chromosomes are contained in the source cell. Here, the one or more recombinant chromosomes may be produced by recombination between chromosomes in the fusion cell. Here, the recombinant chromosome may be an artificial recombinant chromosome.

For example, when a mouse embryonic stem cell (ESC) is a source cell, the fusion cell may be a mouse fusion ESC including one or more chromosomes derived from a human fibroblast. Here, the mouse fusion ESC may have a different genome from the mouse ESC, that is, the source cell, and may also have a different genome from the human fibroblast. Here, the mouse fusion ESC may include 2n (40) mouse ESC-derived chromosomes and one human fibroblast-derived chromosome. Alternatively, the mouse fusion ESC may include 2n-1 (39) mouse ESC-derived chromosomes and one human fibroblast-derived chromosome. Alternatively, the mouse fusion ESC may include 2n-1 (39) mouse ESC-derived chromosomes and two recombinant chromosomes, wherein the two recombinant chromosomes may be generated by recombination between one mouse ESC-derived chromosome and one human fibroblast-derived chromosome. Alternatively, the mouse fusion ESC may include 2n-1 (39) mouse ESC-derived chromosomes and one recombinant chromosome, wherein the one recombinant chromosome may be generated by recombination between one mouse ESC-derived chromosome and one human fibroblast-derived chromosome.

The fusion cell may be an animal cell having 2n+1 chromosomes.

The fusion cell may be an animal cell having 2n chromosomes. Here, the 2n chromosomes may include at least one artificial recombinant chromosome.

The fusion cell may be a cell including one or more artificial recombinant chromosomes.

The fusion cell may undergo normal somatic division (mitosis) or meiosis.

The fusion cell may be an animal germ cell having n+1 chromosomes.

The fusion cell may be an animal germ cell having n chromosomes. Here, the n chromosomes may include at least one artificial recombinant chromosome.

The fusion cell may include a cell including one or more artificial recombinant chromosomes.

Descriptions relating to the artificial recombinant chromosome are the same as described above.

In one exemplary embodiment, the method of producing a cell including one or more artificial recombinant chromosomes may include:
i) production of targeted cells;
ii) production of a microcell using the targeted cell;
iii) production of a fusion cell using the microcell; and
iv) production of a cell including an artificial recombinant chromosome using the fusion cell.

The targeted cells produced in the step i) may include two or more targeted cells.

Here, the two or more targeted cells may include a donor cell and a recipient cell.

The targeted cell used in the step ii) may be a donor cell.

The microcell used in the step iii) may be fused with a recipient cell.

Each step will be described in detail below.

### i) Production of targeted cells

The targeted cells produced in the step i) may include two or more target cells.

The two or more target cells may include a donor cell and a recipient cell. The donor cell may be a cell including one or more targeted chromosomes, and the recipient cell may be a cell including one or more targeted chromosomes. Here, the targeted chromosome included in the donor cell may be associated with the targeted chromosome included in the recipient cell. The association may be the possibility of pairing or homologous binding between constituent elements that can induce recombination between the targeted chromosome included in the donor cell and the targeted chromosome included in the recipient cell.

Here, the constituent element may be an RRS or ASCE, which has been described above.

The association may be pairing of an RRS present in the targeted chromosome included in the donor cell and an RRS present in the targeted chromosome included in the recipient cell.

For example, the targeted chromosome included in the donor cell includes one or more RRSs (e.g., first RRS), and the targeted chromosome included in the recipient cell includes one or more RRSs (e.g., second RRS). Here, the RRS (e.g., the first RRS) of the targeted chromosome included in the donor cell and the RRS (e.g., the second RRS) of the targeted chromosome included in the recipient cell may be designed to be paired with each other.

In another example, when the targeted chromosome included in the donor cell includes two or more RRSs (e. g., the first RRS and the second RRS), and the targeted chromosome included in the recipient cell includes two or more RRSs (e.g., the third RRS and the fourth RRS), one RRS (e.g., the first RRS) of the targeted chromosome included in the donor cell and one of the two RRSs (e.g., the third RRS and the fourth RRS) of the targeted chromosome included in the recipient cell need to be designed for pairing with each other, and in addition, the other RRS (e.g., the second RRS) of the targeted chromosome included in the donor cell and the other of the two RRSs (e.g., the third and fourth RRSs) of the targeted chromosome included in the recipient cell need to be designed for pairing with each other.

The association may be homologous binding between an ASCE present in the targeted chromosome included in the donor cell and an ASCE present in the targeted chromosome included in the recipient cell.

In the step i), a targeted cell, that is, a donor cell and a recipient cell may be produced.

The donor cell and the recipient cell may be produced, independently, according to a method that will be described below.

The "targeted cell" means one type of source cells, which is a cell including one or more targeted chromosomes.

The targeted cell may include at least one targeted chromosome.

The targeted cell may include one or more natural chromosomes.

The description of the targeted chromosome is as described above.

The description of the natural chromosome is as described above.

The targeted cell may be derived from a human cell. The targeted cell may be derived from a non-human cell. For example, the non-human cell may be derived from a mouse cell, a rat cell, a rodent cell, a goral cell, a cattle cell or an ungulate cell, but the present invention is not limited thereto.

The targeted cell may be derived from a somatic cell. For example, the somatic cell may be, for example, a fibroblast, but the present invention is not limited thereto.

The targeted cell may be derived from an immune cell. For example, the immune cell may be a B-cell, a T-cell, an NK cell, a macrophage, a neutrophil, a basophil or eosinophil, but the present invention is not limited thereto.

The targeted cell may be derived from a germ cell. For example, the targeted cell may be a sperm, a spermatocyte, a spermatogonial stem cell, an egg, an oocyte, an oogonial stem cell or a fertilized egg, but the present invention is not limited thereto.

The targeted cell may be derived from a stem cell. For example, the stem cell may be derived from an embryonic stem cell (ES cell), an adult stem cell, an umbilical cord blood stem cell, a spermatogonial stem cell or an oogonial stem cell, but the present invention is not limited thereto.

The targeted cell may be produced from a cell including a natural chromosome and/or a chromosome with an artificial modification, other than the purpose of producing a targeted chromosome.

The cell including a natural chromosome and/or a chromosome with an artificial modification, other than the purpose of producing a targeted chromosome, is one type of source cell, and the description of the source cell is as described above. The cell including a natural chromosome and/or a chromosome with an artificial modification, other than the purpose of producing a targeted chromosome, is disclosed as a non-target source cell below, and the natural chromosome and/or chromosome with an artificial modification, other than the purpose of producing a targeted chromosome, is disclosed as a non-target source chromosome below.

The targeted cell may be produced from a non-target source cell.

For example, the first targeted cell may be produced from a first non-target source cell. The first targeted cell may be produced by substitution of one and/or two or more non-target source chromosomes included in the first non-target source cell with a targeted chromosome.

For example, the second targeted cell may be produced from a second non-target source cell. The second targeted cell may be produced by substitution of one and/or two or more non-target source chromosomes included in the second non-target source cell with a targeted chromosome.

The targeted cell may be produced by providing donor DNA to the non-target source cell.

The donor DNA may include at least one RRS and at least one homologous arm for a non-target source chromosome.

For example, the donor DNA may be a sequence including any one of the LoxP variants disclosed in Table 1 and a homologous arm for a non-target source chromosome.

The donor DNA may be a sequence including at least one ASCE and at least one homologous arm for a non-target source chromosome.

The donor DNA may further include a selection marker gene. There are one or more selection marker genes in the donor DNA. The selection marker gene may be a fluorescent protein gene, an antibiotic-resistant gene, a FISH target sequence or an inverted gene thereof. The fluorescent protein gene and an inverted gene thereof may be known sequences. For example, the fluorescent protein gene may be any one or more of a GFP gene, an YFP gene, an RFP gene and an mCherry gene, but the present invention is not limited thereto. The antibiotic-resistant gene and an inverted gene thereof may be known sequences. For example, the antibiotic-resistant gene may be any one or more of a hygromycin-resistant gene, a neomycin-resistant gene, a kanamycin-resistant gene, a blasticidin-resistant gene, a zeocin-resistant gene and a puroΔTK gene, but the present invention is not limited thereto. The FISH target sequence and an inverted gene thereof may be known sequences.

The donor DNA may further include a transposon ITR sequence. The transposon may be PiggyBac. For example, the transposon ITR sequence may be a PiggyBac right (3') ITR sequence and/or PiggyBac left (5') ITR sequence listed in Table 2. The transposon ITR may include a transposon terminal repeat (TR) sequence. For example, the transposon ITR sequence may include a piggyBac terminal repeat (PB-TR).

For example, the donor DNA may further include a transposon ITR sequence at a position adjacent to the homologous arm for the non-target source chromosome.

For example, the donor DNA may further include a transposon ITR sequence at a position adjacent to the RRS or ASCE.

The donor DNA may be provided to the non-target source cell using a known transfection method. For example, the transfection method may use a viral transfection method, a reagent transfection method, or a physical transfection method. The viral transfection method may use, for example, a lentivirus. The reagent transfection method may use, for example, calcium phosphate, a cation lipid, DEAE-dextran, or polyethylenimine (PEI). The physical transfection method may use, for example, electroporation. In addition, the transfection may use a liposome, but the present invention is not limited thereto.

The donor DNA may be inserted into a target sequence of the non-target source chromosome.

The target sequence may be a sequence on the non-target source chromosome provided for RRS or ASCE insertion, which is a gene locus and/or non-genetic sequence. The target sequence may be determined by an *in silico* design.

In one example, the RRS or ASCE may be inserted upstream of a target gene present in the non-target source chromosome. Therefore, the donor DNA providing the RRS or ASCE may include a homologous arm for one region upstream of the target gene.

In one example, the RRS or ASCE may be inserted downstream of a target gene present in the non-target source chromosome. Therefore, the donor DNA providing the RRS or ASCE may include a homologous arm for one region downstream of the target gene.

The donor DNA may be inserted into the target sequence of the non-target source chromosome through homologous recombination.

To perform homologous recombination on the target sequence, a step of generating SSB and/or DSB may be included. The SSB and/or DSB may naturally occur. The SSB and/or DSB may be generated by a clastogen (substance that causes an abnormality in a chromosome). The clastogen may be ionizing radiation, UV, X-rays, γ-rays, reactive oxygen species or a specific chemical. The specific chemical may be, for example, bleomycin, hydroxyurea, camptothecin, 4-nitroquinoline 1-oxide (4-NQO), cisplatin, or a methylating agent such as EMS or MMS, but the present invention is not limited thereto. The SSB and/or DSB may be generated by engineered nucleases. For example, the SSB and/or DSB may be generated by any one or more of zinc-finger nucleases (ZFN), transcription activator-like effector nucleases (TALEN) and clustered regularly interspaced short palindromic repeats/CRISPR associated protein (CRISPR/Cas).

The targeted cell produced by the above-described method may include at least one targeted chromosome.

Here, the targeted chromosome may be generated in various ways according to the composition of the donor DNA.

In one example, when the donor DNA includes a single RRS, the targeted chromosome may be a targeted chromosome including the single RRS.

In another example, when the donor DNA includes two or more RRSs, the targeted chromosome may be a targeted chromosome including the two or more RRSs.

In still another example, when the donor DNA includes a single RRS and a selection marker gene, the targeted chromosome may be a targeted chromosome including the single RRS and the selection marker gene.

In yet another example, when the donor DNA includes a single RRS and a transposon ITR sequence, the targeted chromosome may be a targeted chromosome including the single RRS and the transposon ITR sequence.

In yet another example, when the donor DNA includes a single RRS, a selection marker gene and a transposon ITR sequence, the targeted chromosome may be a targeted chromosome including the single RRS, the selection marker gene and the transposon ITR sequence.

In yet another example, when the donor DNA includes two or more RRSs, a selection marker gene and a transposon ITR sequence, the targeted chromosome may be a targeted chromosome including the two or more RRSs, the selection marker gene and the transposon ITR sequence.

In one example, when the donor DNA includes a single ASCE, the targeted chromosome may be a targeted chromosome including the single ASCE.

In another example, when the donor DNA includes two or more ASCEs, the targeted chromosome may be a targeted chromosome including the two or more ASCEs.

In still another example, when the donor DNA includes a single ASCE and a selection marker gene, the targeted chromosome may be a targeted chromosome including the single ASCE and the selection marker gene.

In yet another example, when the donor DNA includes a single ASCE and a transposon ITR sequence, the targeted chromosome may be a targeted chromosome including the single ASCE and the transposon ITR sequence.

In yet another example, when the donor DNA includes a single ASCE, a selection marker gene and a transposon ITR sequence, the targeted chromosome may be a targeted chromosome including the single ASCE, the selection marker gene and the transposon ITR sequence.

In yet another example, when the donor DNA includes two or more ASCEs, a selection marker gene and a transposon ITR sequence, the targeted chromosome may be a targeted chromosome including the two or more ASCEs, the selection marker gene and the transposon ITR sequence.

### i-1) Single RRS-inserted chromosome-containing cell

According to an exemplary embodiment disclosed herein, a targeted cell including a targeted chromosome and a method of producing the same may be provided. The targeted chromosome may include a single RRS.

The single RRS may be an RRS used in the generation of an artificial recombinant chromosome.

The description of the RRS and the targeted chromosome is as described above.

The targeted cell including a targeted chromosome containing the single RRS may be produced by providing donor DNA containing an RRS to a non-target source cell.

The donor DNA may be donor DNA having the single RRS and a homologous arm for the non-target source chromosome.

For example, the donor DNA may be a sequence including any one of the LoxP variants listed in Table 1 and a homologous arm for the non-target source chromosome. Here, the LoxP variants may be used in the generation of an artificial recombinant chromosome.

Here, the donor DNA may further include a selection marker gene and/or a transposon ITR sequence. The description of the selection marker gene and the transposon ITR sequence is as described above.

Here, the donor DNA may further include an additional RRS. Here, the additional RRS may be an RRS which is not used in the generation of an artificial recombinant chromosome.

For example, the donor DNA may be a sequence including any one of the LoxP variants listed in Table 1, an FRT, transposon ITR, an antibiotic-resistant gene and a homologous arm for a non-target source chromosome. Here, the FRT and the transposon ITR may be an RRS which is an additional RRS not used in the generation of an artificial recombinant chromosome.

The donor DNA may be provided to the non-target source cell using a known transfection method. The description of the transfection method is as described above.

A targeted cell including a targeted chromosome containing a single RRS may be produced by the donor DNA provided to the non-target source cell. The donor DNA provided to the non-target source cell may be inserted into a target sequence of the non-target source chromosome, and changed into the targeted chromosome. A cell including the targeted chromosome is a targeted cell.

To produce the targeted cell, the donor DNA having a single RRS may be transfected into the non-target source cell.

The donor DNA may include the single RRS and a homologous arm for the non-target source chromosome.

The single RRS may be an RRS used in the generation of an artificial recombinant chromosome.

Here, the donor DNA may further include a selection marker gene and/or a transposon ITR sequence. The description of the selection marker gene and the transposon ITR sequence is as described above.

Here, the donor DNA may further include an additional RRS. Here, the additional RRS may be an RRS which is not used in the generation of an artificial recombinant chromosome.

In one exemplary embodiment, a targeted cell having one targeted chromosome may be produced using one donor DNA.

Here, the targeted cell may be a donor cell.

The donor DNA may be a sequence including any one selected from the LoxP variants listed in Table 1 and a homologous arm for a non-target source chromosome.

Here, the homologous arm may be a sequence homologous to a part of the sequence of the non-target source chromosome.

The donor DNA may be introduced into the non-target source cell. Here, the donor DNA may have the homologous arm for a non-target source chromosome included in the non-target source cell.

Here, the donor DNA may further include a selection marker gene and/or a transposon ITR sequence. The description of the selection marker gene and the transposon ITR sequence is as described above.

Here, the donor DNA may further include an additional RRS. Here, the additional RRS may be an RRS which is not used in the generation of an artificial recombinant chromosome.

The targeted chromosome may include the LoxP variant included in the donor DNA.

Here, the targeted chromosome may further include a selection marker gene and/or a transposon ITR sequence.

Here, the targeted chromosome may further include an additional RRS.

A recipient cell for the donor cell may be produced using one donor DNA. Here, the donor DNA may be a sequence including a LoxP variant capable of being paired with the LoxP variant included in the donor DNA used in the production of the donor cell and a homologous arm for a non-target source chromosome.

In another exemplary embodiment, a targeted cell having two or more targeted chromosomes may be produced using two or more donor DNAs.

Here, the targeted cell may be a donor cell.

The two or more donor DNAs may include first donor DNA and second donor DNA.

The first donor DNA may be a sequence including one selected from the LoxP variants listed in Table 1 and a homologous arm for a first non-target source chromosome. Here, the homologous arms may be a sequence homologous to a part of the sequence of the first non-target source chromosome.

The second donor DNA may be a sequence including one selected from the LoxP variants listed in Table 1 and a homologous arm for a second non-target source chromosome. Here, the homologous arms may be a sequence homologous to a part of the sequence of the second non-target source chromosome.

The first donor DNA and the second donor DNA may be introduced into a non-target source cell. Here, the first donor DNA and the second donor DNA may have homologous arms for the first non-target source chromosome and the second non-targeted chromosome included in the non-target source cell, respectively.

Here, each of the first donor DNA and the second donor DNA may further include a selection marker gene and/or a transposon ITR sequence. The description of the selection marker gene and the transposon ITR sequence is as described above.

Here, each of the first donor DNA and the second donor DNA may further include an additional RRS. Here, the additional RRS may be an RRS which is not used in the generation of an artificial recombinant chromosome.

The two or more targeted chromosomes may include a first targeted chromosome and a second targeted chromosome.

Here, the first targeted chromosome may include the LoxP variant included in the first donor DNA.

Here, the second targeted chromosome may include the LoxP variant included in the second donor DNA.

Here, the LoxP variant included in the first donor DNA may be the same as or different from that included in the second donor DNA.

Here, each of the first targeted chromosome and the second targeted chromosome may further include a selection marker gene and/or a transposon ITR sequence.

Here, each of the first targeted chromosome and the second targeted chromosome may further include an additional RRS.

A recipient cell for the donor cell may be produced using two or more donor DNAs (third donor DNA and fourth donor DNA). Here, the third donor DNA may be a sequence including a LoxP variant capable of being paired with the LoxP variant included in the first donor DNA used in the production of the donor cell and a homologous arm for a non-target source chromosome. Here, the fourth donor DNA may be a sequence including a LoxP variant capable of being paired with the LoxP variant included in the second donor DNA used in the production of the donor cell and a homologous arm for a non-target source chromosome.

To produce two or more different targeted cells, a first donor DNA having a first RRS may be transfected into a first non-target source cell. In addition, a second donor DNA having a second RRS may be transfected into a second non-target source cell.

The single RRS may be an RRS used in the generation of an artificial recombinant chromosome.

Each of the first donor DNA and the second donor DNA may include a homologous arm for a non-target source chromosome.

Here, the first donor DNA may have the first RRS and a homologous arm for a first non-target source chromosome. The homologous arm may be a sequence homologous to a part of the sequence of the first non-target source chromosome.

The second donor DNA may have the second RRS and a homologous arm for a second non-target source chromosome. The homologous arm may be a sequence homologous to a part of the sequence of the second non-target source chromosome.

Here, each of the first donor DNA and the second donor DNA may further include a selection marker gene and/or a transposon ITR sequence. The description of the selection marker gene and the transposon ITR sequence is as described above.

Here, each of the first donor DNA and the second donor DNA may further include an additional RRS. Here, the additional RRS may be an RRS which is not used in the generation of an artificial recombinant chromosome.

In one exemplary embodiment, two or more targeted cells may be produced using two or more donor DNAs.

The two or more donor DNAs may include a first donor DNA and a second donor DNA.

The first donor DNA may be a sequence including any one selected from the LoxP variants listed in Table 1 and a homologous arm for a first non-target source chromosome. Here, the homologous arm may be a sequence homologous to a part of the sequence of the first non-target source chromosome.

The second donor DNA may be a sequence including one selected form the LoxP variants listed in Table 1 and a homologous arm for a second non-target source chromosome. Here, the homologous arm may be a sequence homologous to a part of the sequence of the second non-target source chromosome. Here, the selected LoxP variant may be paired with the LoxP variant included in the first donor DNA.

Here, each of the first donor DNA and the second donor DNA may further include a selection marker gene and/or a transposon ITR sequence. The description of the selection marker gene and the transposon ITR sequence is as described above.

Here, each of the first donor DNA and the second donor DNA may further include an additional RRS. Here, the additional RRS may be an RRS which is not used in the generation of an artificial recombinant chromosome.

The first donor DNA may be introduced into a first non-target source cell. The second donor DNA may be introduced into a second non-target source cell. Here, the first donor DNA may have a homologous arm for the first non-target source chromosome included in the first non-target source cell. The second donor DNA may have a homologous arm for the second non-target source chromosome included in the second non-target source cell.

The two or more targeted cells may include a first targeted cell and a second targeted cell.

Here, the first targeted cell may be a donor cell, and the second targeted cell may be a recipient cell.

Here, the first targeted cell may include the first targeted chromosome including the LoxP variant included in the first donor DNA.

Here, the second targeted cell may include the second targeted chromosome including the LoxP variant included in the second donor DNA.

Here, the LoxP variant included in the first donor DNA may be the same as or different from that included in the second donor DNA.

Here, the LoxP variant included in the first donor DNA may be paired with that included in the second donor DNA.

Here, each of the first targeted chromosome and the second targeted chromosome may further include a selection marker gene and/or a transposon ITR sequence.

Here, each of the first targeted chromosome and the second targeted chromosome may further include an additional RRS.

### i-2) Two RRSs-inserted chromosome-containing cell

According to another exemplary embodiment disclosed herein, a targeted cell including a targeted chromosome and a method of producing the same may be provided. The targeted chromosome may include two or more RRSs.

The two or more RRSs may be RRSs used in the generation of an artificial recombinant chromosome.

The description of the RRS and the targeted chromosome is as described above.

The targeted cell including the targeted chromosome including two or more RRSs may be produced by providing donor DNA having RRSs to a non-target source cell.

The donor DNA may be provided to the source cell using a known transfection method. The description of the transfection method is as described above.

The targeted cell including the targeted chromosome including two or more RRSs may be produced by the donor DNA provided to the non-target source cell. The donor DNA provided to the non-target source cell may be inserted into a target sequence of a non-target source chromosome, and changed into the targeted chromosome. A cell including the targeted chromosome may be a targeted cell.

To produce a targeted cell, a donor DNA having two or more RRSs may be transfected into a non-target source cell.

The two or more RRSs may be RRSs used in the generation of an artificial recombinant chromosome.

The donor DNA may include two or more RRSs and a homologous arm for a non-target source chromosome.

Here, the donor DNA may further include a selection marker gene and/or a transposon ITR sequence. The description of the selection marker gene and the transposon ITR sequence is as described above.

Here, the donor DNA may further include an additional RRS. Here, the additional RRS may be an RRS which is not used in the generation of an artificial recombinant chromosome.

In one exemplary embodiment, a targeted cell having one targeted chromosome may be produced using one donor DNA.

Here, the targeted cell may be a donor cell.

The donor DNA may include two or more RRSs.

The two or more RRSs may include a first RRS and a second RRS.

Here, the first RRS may be selected from the LoxP variants listed in Table 1.

Here, the second RRS may be selected from the LoxP variants listed in Table 1.

Here, the first RRS and the second RRS may be the same or different from each other.

The donor DNA may include sequences including two or more homologous arms for a non-target source chromosome.

The two or more homologous arms may include a first homologous arm and a second homologous arm.

Here, the first homologous arm may be a sequence homologous to a part of the sequence of the non-target source chromosome.

Here, the second homologous arm may be a sequence homologous to a part of the sequence of the non-target source chromosome.

Here, the first homologous arm may have a sequence different from the second homologous arm.

The donor DNA may further include a selection marker gene and/or a transposon ITR sequence. The description of the selection marker gene and the transposon ITR sequence is as described above.

The donor DNA may further include an additional RRS. Here, the additional RRS may be an RRS which is not used in the generation of an artificial recombinant chromosome.

The donor DNA may be introduced into a non-target source cell. Here, the donor DNA may have two or more homologous arms for the non-target source chromosome included in the non-target source cell.

The targeted chromosome may include a first RRS and a second RRS, which are included in the donor DNA.

Here, the first RRS and the second RRS may be the same or different from each other.

The targeted chromosome may include two or more LoxP variants.

Here, the targeted chromosome may further include a selection marker gene and/or a transposon ITR sequence.

Here, the targeted chromosome may further include an additional RRS.

A recipient cell for the donor cell may be produced using one or more donor DNAs. Here, the donor DNA may be a sequence including a third RRS capable of being paired with the first RRS included in the donor DNA used in the production of the donor cell and a homologous arm for a non-target source chromosome. In addition, the donor DNA may be a sequence including a fourth RRS capable of being paired with the second RRS included in the donor DNA used in the production of the donor cell and a homologous arm for the non-target source chromosome.

In another exemplary embodiment, a targeted cell having one targeted chromosome may be produced using two or more donor DNAs.

Here, the targeted cell may be a donor cell.

The two or more donor DNAs may include first donor DNA and second donor DNA.

The first donor DNA may be a sequence including one selected from the LoxP variants listed in Table 1 and a first homologous arm for a non-target source chromosome.

The second donor DNA may be a sequence including one selected from the LoxP variants listed in Table 1 and a second homologous arm for a non-target source chromosome.

Here, the first homologous arm may be a sequence homologous to a part of the sequence of the non-target source chromosome.

Here, the second homologous arm may be a sequence homologous to a part of the sequence of the non-target source chromosome.

Here, the first homologous arm may have a sequence different from the second homologous arm.

Each of the first donor DNA and the second donor DNA may further include a selection marker gene and/or a transposon ITR sequence. The description of the selection marker gene and the transposon ITR sequence is as described above.

Each of the first donor DNA and the second donor DNA may further include an additional RRS. Here, the additional RRS may be an RRS which is not used in the generation of an artificial recombinant chromosome.

The first donor DNA and the second donor DNA may be introduced into a non-target source cell. Here, each of the first donor DNA and the second donor DNA may have the homologous arm for the non-target source chromosome included in the non-target source cell.

The targeted chromosome may include two or more LoxP variants.

Here, one of the two or more LoxP variants may be LoxP variants included in the first donor DNA. The other of the two or more LoxP variants may be LoxP variants included in the second donor DNA.

Here, the LoxP variant included in the first donor DNA may be the same as or different from that included in the second donor DNA.

The targeted chromosome may further include a selection marker gene and/or a transposon ITR sequence.

Here, the targeted chromosome may further include an additional RRS.

A recipient cell for the donor cell may be produced using two or more donor DNAs (third donor DNA and fourth donor DNA). Here, the third donor DNA may be a sequence including a LoxP variant capable of being paired with the LoxP variant included in the first donor DNA used in the production of the donor cell and a homologous arm for a non-target source chromosome. Here, the fourth donor DNA may be a sequence including a LoxP variant capable of being paired with the LoxP variant included in the second donor DNA used in the production of the donor cell and a homologous arm for the non-target source chromosome.

In still another exemplary embodiment, a targeted cell having two or more targeted chromosomes may be produced using two or more donor DNAs.

Here, the targeted cell may be a donor cell.

The two or more donor DNAs may include first donor DNA and second donor DNA.

The first donor DNA may include two or more RRSs. Here, the two or more RRSs may include a first RRS and a second RRS.

The second donor DNA may include two or more RRSs. Here, the two or more RRSs may include a third RRS and a fourth RRS.

Here, the first RRS may be selected from the LoxP variants listed in Table 1.

Here, the second RRS may be selected from the LoxP variants listed in Table 1.

Here, the third RRS may be selected form the LoxP variants listed in Table 1.

Here, the fourth RRS may be selected from the LoxP variants listed in Table 1.

Here, all of the first RRS, the second RRS, the third RRS and the fourth RRS may be the same or different from each other. Alternatively, the first RRS, the second RRS, the third RRS and the fourth RRS may be partly the same or different from each other. For example, the first RRS and the third RRS may be the same, and the second RRS and the fourth RRS may be different from the first RRS. Alternatively, the first RRS and the fourth RRS may be the same, and the second RRA and the third RRS may be the same. Alternatively, all of the first RRS, the second RRS, the third RRS and the fourth RRS may be different from each other. Alternatively, all of the first RRS, the second RRS, the third RRS and the fourth RRS may be the same.

The first donor DNA may include a sequence including two or more homologous arms for a first non-target source chromosome. Here, the two or more homologous arms may include a first homologous arm and a second homologous arm.

Here, the first homologous arm may be a sequence homologous to a part of the sequence of the first non-target source chromosome.

Here, the second homologous arm may be a sequence homologous to a part of the sequence of the first non-target source chromosome.

Here, the first homologous arm may have a sequence different from the second homologous arm.

The second donor DNA may include a sequence including two or more homologous arms for a second non-target source chromosome. Here, the two or more homologous arms may include a third homologous arm and a fourth homologous arm.

Here, the third homologous arm may be a sequence homologous to a part of the sequence of the second non-target source chromosome.

Here, the fourth homologous arm may be a sequence homologous to a part of the sequence of the second non-target source chromosome.

Here, the third homologous arm may have a sequence different from the fourth homologous arm.

Each of the first donor DNA and the second donor DNA may further include a selection marker gene and/or a transposon ITR sequence. The description of the selection marker gene and the transposon ITR sequence is as described above.

Here, each of the first donor DNA and the second donor DNA may further include an additional RRS. Here, the additional RRS may be an RRS which is not used in the generation of an artificial recombinant chromosome.

The first donor DNA and the second donor DNA may be introduced into a non-target source cell. Here, the first donor DNA may have homologous arms for the first non-target source chromosome included in the non-target source cell. The second donor DNA may have homologous arms for the second non-targeted chromosome included in the non-target source cell.

The two or more targeted chromosomes may include a first targeted chromosome and a second targeted chromosome.

Here, the first targeted chromosome may include the first RRS and the second RRS. Here, the first RRS and the second RRS may be the same or different from each other.

Here, the second targeted chromosome may include the third RRS and the fourth RRS. Here, the third RRS and the fourth RRS may be the same or different from each other.

The first targeted chromosome may include two or more LoxP variants.

The second targeted chromosome may include two or more LoxP variants.

Here, each of the first targeted chromosome and the second targeted chromosome may further include a selection marker gene and/or a transposon ITR sequence.

Here, each of the first targeted chromosome and the second targeted chromosome may further include an additional RRS.

A recipient cell for the donor cell may be produced using two or more donor DNAs (third donor DNA and fourth donor DNA). Here, the third donor DNA may be a sequence including a fifth RRS and a sixth RRS capable of being paired with the first and second RRSs included in the first donor DNA used in the production of the donor cell, respectively, and a homologous arm for a non-target source chromosome. Here, the fourth donor DNA may be a sequence including a seventh RRS and an eighth RRS capable of being paired with the third and fourth RRSs included in the second donor DNA used in the production of the donor cell, respectively, and a homologous arm for a non-target source chromosome.

To produce two or more different targeted cells, a first donor DNA having two or more RRSs may be transfected into a first non-target source cell. In addition, a second donor DNA having two or more RRSs may be transfected into a second non-target source cell.

The two or more RRSs may be RRSs used in the generation of an artificial recombinant chromosome.

Each of the first donor DNA and the second donor DNA may include a homologous arm for a non-target source chromosome.

The first donor DNA may have a first RRS, a second RRS and a homologous arm for a first non-target source chromosome.

The second donor DNA may have a third RRS, a fourth RRS and a homologous arm for a second non-target source chromosome.

Here, each of the first donor DNA and the second donor DNA may further include a selection marker gene and/or a transposon ITR sequence. The description of the selection marker gene and the transposon ITR sequence is as described above.

Here, each of the first donor DNA and the second donor DNA may further include an additional RRS. Here, the additional RRS may be an RRS which is not used in the generation of an artificial recombinant chromosome.

In one exemplary embodiment, two or more targeted cells may be produced using two or more donor DNAs.

The two or more donor DNAs may include a first donor DNA and a second donor DNA.

The first donor DNA may include two or more RRSs. Here, the two or more RRSs may include a first RRS and a second RRS.

The second donor DNA may include two or more RRSs. Here, the two or more RRSs may include a third RRS and a fourth RRS.

Here, the first RRS may be selected from the LoxP variants listed in Table 1.

Here, the second RRS may be selected from the LoxP variants listed in Table 1.

Here, the third RRS may be selected from the LoxP variants listed in Table 1.

Here, the fourth RRS may be selected from the LoxP variants listed in Table 1.

Here, all of the first RRS, the second RRS, the third RRS and the fourth RRS may be the same or different from each other. Alternatively, the all of the first RRS, the second RRS, the third RRS and the fourth RRS may be partly the same or different from each other. For example, the first RRS and the fourth RRS may be the same, and the second RRS and the third RRS may be different from the first RRS. Alternatively, the second RRS and the fourth RRS may be the same, and the first RRA and the third RRS may be the same. Alternatively, all of the first RRS, the second RRS, the third RRS and the fourth RRS may be different from each other. Alternatively, all of the first RRS, the second RRS, the third RRS and the fourth RRS may be the same.

The first donor DNA may include a sequence including two or more homologous arms for a first non-target source chromosome. Here, the two or more homologous arms may include a first homologous arm and a second homologous arm.

Here, the first homologous arm may be a sequence homologous to a part of the sequence of the first non-target source chromosome.

Here, the second homologous arm may be a sequence homologous to a part of the sequence of the first non-target source chromosome.

Here, the first homologous arm may be a sequence different from the second homologous arm.

The second donor DNA may include a sequence including two or more homologous arms for a second non-target source chromosome. Here, the two or more homologous arms may include a third homologous arm and a fourth homologous arm.

Here, the third homologous arm may be a sequence homologous to a part of the sequence of the second non-target source chromosome.

Here, the fourth homologous arm may be a sequence homologous to a part of the sequence of the second non-target source chromosome.

Here, the third homologous arm may have a sequence different from the fourth homologous arm.

Here, each of the first donor DNA and the second donor DNA may further include a selection marker gene and/or a transposon ITR sequence. The description of the selection marker gene and the transposon ITR sequence is as described above.

Here, each of the first donor DNA and the second donor DNA may include an additional RRS. Here, the additional RRS may be an RRS which is not used in the generation of an artificial recombinant chromosome.

The first donor DNA may be introduced into a first non-target source cell. The second donor DNA may be introduced into a second non-target source cell. Here, the first donor DNA may have the homologous arm for the first non-target source chromosome included in the first non-target source cell. The second donor DNA may have the homologous arm for the second non-target source chromosome included in the second non-target source cell.

The two or more targeted cells may include a first targeted cell and a second targeted cell.

Here, the first targeted cell may be a donor cell, and the second targeted cell may be a recipient cell.

Here, the first targeted cell may include a first targeted chromosome including the first and second RRSs.

Here, the second targeted cell may include a second targeted chromosome including the third and fourth RRSs. The third RRS may be paired with one of the first RRS and the second RRS, and the fourth RRS may be paired with the other of the first RRS and the second RRS.

Here, each of the first targeted chromosome and the second targeted chromosome may further include a selection marker gene and/or a transposon ITR sequence.

Here, each of the first targeted chromosome and the second targeted chromosome may further include an additional RRS.

### i-3) Single ASCE-inserted chromosome-containing cell

According to an exemplary embodiment disclosed herein, a targeted cell including a targeted chromosome and a method of producing the same may be provided. The targeted chromosome may include a single ASCE.

The description of the ASCE and the targeted chromosome is as described above.

The targeted cell including the targeted chromosome including the single ASCE may be produced by providing donor DNA having an ASCE to a non-target source cell.

The donor DNA may be provided to the source cell using a known transfection method. The description of the transfection method is as described above.

A targeted cell including a targeted chromosome having a single ASCE may be produced using donor DNA provided to the non-target source cell. The donor DNA provided to the non-target source cell may be inserted into a target sequence of a non-target source chromosome, and changed into the targeted chromosome. A cell including the targeted chromosome is a targeted cell.

To produce a targeted cell, a donor DNA having a single ASCE may be transfected into a non-target source cell.

The donor DNA may include the single ASCE and a homologous arm for a non-target source chromosome.

The single ASCE may be an ASCE used in the generation of an artificial recombinant chromosome.

Here, the donor DNA may further include a selection marker gene and/or a transposon ITR sequence. The description of the selection marker gene and the transposon ITR sequence is as described above.

Here, the donor DNA may further include an additional RRS. Here, the additional RRS may be an RRS which is not used in the generation of an artificial recombinant chromosome.

In one exemplary embodiment, a target cell having one targeted chromosome may be produced using one donor DNA.

The donor DNA may be a sequence including a single ASCE and a homologous arm for a non-target source chromosome.

The donor DNA may be introduced into a non-target source cell. Here, the donor DNA may have the homologous arm for the non-target source chromosome included in the non-target source cell.

Here, the donor DNA may further include a selection marker gene and/or a transposon ITR sequence. The description of the selection marker gene and the transposon ITR sequence is as described above.

Here, the donor DNA may further include an additional RRS. Here, the additional RRS may be an RRS which is not used in the generation of an artificial recombinant chromosome.

The targeted chromosome may include a single ASCE included in the donor DNA.

Here, the targeted chromosome may further include a selection marker gene and/or a transposon ITR sequence.

Here, the targeted chromosome may further include an additional RRS.

In another one exemplary embodiment, a target cell having two or more targeted chromosomes may be produced using two or more donor DNAs.

The two or more donor DNAs may include a first donor DNA and a second donor DNA.

The first donor DNA may be a sequence including a first ASCE and a homologous arm for a first non-target source chromosome. Here, the homologous arms may be a sequence homologous to a part of the sequence of the first non-target source chromosome.

The second donor DNA may be a sequence including a second ASCE and homologous arm for a second non-target source chromosome. Here, the homologous arm may be a sequence homologous to a part of the sequence of the second non-target source chromosome.

Here, the first ASCE may be the same as or different from the second ASCE.

Each of the first donor DNA and the second donor DNA may further include a selection marker gene and/or a transposon ITR. The description of the selection marker gene and the transposon ITR sequence is as described above.

Here, each of the first donor DNA and the second donor DNA may further include an additional RRS. Here, the additional RRS may be an RRS which is not used in the generation of an artificial recombinant chromosome.

The first donor DNA and the second donor DNA may be introduced into a non-target source cell. Here, each of the first donor DNA and the second donor DNA may have each homologous arm for the first non-target source chromosome and the second non-target chromosome included in the non-target source cell.

The two or more targeted chromosomes may include a first targeted chromosome and a second targeted chromosome.

Here, the first targeted chromosome may include the first ASCE.

Here, the second targeted chromosome may include the second ASCE.

Here, each of the first targeted chromosome and the second targeted chromosome may further include a selection marker gene and/or a transposon ITR sequence.

Here, each of the first targeted chromosome and the second targeted chromosome may further include an additional RRS.

To produce two or more different targeted cells, a first donor DNA having a first ASCE may be transfected into a first non-target source cell. In addition, a second donor DNA having a second ASCE may be transfected into a second non-target source cell.

Each of the first donor DNA and the second donor DNA may include a homologous arm for a non-target source chromosome.

The first donor DNA may have the first ASCE and a homologous arm for a first non-target source chromosome.

The second donor DNA may have the second ASCE and a homologous arm for a second non-target source chromosome.

Each of the first donor DNA and the second donor DNA may further include a selection marker gene and/or a transposon ITR sequence. The description of the selection marker gene and the transposon ITR sequence is as described above.

Here, each of the first donor DNA and the second donor DNA may further include an additional RRS. Here, the additional RRS may be an RRS which is not used in the generation of an artificial recombinant chromosome.

In one exemplary embodiment, two or more targeted cells may be produced using two or more donor DNAs.

The two or more donor DNAs may include a first donor DNA and a second donor DNA.

The first donor DNA may be a sequence including a first ASCE and a homologous arm for a first non-target source chromosome. Here, the homologous arm may be a sequence homologous to a part of the sequence of the first non-target source chromosome.

The second donor DNA may be a sequence including a second ASCE and a homologous arm for a second non-target source chromosome. Here, the homologous arm may be a sequence homologous to a part of the sequence of the second non-target source chromosome.

Here, the first ASCE may be may be the same as or different from the second ASCE.

Each of the first donor DNA and the second donor DNA may further include a selection marker gene and/or a transposon ITR sequence. The description of the selection marker gene and the transposon ITR sequence is as described above.

Here, each of the first donor DNA and the second donor DNA may further include an additional RRS. Here, the additional RRS may be an RRS which is not used in the generation of an artificial recombinant chromosome.

The first donor DNA may be introduced into a first non-target source cell. The second donor DNA may be introduced into a second non-target source cell. Here, the first donor DNA may have the homologous arm for the first non-target source chromosome in the first non-target source cell. The second donor DNA may have the homologous arm for the second non-target source chromosome in the second non-target source cell.

The two or more targeted cells may include a first targeted cell and a second targeted cell.

Here, the first targeted cell may include a first targeted chromosome having the first ASCE.

Here, the second targeted cell may include a second targeted chromosome having the second ASCE.

Here, each of the first targeted chromosome and the second targeted chromosome may further include a selection marker gene and/or a transposon ITR sequence.

Here, the first targeted chromosome and the second targeted chromosome may further include an additional RRS.

### i-4) Double ASCE-inserted chromosome-containing cell

According to another exemplary embodiment disclosed herein, a targeted cell including a targeted chromosome and a method of producing the same may be provided. The targeted chromosome may include two or more ASCEs.

The two or more ASCEs may be ASCEs used in the generation of an artificial recombinant chromosome.

The description of the ASCE and the targeted chromosome is as described above.

A targeted cell including the targeted chromosome having two or more ASCEs may be produced by providing a donor DNA having ASCEs to a non-target source cell.

The donor DNA may be provided to the source cell using a known transfection method. The description of the transfection method is as described above.

A targeted cell including a targeted chromosome having two or more ASCEs may be produced using the donor DNA provided to the non-target source cell. The donor DNA provided to the non-target source cell may be inserted into a target sequence of the non-target source chromosome, and changed into the targeted chromosome. A cell including the targeted chromosome is a targeted cell.

To produce a target cell, a donor DNA having two or more ASCEs may be transfected into a non-target source cell.

The donor DNA may include the two or more ASCEs and a homologous arm for a non-target source chromosome.

The two or more ASCEs may be ASCEs used in the generation of an artificial recombinant chromosome.

Here, the donor DNA may further include a selection marker gene and/or a transposon ITR sequence. The description of the selection marker gene and the transposon ITR sequence is as described above.

Here, the donor DNA may further include an additional RRS. Here, the additional RRS may be an RRS which is not used in the generation of an artificial recombinant chromosome.

In one exemplary embodiment, a target cell having one targeted chromosome may be produced using one donor DNA.

The donor DNA may include two or more ASCEs.

The two or more ASCEs may include a first ASCE and a second ASCE.

Here, the first ASCE and the second ASCE may be the same or different from each other.

The donor DNA may include a sequence including two or more homologous arms for a non-target source chromosome.

The two or more homologous arms may include a first homologous arm and a second homologous arm.

Here, the first homologous arm may be a sequence homologous to a part of the sequence of the non-target source chromosome.

Here, the second homologous arm may be a sequence homologous to a part of the sequence of the non-target source chromosome.

Here, the first homologous arm may have a sequence different from the second homologous arm.

The donor DNA may further include a selection marker gene and/or a transposon ITR sequence. The description of the selection marker gene and the transposon ITR sequence is as described above.

Here, the donor DNA may further include an additional RRS. Here, the additional RRS may be an RRS which is not used in the generation of an artificial recombinant chromosome.

The donor DNA may be introduced into a non-target source cell. Here, the donor DNA may have two or more homologous arms for the non-target source chromosome included in the non-target source cell.

The targeted chromosome may include a first ASCE and a second ASCE, which are included in the donor DNA.

Here, the first ASCE and the second ASCE may be the same or different from each other.

The targeted chromosome may further include a selection marker gene and/or a transposon ITR sequence.

The targeted chromosome may further include an additional RRS.

In another exemplary embodiment, a targeted cell having one targeted chromosome may be produced using two or more donor DNAs.

The two or more donor DNAs may include a first donor DNA and a second donor DNA.

The first donor DNA may be a sequence including a first ASCE and a first homologous arm for a non-target source chromosome.

The second donor DNA may be a sequence including a second ASCE and a second homologous arm for a non-target source chromosome.

Here, the first ASCE and the second ASCE may be the same or different from each other.

Here, the first homologous arm may be a sequence homologous to a part of the sequence of the non-target source chromosome.

Here, the second homologous arm may be a sequence homologous to a part of the sequence of the non-target source chromosome.

Here, the first homologous arm may have a sequence different from the second homologous arm.

Each of the first donor DNA and the second donor DNA may further include a selection marker gene and/or a transposon ITR sequence. The description of the selection marker gene and the transposon ITR sequence is as described above.

Here, each of the first donor DNA and the second donor DNA may further include an additional RRS. Here, the additional RRS may be an RRS which is not used in the generation of an artificial recombinant chromosome.

The first donor DNA and the second donor DNA may be introduced into a non-target source cell. Here, each of the first donor DNA and the second donor DNA may have the homologous arm for the non-target source chromosome included in the non-target source cell.

The targeted chromosome may include two or more ASCEs.

Here, one of the two or more ASCEs may be a first ASCE included in the first donor DNA. The other of the two or more ASCEs may be a second ASCE included in the second donor DNA.

The targeted chromosome may further include a selection marker gene and/or a transposon ITR sequence.

The targeted chromosome may further include an additional RRS.

In still another exemplary embodiment, a targeted cell having two or more targeted chromosomes may be produced using two or more donor DNAs.

The two or more donor DNAs may include a first donor DNA and a second donor DNA.

The first donor DNA may include two or more ASCEs. Here, the two or more ASCEs may include a first ASCE and a second ASCE.

The second donor DNA may include two or more ASCEs. Here, the two or more ASCEs may include a third ASCE and a fourth ASCE.

Here, all of the first ASCE, the second ASCE, the third ASCE and the fourth ASCE may be the same or different from each other. Alternatively, the first ASCE, the second ASCE, the third ASCE and the fourth ASCE may be partly the same or different from each other. For example, the first ASCE and the third ASCE may be the same, and the second ASCE and the fourth ASCE may be different from the first ASCE. Alternatively, the first ASCE and the fourth ASCE may be the same, and the second RRA and the third ASCE may be the same. Alternatively, all of the first ASCE, the second ASCE, the third ASCE and the fourth ASCE may be different from each other. Alternatively, all of the first ASCE, the second ASCE, the third ASCE and the fourth ASCE may be the same.

The first donor DNA may include a sequence including two or more homologous arms for a first non-target source chromosome. Here, the two or more homologous arm may include a first homologous arm and a second homologous arm.

Here, the first homologous arm may be a sequence homologous to a part of the sequence of the first non-target source chromosome.

Here, the second homologous arm may be a sequence homologous to a part of the sequence of the first non-target source chromosome.

Here, the first homologous arm may have a sequence different from the second homologous arm.

The second donor DNA may include a sequence including two or more homologous arms for a second non-target source chromosome. Here, the two or more homologous arms may include a third homologous arm and a fourth homologous arm.

Here, the third homologous arm may be a sequence homologous to a part of the sequence of the second non-target source chromosome.

Here, the fourth homologous arm may be a sequence homologous to a part of the sequence of the second non-target source chromosome.

Here, the third homologous arm may have a sequence different from the fourth homologous arm.

Each of the first donor DNA and the second donor DNA may further include a selection marker gene and/or a transposon ITR sequence. The description of the selection marker gene and the transposon ITR sequence is as described above.

Each of the first donor DNA and the second donor DNA may further include an additional RRS. Here, the additional RRS may be an RRS which is not used in the generation of an artificial recombinant chromosome.

The first donor DNA and the second donor DNA may be introduced into a non-target source cell. Here, the first donor DNA may have the homologous arm for the first non-target source chromosome included in the non-target source cell. The second donor DNA may have the homologous arm for a second non-target chromosome included in the non-target source cell.

The two or more targeted chromosomes may include a first targeted chromosome and a second targeted chromosome.

Here, the first targeted chromosome may include the first ASCE and the second ASCE. Here, the first ASCE and the second ASCE may be the same or different from each other.

Here, the second targeted chromosome may include the third ASCE and the fourth ASCE. Here, the third ASCE and the fourth ASCE may be the same or different from each other.

Here, each of the first targeted chromosome and the second targeted chromosome may further include a selection marker gene and/or a transposon ITR sequence.

Here, each of the first targeted chromosome and the second targeted chromosome may further include an additional RRS.

To produce two or more different targeted cells, a first donor DNA having a first ASCE may be transfected into a first non-target source cell. In addition, a second donor DNA having a second ASCE may be transfected into a second non-target source cell.

The two or more ASCEs may be ASCEs used in the generation of an artificial recombinant chromosome.

Each of the first donor DNA and the second donor DNA may include a homologous arm for a non-target source chromosome.

The first donor DNA may have the first ASCE, the second ASCE and a homologous arm for a first non-target source chromosome.

The second donor DNA may have the third ASCE, the fourth ASCE and a homologous arm for a second non-target source chromosome.

Each of the first donor DNA and the second donor DNA may further include a selection marker gene and/or a transposon ITR sequence. The description of the selection marker gene and the transposon ITR sequence is as described above.

Each of the first donor DNA and the second donor DNA may further include an additional RRS. Here, the additional RRS may be an RRS which is not used in the generation of an artificial recombinant chromosome.

In one exemplary embodiment, two or more targeted cells may be produced using two or more donor DNAs.

The two or more donor DNAs may include a first donor DNA and a second donor DNA.

The first donor DNA may include two or more ASCEs. Here, the two or more ASCEs may include a first ASCE and a second ASCE.

The second donor DNA may include two or more ASCEs. Here, the two or more ASCEs may include a third ASCE and a fourth ASCE.

Here, all of the first ASCE, the second ASCE, the third ASCE and the fourth ASCE may be the same or different from each other. Alternatively, the first ASCE, the second ASCE, the third ASCE and the fourth ASCE may be partly the same or different from each other. For example, the first ASCE and the fourth ASCE may be the same, and the second ASCE and the third ASCE may be different from the first ASCE. Alternatively, the second ASCE and the fourth ASCE may be the same, and the first RRA and the third ASCE may be the same. Alternatively, all of the first ASCE, the second ASCE, the third ASCE and the fourth ASCE may be different from each other. Alternatively, the first ASCE, the second ASCE, the third ASCE and the fourth ASCE may be the same.

The first donor DNA may include a sequence including two or more homologous arms for a first non-target source chromosome. Here, the two or more homologous arms may include a first homologous arm and a second homologous arm.

Here, the first homologous arm may be a sequence homologous to a part of the sequence of the first non-target source chromosome.

Here, the second homologous arm may be a sequence homologous to a part of the sequence of the first non-target source chromosome.

Here, the first homologous arm may have a sequence different from the second homologous arm.

The second donor DNA may include a sequence including two or more homologous arms for a second non-target source chromosome. Here, the two or more homologous arms may include a third homologous arm and a fourth homologous arm.

Here, the third homologous arm may be a sequence homologous to a part of the sequence of the second non-target source chromosome.

Here, the fourth homologous arm may be a sequence homologous to a part of the sequence of the second non-target source chromosome.

Here, the third homologous arm may have a sequence different from the fourth homologous arm.

Each of the first donor DNA and the second donor DNA may further include a selection marker gene and/or a transposon ITR sequence. The description of the selection marker gene and the transposon ITR sequence is as described above.

Each of the first donor DNA and the second donor DNA may further include an additional RRS. Here, the additional RRS may be an RRS which is not used in the generation of an artificial recombinant chromosome.

The first donor DNA may be introduced into a first non-target source cell. The second donor DNA may be introduced into a second non-target source cell. Here, the first donor DNA may have the homologous arm for the first non-target source chromosome included in the first non-target source cell. The second donor DNA may have the homologous arm for the second non-target chromosome included in the second non-target source cell.

The two or more targeted cells may include a first targeted cell and a second targeted cell.

Here, the first targeted cell may include a first targeted chromosome including the first ASCE and the second ASCE.

Here, the second targeted cell may include a second targeted chromosome including the third ASCE and the fourth ASCE.

Here, each of the first targeted chromosome and the second targeted chromosome may further include a selection marker gene and/or a transposon ITR sequence.

Here, each of the first targeted chromosome and the second targeted chromosome may further include an additional RRS.

In an example for producing a targeted cell, to produce a recipient cell, a first donor DNA and a second donor DNA may be used to produce a targeted embryonic stem cell (ESC) from ESCs.

The first donor DNA may include a first homologous arm used to target the 5' end of a variable region (including all of V segments and J segments) of an Ig light chain (IgL) locus of the genome of the ESC, a piggyBac terminal repeat (PB-TR), a promoter, loxm2/66 (first RRS), a first antibiotic-resistant gene, and a second homologous arm used to target the 5' end of the variable region of the IgL locus in the genome of the ESC.

The second donor DNA may include a third homologous arm used to target the 3' end of the variable region (including all of V segments and J segments) of the IgL locus of the genome of the ESC, a promoter, a second antibiotic-resistant gene (zeocin resistant gene), lox71 (second RRS), and a fourth homologous arm used to target the 3' end of the variable region of the IgL locus in the genome of the ESC.

A cell in which the first donor DNA is inserted into the genome of the ESC may be selected by a first antibiotic.

A cell in which the second donor DNA is inserted into the genome of the ESC may be selected using a second antibiotic.

Here, the first donor DNA and the second donor DNA may be sequentially, randomly or simultaneously introduced into the ESC.

Here, the cell with the genome into which all of the first donor DNA and the second donor DNA are inserted may be selected using a first antibiotic and a second antibiotic. The selected cell generated as described above, that is, the cell selected by the first antibiotic and the second antibiotic may be a targeted ESC.

In addition, to produce a donor cell, a third donor DNA and a fourth donor DNA for producing a targeted fibroblast from a fibroblast are used.

The third donor DNA may include a first homologous arm used to target the 5' end of a variable region (including all of V segments and J segments) of an Ig light chain (IgL) locus of the genome of the fibroblast, a promoter, a gene encoding a fluorescent protein, lox66 (third RRS), and a second homologous arm used to target the 5' end of the variable region of the IgL locus in the genome of the fibroblast.

The fourth vector may include a third homologous arm used to target the 3' end of the variable region (including all of V segments and J segments) of the IgL locus of the genome of the fibroblast, a promoter, an antibiotic-resistant gene, loxm2/71 (fourth RRS), and a fourth homologous arm used to target the 3' end of the variable region of the IgL locus in the genome of the fibroblast.

A cell in which the third donor DNA is inserted into the genome of the fibroblast may be selected by a fluorescent protein.

A cell in which the fourth donor DNA is inserted into the genome of the fibroblast may be selected by an antibiotic.

Here, the third donor DNA and the fourth donor DNA may be sequentially, randomly or simultaneously introduced into the fibroblast.

Here, the cell with the genome into which all of the third donor DNA and the fourth donor DNA are inserted may be selected using a fluorescent protein and an antibiotic. The selected cell generated as described above, that is, the cell selected by the fluorescent protein and the antibiotic may be a targeted fibroblast.

A chromosome including an Ig light chain (IgL) locus of the produced donor cell may include the first RRS and the second RRS. In addition, a chromosome including an IgL locus of the produced recipient cell may include the third RRS and the fourth RRS.

Here, the first RRS may be paired with one of the third RRS and the fourth RRS, and the second RRS may be paired with the other of the third RRS and the fourth RRS.

The examples described above are merely illustrative, and each constituent element (non-target cells, donor DNA, targeted chromosomes, and targeted cells (donor cells and recipient cells), etc.) may be modified or altered in various ways according to purpose.

### ii) Production of microcell

The "microcell" means any one of the parts separated into two or more by exposure of one cell to artificial manipulation, a specific reagent or a specific condition. In addition, the microcell means a cell analog which includes one or more chromosomes or chromosome fragments, but does not undergo somatic cell division (mitosis) or meiosis. In one example, the microcell may be any one obtained by dividing one animal cell into two or more cells or cell analogs. In another example, the microcell may be any one obtained by dividing one animal cell into the number of chromosomes, that is, 2n or more. For example, when the animal cell is a human fibroblast, the microcell may be any one obtained by dividing a human fibroblast into the number of chromosomes, that is, 2n (46) or more. In this case, the human fibroblast may be divided into 46 or more microcells.

The microcell may include one or more chromosomes or chromosome fragments.

Here, the one or more chromosomes or chromosome fragments may be a source chromosome or a source chromosome fragment.

The microcell may be a cell analog that cannot undergo normal somatic cell division or meiosis.

The microcell may have a part of the cytoplasm of a cell.

The microcell may have a part of the cell membrane of a cell.

The microcell may have a part of the nucleoplasm of a cell.

According to one aspect disclosed herein, a method of dividing a targeted cell into microcells may be provided.

The "dividing a cell into microcells" means production of microcells using one or more cells. Here, the dividing a cell into microcells is to produce one cell into multiple cells or cell analogs. The description of the microcell is as described above.

The description of the target cell is as described above.

The target cell may be a donor cell.

A method of producing a microcell from a targeted cell may use a known method. It is disclosed in the literature [Thorfinn Ege et al 1974; Thorfinn Ege et al 1977].

In one exemplary embodiment, the microcell may be produced by micronucleation for producing a micronucleated cell by treating a targeted cell with a microtubule inhibitor; and enucleation for producing a microcell by centrifugation following treatment of the micronucleated cell with a microfilament inhibitor.

Here, the targeted cell may be a donor cell.

Here, the microtubule inhibitor may be a material that inhibits the elongation of a microtubule. For example, the microtubule inhibitor may be any one or more of colchicine, nocodazole and colcemid.

Here, the microfilament inhibitor may be a material that inhibits the elongation of a microfilament. For example, the microfilament inhibitor may be cytochalasin B.

Here, the centrifugation may be performed under a Percoll gradient.

The nuclear membrane of the targeted cell may be divided into two or more small vesicles by the micronucleation.

The cell membrane of the targeted cell may be separated into two or more small vesicles by the enucleation.

The microcell formed as described above may include a targeted chromosome or a targeted chromosome fragment. Here, the targeted chromosome or targeted chromosome fragment may include at least one or more RRSs. Alternatively, the targeted chromosome or targeted chromosome fragment may include at least one or more ASCEs. Here, the RRS may be paired with an RRS located on the targeted chromosome included in a targeted cell with which the microcell is fused. Here, the ASCE may form homologous bonds with an ASCE located on the targeted chromosome included in a targeted cell with which the microcell is fused.

For example, the microcell may include a targeted chromosome including a first RRS. Here, the first RRS may be paired with a second RRS located on the targeted chromosome included in a targeted cell with which the microcell is fused.

In another example, the microcell may include a targeted chromosome including a first RRS and a second RRS. A targeted cell with which the microcell is fused may have a targeted chromosome including a third RRS and a fourth RRS. Here, the first RRS may be paired with one of the third RRS and the fourth RRS, and the second RRS may be paired with the other of the third RRS and the fourth RRS.

### ii-1) RRS-inserted chromosome-containing microcell

According to one exemplary embodiment disclosed herein, a microcell including a targeted chromosome and a method of producing the same may be provided. The targeted chromosome may include a single RRS. The targeted chromosome may include two or more RRSs.

The RRS and the targeted chromosome have been described above.

The microcell including the targeted chromosome having an RRS may be produced from a targeted cell. The targeted cell may be a targeted cell including a targeted chromosome having an RRS. The targeted chromosome having an RRS is described above.

According to an exemplary embodiment, the microcell may be produced from a targeted cell. The targeted cell may include a targeted chromosome including an RRS. Here, the targeted cell may be a donor cell.

The microcell may be produced by micronucleation for producing a micronucleated cell by treating the targeted cell including a targeted chromosome having an RRS with a microtubule inhibitor; and enucleation for producing a microcell by centrifugation following treatment of the micronucleated cell with a microfilament inhibitor.

Here, the microtubule inhibitor may be a material that inhibits the elongation of a microtubule. For example, the microtubule inhibitor may be any one or more of colchicine, nocodazole and colcemid.

Here, the microfilament inhibitor may be a material that inhibits the elongation of a microfilament. For example, the microfilament inhibitor may be cytochalasin B.

Here, the centrifugation may be performed under a Percoll gradient.

The microcell may include one or more non-target chromosomes or fragments thereof.

The microcell may include a targeted chromosome having an RRS and a fragment thereof. Here, the microcell may further include one or more non-target chromosomes or fragments thereof.

According to another exemplary embodiment, the microcell may be produced as a microcell including one chromosome or a fragment thereof. The chromosome may be a targeted chromosome having an RRS. The chromosome may be a non-target source chromosome.

The microcell including one chromosome or a fragment thereof may be produced by micronucleation for producing a micronucleated cell by treating a targeted cell including a targeted chromosome having an RRS with a microtubule inhibitor; enucleation for producing a microcell by centrifugation following treatment of the micronucleated cell with a microfilament inhibitor; and filtration of the microcell.

Here, the targeted cell may be a donor cell.

Here, the microtubule inhibitor may be a material for inhibiting the elongation of a microtubule. For example, the microtubule inhibitor may be any one or more of colchicine, nocodazole and colcemid.

Here, the microfilament inhibitor may be a material that inhibits the elongation of a microfilament. For example, the microfilament inhibitor may be cytochalasin B.

Here, the centrifugation may be performed under a Percoll gradient.

Here, the filtration may be performed using a membrane filter.

The pore size of the membrane filter may be 3 to 10 µm.

The pore size of the membrane filter may be 5 to 8 µm.

According to still another exemplary embodiment, the microcell may be produced as a microcell including two chromosomes and fragments thereof. The two chromosomes may include a targeted chromosome having an RRS and/or a non-target source chromosome.

The microcell including two chromosomes and fragments thereof may be produced by micronucleation for producing a micronucleated cell by treating a targeted cell including two or more targeted chromosomes having an RRS with a microtubule inhibitor; enucleation for producing a microcell by centrifugation following treatment of the micronucleated cell with a microfilament inhibitor; and filtration of the microcell.

Here, the targeted cell may be a donor cell.

Here, the microtubule inhibitor may be a material that inhibits the elongation of a microtubule. For example, the microtubule inhibitor may be any one or more of colchicine, nocodazole and colcemid.

Here, the microfilament inhibitor may be a material that inhibits the elongation of a microfilament. For example, the microfilament inhibitor may be cytochalasin B.

Here, the centrifugation may be performed under a Percoll gradient.

Here, the filtration may be performed using a membrane filter.

The pore size of the membrane filter may be 8 to 15 µm.

According to yet another exemplary embodiment, the microcell may be produced as a microcell including three chromosomes or fragments thereof. The three chromosomes may include a targeted chromosome having an RRS and/or a non-target source chromosome.

The microcell including three chromosomes or fragments thereof may be produced by micronucleation for producing a micronucleated cell by treating a targeted cell including two or more targeted chromosomes having an RRS with a microtubule inhibitor; enucleation for producing a microcell by centrifugation following treatment of the micronucleated cell with a microfilament inhibitor; and filtration of the microcell.

Here, the targeted cell may be a donor cell.

Here, the microtubule inhibitor may be a material that inhibits the elongation of a microtubule. For example, the microtubule inhibitor may be any one or more of colchicine, nocodazole and colcemid.

Here, the microfilament inhibitor may be a material that inhibits the elongation of a microfilament. For example, the microfilament inhibitor may be cytochalasin B.

Here, the centrifugation may be performed under a Percoll gradient.

Here, the filtration may be performed using a membrane filter.

The pore size of the membrane filter may be 12 to 20 µm.

The number of chromosomes included in the microcell is not limited to the disclosed exemplary embodiments. The number of the chromosomes included in the microcell may be selected by a membrane filter pore size.

### ii-2) ASCE-inserted chromosome-containing microcell

According to one exemplary embodiment disclosed herein, a microcell including a targeted chromosome and a method of producing the same may be provided. The targeted chromosome may include a single ASCE. The targeted chromosome may include two or more ASCEs.

The ASCE and the targeted chromosome have been described above.

The microcell including the targeted chromosome having an ASCE may be produced from a targeted cell. The target cell may be a targeted cell including a targeted chromosome having an ASCE. The targeted chromosome having an ASCE has been described above. Here, the targeted cell may be a donor cell.

According to an exemplary embodiment, the microcell may be produced from a targeted cell. The targeted cell may include a targeted chromosome having an ASCE. Here, the targeted cell may be a donor cell.

The microcell may be produced by micronucleation for producing a micronucleated cell by treating a targeted cell including a targeted chromosome having an ASCE with a microtubule inhibitor; and enucleation for producing a microcell by centrifugation following treatment of the micronucleated cell with a microfilament inhibitor.

Here, the microtubule inhibitor may be a material that inhibits the elongation of a microtubule. For example, the microtubule inhibitor may be any one or more of colchicine, nocodazole and colcemid.

Here, the microfilament inhibitor may be a material that inhibits the elongation of a microfilament. For example, the microfilament inhibitor may be cytochalasin B.

Here, the centrifugation may be performed under a Percoll gradient.

The microcell may include one or more non-target chromosomes or fragments thereof.

The microcell may include a targeted chromosome having an ASCE or a fragment thereof. Here, the microcell may further include one or more non-target chromosomes or fragments thereof.

According to another exemplary embodiment, the microcell may be produced as a microcell including one chromosome or a fragment thereof. The chromosome may be a targeted chromosome having an ASCE. The chromosome may be a non-target source chromosome.

The microcell including one chromosome or a fragment thereof may be produced by micronucleation for producing a micronucleated cell by treating a targeted cell including a targeted chromosome having an ASCE with a microtubule inhibitor; enucleation for producing a microcell by centrifugation following treatment of the micronucleated cell with a microfilament inhibitor; and filtration of the microcell.

Here, the targeted cell may be a donor cell.

Here, the microtubule inhibitor may be a material that inhibits the elongation of a microtubule. For example, the microtubule inhibitor may be any one or more of colchicine, nocodazole and colcemid.

Here, the microfilament inhibitor may be a material that inhibits the elongation of a microfilament. For example, the microfilament inhibitor may be cytochalasin B.

Here, the centrifugation may be performed under a Percoll gradient.

Here, the filtration may be performed using a membrane filter.

The pore size of the membrane filter may be 3 to 10 µm.

The pore size of the membrane filter may be 5 to 8 µm.

According to yet another exemplary embodiment, the microcell may be produced as a microcell including two chromosomes and fragments thereof. The two chromosomes may include a targeted chromosome having an ASCE and/or a non-target source chromosome.

The microcell including two chromosomes and fragments thereof may be produced by micronucleation for producing a micronucleated cell by treating a targeted cell including two or more targeted chromosomes having an ASCE with a microtubule inhibitor; enucleation for producing a microcell by centrifugation following treatment of the micronucleated cell with a microfilament inhibitor; and filtration of the microcell.

Here, the targeted cell may be a donor cell.

Here, the microtubule inhibitor may be a material that inhibits the elongation of a microtubule. For example, the microtubule inhibitor may be any one or more of colchicine, nocodazole and colcemid.

Here, the microfilament inhibitor may be a material that inhibits the elongation of a microfilament. For example, the microfilament inhibitor may be cytochalasin B.

Here, the centrifugation may be performed under a Percoll gradient.

Here, the filtration may be performed using a membrane filter.

The pore size of the membrane filter may be 8 to 15 µm.

According to an exemplary embodiment, the microcell may be produced as a microcell including three chromosomes or fragments thereof. The three chromosomes may include a targeted chromosome having an ASCE and/or a non-target source chromosome.

The microcell including three chromosomes or fragments thereof may be produced by micronucleation for producing a micronucleated cell by treating a targeted cell including two or more targeted chromosomes having an ASCE with a microtubule inhibitor; enucleation for producing a microcell by centrifugation following treatment of the micronucleated cell with a microfilament inhibitor; and filtration of the microcell.

Here, the target cell may be a donor cell.

Here, the microtubule inhibitor may be a material that inhibits the elongation of a microtubule. For example, the microtubule inhibitor may be any one or more of colchicine, nocodazole and colcemid.

Here, the microfilament inhibitor may be a material that inhibits the elongation of a microfilament. For example, the microfilament inhibitor may be cytochalasin B.

Here, the centrifugation may be performed under a Percoll gradient.

Here, the filtration may be performed using a membrane filter.

The pore size of the membrane filter may be 12 to 20 µm.

The number of chromosomes included in the microcell is not limited to the disclosed embodiments. The number of chromosomes included in the microcell may be selected by the pore size of the membrane filter.

In an example for the production of the microcell, a targeted fibroblast may be treated with colcemid. Here, a micronucleated cell may be produced according to the induction of micronucleation by the colcemid treatment.

A microcell may be isolated by treating the produced micronucleated cell with cytochalasin B, and performing centrifugation.

Through the above-described process, the microcell may be produced and obtained from a targeted fibroblast.

The examples described above are merely examples, and each constituent element (a targeted cell, a microcell or the like) may be modified or altered in various ways according to purpose.

### iii) Production of fusion cell using microcell

In one aspect disclosed herein, a first fusion cell and a method of producing the same may be provided.

The first fusion cell may be a cell additionally having one or more chromosomes or fragments thereof, in addition to a source cell.

Here, the first fusion cell may be a cell having a plurality of chromosomes greater than the total number of chromosomes that constitute the source cell. For example, when the total number of chromosomes of the source cell is 2n (40), the first fusion cell may be a cell having 2n+1 (41) chromosomes. In this case, the first fusion cell may be a cell having the total number of chromosomes (2n, that is, 40) of the source cell and one additional chromosome.

The first fusion cell may include at least one targeted chromosome. Here, the targeted chromosome may be a chromosome which is additionally included in the first fusion cell.

The first fusion cell may include at least two targeted chromosomes. Here, one of the two or more targeted chromosomes may be one of the whole chromosomes of the source cell present in the first fusion cell. The other of the two or more targeted chromosome may be a chromosome which is additionally included in the first fusion cell.

The first fusion cell may include at least two targeted chromosomes. Here, the two or more targeted chromosomes may be chromosomes additionally included in the first fusion cell. In this case, the first fusion cell may be a cell having two or more additional chromosomes in the source cell, and the first fusion cell may be a cell having the whole chromosomes of the source cell and two or more additional chromosomes.

The first fusion cell may be produced by fusion of one or more source cells and one or more microcells. A method of producing the first fusion cell by fusion of a microcell and a source cell may use a known method. It is disclosed in the literature [Fournier RE et al 1977; McNeill CA et al 1980]. As an example, Tomizuka et al., Nature Genetics, 16: 133 (1997) is referenced.

The first fusion cell may be produced by cell fusion of a targeted cell and one or more microcells.

Here, the targeted cell may be a recipient cell.

Here, the one or more microcell may be produced from a donor cell.

The descriptions of the targeted cell (a first targeted cell) and the microcell are as described above.

The microcell may be produced using a targeted cell (a second targeted cell).

Here, the first targeted cell may be derived from the same individual as the second targeted cell.

Here, the first targeted cell and the second targeted cell may be derived from different individuals. The different individuals include both homologous and heterologous ones.

For example, the first targeted cell may be a mouse embryonic stem cell (ES cell). Here, the second targeted cell may be a human fibroblast.

For example, the first targeted cell may be a mouse ES cell. Here, the second targeted cell may be a mouse fibroblast.

The microcell may include one or more targeted chromosomes or fragments thereof. Here, the one or more targeted chromosomes or fragments thereof may include one or more RRSs. Alternatively, the one or more targeted chromosomes or fragments thereof may include one or more ASCEs. Here, the one or more RRSs may be paired with one or more RRSs included in the first targeted cell. Alternatively, the one or more ASCEs may form homologous bonds with one or more ASCEs included in the first targeted cell.

When there is a plurality of the microcells, at least one microcell may include one or more targeted chromosomes or fragments thereof. Here, the one or more targeted chromosomes or fragments thereof may include one or more RRSs. Alternatively, the one or more targeted chromosomes or fragments thereof may include one or more ASCEs. Here, the one or more RRSs may be paired with one or more RRSs included in the first targeted cell. Alternatively, the one or more ASCEs may form homologous bonds with one or more ASCEs included in the first targeted cell.

In one exemplary embodiment, the first fusion cell may be produced by cell fusion of a targeted cell (a first targeted cell) and a first microcell. Here, the first microcell may include one or more targeted chromosomes or fragments thereof. Here, the one or more targeted chromosomes or fragments thereof may include one or more RRSs (a first RRS). The targeted cell (first targeted cell) may include a targeted chromosome having one or more RRSs (a second RRS). Here, the first RRS may be paired with the second RRS.

In another exemplary embodiment, the first fusion cell may be produced by cell fusion of a targeted cell (a first targeted cell) and a first microcell. Here, the first microcell may include one or more targeted chromosomes or fragments thereof. Here, the one or more targeted chromosomes or fragments thereof may include two or more RRSs (a first RRS and a second RRS). The targeted cell (first targeted cell) may include a targeted chromosome including two or more RRSs (a third RRS and a fourth RRS). Here, the first RRS may be paired with one of the third RRS and the fourth RRS, and the second RRS may be paired with the other of the third RRS and the fourth RRS.

In another exemplary embodiment, the first fusion cell may be produced by cell fusion of a targeted cell (first targeted cell), a first microcell and a second microcell. Here, the first microcell may include one or more targeted chromosomes or fragments thereof. The second microcell may include one or more non-target source chromosomes or fragments thereof. Here, the one or more targeted chromosomes or fragments thereof may include one or more RRSs (a first RRS). The targeted cell (first targeted cell) may include a targeted chromosome including one or more RRSs (a second RRS). Here, the first RRS may be paired with the second RRS.

In still another exemplary embodiment, the first fusion cell may be produced by cell fusion of a targeted cell (a first targeted cell), a first microcell and a second microcell. Here, the first microcell may include one or more targeted chromosomes or fragments thereof. The second microcell may include one or more targeted chromosomes or fragments thereof. In this case, the targeted chromosome included in the first microcell may be the same as or different from the targeted chromosome included in the second microcell. Here, the targeted chromosome included in the first microcell may include one or more RRSs (a first RRS). The targeted cell (first targeted cell) may include a targeted chromosome including one or more RRSs (a second RRS). Here, the first RRS may be paired with the second RRS.

In yet another exemplary embodiment, the first fusion cell may be produced by cell fusion of a targeted cell (a first targeted cell), a first microcell, a second microcell, a third microcell and an n^{th} microcell. Here, one or more microcells of the first microcell, the second microcell, the third microcell and the n^{th} microcell may include one or more targeted chromosomes or fragments thereof.

According to an exemplary embodiment, a method of producing the first fusion cell may include cell fusion performed by bringing a targeted cell (a first targeted cell) and one or more microcells into contact with each other; and treating the targeted cell and the microcell with a positively-charged surface active material.

The description of the targeted cell is as described above.

The targeted cell (first targeted cell) may include one or more targeted chromosomes.

The microcell may be produced using a targeted cell (a second targeted cell). The description of the microcell is as described above.

Here, the first targeted cell may be derived from the same individual as the second targeted cell.

Here, the first targeted cell and the second targeted cell may be derived from different individuals. The different individuals include both homologous and heterologous ones.

For example, the first targeted cell may be a mouse ES cell. Here, the second targeted cell may be a human fibroblast.

For example, the first targeted cell may be a mouse ES cell. Here, the second targeted cell may be a mouse fibroblast.

The microcell may include one or more targeted chromosomes or fragments thereof.

When there is a plurality of the microcells, at least one microcell may include one or more targeted chromosomes or fragments thereof.

The bringing of the targeted cell (first targeted cell) and one or more microcells into contact with each other may be to locate the targeted cell (first targeted cell) and the one or more microcells in the same medium or buffer.

The positively-charged surface active material may be polyethylene glycol (PEG).

When there is a plurality of the microcells, each of the plurality of microcells may be sequentially cell-fused with the targeted cell (first targeted cell).

When there is a plurality of the microcells, the plurality of microcells may be cell-fused with the targeted cell (first targeted cell) at one time.

When there is a plurality of the microcells, the plurality of microcells may be randomly cell-fused with the targeted cell (first targeted cell).

According to another exemplary embodiment, a method of producing the first fusion cell may include cell fusion performed by bringing a targeted cell (a first targeted cell) and one or more microcells into contact with each other; and treating the microcells and the targeted cell (first targeted cell) with a mitogen and a positively-charged surface active material.

The description of the targeted cell is as described above.

The targeted cell (first targeted cell) may include one or more targeted chromosomes.

The microcell may be produced using a targeted cell (a second targeted cell). The description of the microcell is as described above.

Here, the first targeted cell may be derived from the same individual as the second targeted cell.

Here, the first targeted cell and the second targeted cell may be derived from different individuals. The different individuals may include both homologous and heterologous individuals.

For example, the first targeted cell may be a mouse ES cell. Here, the second targeted cell may be a human fibroblast.

For example, the first targeted cell may be a mouse ES cell. Here, the second targeted cell may be a mouse fibroblast.

The microcell may include one or more targeted chromosomes or fragments thereof.

When there is a plurality of the microcells, at least one microcell may include one or more targeted chromosomes or fragments thereof.

The bringing of the targeted cell (first targeted cell) and one or more microcells into contact with each other may be to locate the targeted cell (first targeted cell) and the one or more microcells in the same medium or buffer.

The mitogen may be phytohemagglutinin-P (PHA-P).

The positively-charged surface active material may be PEG.

When there is a plurality of the microcells, each of the plurality of microcells may be sequentially cell-fused with the targeted cell (first targeted cell).

When there is a plurality of the microcells, the plurality of microcells may be cell-fused with the targeted cell (first targeted cell) at one time.

When there is a plurality of the microcells, the plurality of microcells may be randomly cell-fused with the targeted cell (first targeted cell).

### iii-1) RRS-inserted chromosome-containing first fusion cell

According to an exemplary embodiment disclosed herein, a first fusion cell including two or more targeted chromosomes and a method of producing the same may be provided.

The two or more targeted chromosomes may be targeted chromosomes each having at least one RRS.

The two or more targeted chromosomes may be a first targeted chromosome and a second targeted chromosome.

The first targeted chromosome may be a targeted chromosome including at least one RRS.

The second targeted chromosome may be a targeted chromosome including at least one RRS.

In one example, the first targeted chromosome may include a first RRS, and the second targeted chromosome may include a second RRS. Here, the first RRS may be the same as or different from the second RRS.

In another example, the first targeted chromosome may include a first RRS and a second RRS, and the second targeted chromosome may include a third RRS and a fourth RRS. Here, all of the first RRS, the second RRS, the third RRS and the fourth RRS are the same or different from each other. Alternatively, the first RRS, the second RRS, the third RRS and the fourth RRS may be partly the same or different from each other.

Here, each of the first targeted chromosome and the second targeted chromosome may further include a selection marker gene and/or a transposon ITR sequence.

The first fusion cell may include the first targeted chromosome and the second targeted chromosome.

The first targeted chromosome may be provided from a first targeted cell. The second targeted chromosome may be provided from a microcell. Here, the microcell may be produced using a second targeted cell including the second targeted chromosome. In this case, the first fusion cell may be a fusion cell having the whole chromosomes of the first targeted cell and the second targeted chromosome.

Alternatively, the first targeted chromosome may be provided from a microcell. The second targeted chromosome may be provided from the second targeted cell. Here, the microcell may be produced using the first targeted cell including the first targeted chromosome. In this case, the first fusion cell may be a fusion cell having the whole chromosomes of the second targeted cell and the first targeted chromosome.

The descriptions of the targeted chromosome, the targeted cell and the microcell are as described above.

According to an exemplary embodiment, a method of producing a first fusion cell including two or more targeted chromosomes may include cell fusion performed by bringing a first targeted cell and one or more microcells into contact with each other; and treating the first targeted cell and the microcell with a positively-charged surface active material.

The first targeted cell may include one or more targeted chromosomes.

The first targeted cell may include a first targeted chromosome.

Here, the first targeted chromosome may include at least one RRS.

Here, the one or more RRSs included in the first targeted chromosome may be one or more selected from the LoxP variants listed in Table 1.

The microcell may be produced using a second targeted cell. Here, the second targeted cell may include one or more targeted chromosomes. The second target cell may include a second targeted chromosome.

Here, the first targeted cell may be derived from the same individual as the second targeted cell.

Here, the first targeted cell and the second targeted cell may be derived from different individuals. The different individuals may include both homologous and heterologous ones.

For example, the first targeted cell may be a mouse ES cell. Here, the second targeted cell may be a human fibroblast.

For example, the first targeted cell may be a mouse ES cell. Here, the second targeted cell may be a mouse fibroblast.

The microcell may include one or more targeted chromosomes or fragments thereof. Here, the microcell may include the second targeted chromosome or a fragment thereof.

Here, the second targeted chromosome may include at least one RRS.

Here, the one or more RRSs included in the second targeted chromosome may be one or more selected from the LoxP variants listed in Table 1.

Here, the one or more RRSs included in the second targeted chromosome may be the same as or different from the one or more RRSs included in the first targeted chromosome.

Here, the one or more RRSs included in the second targeted chromosome may be paired with one or more RRSs included in the first targeted chromosome.

Here, each of the first targeted chromosome and the second targeted chromosome may further include a selection marker gene and/or a transposon ITR sequence.

The bringing of the first targeted cell and one or more microcell into contact with each other may be to locate the targeted cell and the one or more microcell in the same medium or buffer.

The positively-charged surface active material may be PEG.

In the step of treating the first targeted cell and the microcell with a positively-charged surface active material, the cells may be further treated with a mitogen.

The mitogen may be PHA-P.

According to another exemplary embodiment, a method of producing a first fusion cell including two or more targeted chromosomes may include cell fusion performed by bringing a first targeted cell and one or more microcells into contact with each other; and treating the first targeted cell and the microcell with a positively-charged surface active material.

The first targeted cell may include one or more targeted chromosomes.

The first targeted cell may include a first targeted chromosome.

The first targeted chromosome may include at least two or more RRSs.

Here, the two or more RRSs may be a first RRS and a second RRS.

Here, each of the first RRS and the second RRS may be one or more selected from the LoxP variants listed in Table 1.

Here, the first RRS may be the same as or different from the second RRS.

The microcell may be produced using a second targeted cell. Here, the second target cell may include one or more targeted chromosomes. The second target cell may include a second targeted chromosome.

Here, the first targeted cell may be derived from the same individual as the second targeted cell.

Here, the first targeted cell and the second targeted cell may be derived from different individuals. The different individuals include both homologous and heterologous ones.

For example, the first targeted cell may be a mouse ES cell. Here, the second targeted cell may be a human fibroblast.

For example, the first targeted cell may be a mouse ES cell. Here, the second targeted cell may be a mouse fibroblast.

The microcell may include one or more targeted chromosomes or fragments thereof. Here, the microcell may include the second targeted chromosome or a fragment thereof.

The second targeted chromosome may include at least two RRSs.

Here, the two or more RRSs may include a third RRS and a fourth RRS.

Here, each of the third RRS and the fourth RRS may be one or more selected from the LoxP variants listed in Table 1.

Here, the third RRS may be the same as or different from the fourth RRS.

Here, the third RRS may be paired with the first RRS and/or the second RRS.

Here, the fourth RRS may be paired with the first RRS and/or the second RRS.

Here, each of the first targeted chromosome and the second targeted chromosome may further include a selection marker gene and/or a transposon ITR sequence.

The bringing of the first targeted cell and one or more microcell into contact with each other may be to locate the targeted cell and the one or more microcell in the same medium or buffer.

The positively-charged surface active material may be PEG.

In the step of treating the first targeted cell and the microcell with a positively-charged surface active material, the cells may be further treated with a mitogen.

The mitogen may be PHA-P.

### iii-2) ASCE-inserted chromosome-containing first fusion cell

According to an exemplary embodiment disclosed herein, a first fusion cell including two or more targeted chromosomes and a method of producing the same may be provided.

The two or more targeted chromosomes may be targeted chromosomes each having at least one ASCE.

The two or more targeted chromosomes may be a first targeted chromosome and a second targeted chromosome.

The first targeted chromosome may be a targeted chromosome including at least one ASCE.

The second targeted chromosome may be a targeted chromosome including at least one ASCE.

In one example, the first targeted chromosome may include a first ASCE, and the second targeted chromosome may include a second ASCE. Here, the first ASCE may be may be the same as or different from the second ASCE.

In another example, the first targeted chromosome may include a first ASCE and a second ASCE, and the second targeted chromosome may include a third ASCE and a fourth ASCE. Here, all of the first ASCE, the second ASCE, the third ASCE and the fourth ASCE are the same or different from each other. Alternatively, the first ASCE, the second ASCE, the third ASCE and the fourth ASCE may be partly the same or different from each other.

Here, each of the first targeted chromosome and the second targeted chromosome may further include a selection marker gene and/or a transposon ITR sequence.

The first fusion cell may include the first targeted chromosome and the second targeted chromosome.

The first targeted chromosome may be provided from a first targeted cell. The second targeted chromosome may be provided from a microcell. Here, the microcell may be produced using a second targeted cell including the second targeted chromosome. In this case, the first fusion cell may be a fusion cell having the whole chromosomes of the first targeted cell and the second targeted chromosome.

Alternatively, the first targeted chromosome may be provided from a microcell. The second targeted chromosome may be provided from the second targeted cell. Here, the microcell may be produced using the first targeted cell including the first targeted chromosome. In this case, the first fusion cell may be a fusion cell having the whole chromosomes of the second targeted cell and the first targeted chromosome.

The descriptions of the targeted chromosome, the targeted cell and the microcell are as described above.

According to an exemplary embodiment, a method of producing a first fusion cell including two or more targeted chromosomes may include cell fusion performed by bringing a first targeted cell and one or more microcells into contact with each other; and treating the first targeted cell and the microcell with a positively-charged surface active material.

The first targeted cell may include one or more targeted chromosomes.

The first targeted cell may include a first targeted chromosome.

Here, the first targeted chromosome may include at least one ASCE.

The microcell may be produced using a second targeted cell. Here, the second targeted cell may include one or more targeted chromosomes. The second targeted cell may include a second targeted chromosome.

Here, the first targeted cell may be derived from the same individual as the second targeted cell.

Here, the first targeted cell and the second targeted cell may be derived from different individuals. The different individuals may include both homologous and heterologous ones.

For example, the first targeted cell may be a mouse ES cell. Here, the second targeted cell may be a human fibroblast.

For example, the first targeted cell may be a mouse ES cell. Here, the second targeted cell may be a mouse fibroblast.

The microcell may include one or more targeted chromosomes or fragments thereof. Here, the microcell may include the second targeted chromosome or a fragment thereof.

Here, the second targeted chromosome may include at least one ASCE.

Here, the one or more ASCEs included in the second targeted chromosome may be the same as or different from the one or more ASCEs included in the first targeted chromosome.

Here, the one or more ASCEs included in the second targeted chromosome may form homologous bonds with one or more ASCEs included in the first targeted chromosome.

Here, each of the first targeted chromosome and the second targeted chromosome may further include a selection marker gene and/or a transposon ITR sequence.

The bringing of the first targeted cell and one or more microcell into contact with each other may be to locate the targeted cell and the one or more microcell in the same medium or buffer.

The positively-charged surface active material may be PEG.

In the step of treating the first targeted cell and the microcell with a positively-charged surface active material, the cells may be further treated with a mitogen.

The mitogen may be PHA-P.

According to another exemplary embodiment, a method of producing a first fusion cell including two or more targeted chromosomes may include cell fusion performed by bringing a first targeted cell and one or more microcells into contact with each other; and treating the first targeted cell and the microcell with a positively-charged surface active material.

The first targeted cell may include one or more targeted chromosomes.

The first targeted cell may include a first targeted chromosome.

The first targeted chromosome may include at least two or more ASCEs.

Here, the two or more ASCEs may be a first ASCE and a second ASCE.

Here, the first ASCE may be the same as or different from the second ASCE.

The microcell may be produced using a second targeted cell. Here, the second targeted cell may include one or more targeted chromosomes. The second targeted cell may include a second targeted chromosome.

Here, the first targeted cell may be derived from the same individual as the second targeted cell.

Here, the first targeted cell and the second targeted cell may be derived from different individuals. The different individuals include both homologous and heterologous ones.

For example, the first targeted cell may be a mouse ES cell. Here, the second targeted cell may be a human fibroblast.

For example, the first targeted cell may be a mouse ES cell. Here, the second targeted cell may be a mouse fibroblast.

The microcell may include one or more targeted chromosomes or fragments thereof. Here, the microcell may include the second targeted chromosome or a fragment thereof.

The second targeted chromosome may include at least two ASCEs.

Here, the two or more ASCEs may include a third ASCE and a fourth ASCE.

Here, the third ASCE may be the same as or different from the fourth ASCE.

Here, the third ASCE may form homologous bonds with the first ASCE and/or the second ASCE.

Here, the fourth ASCE may form homologous bonds with the first ASCE and/or the second ASCE.

Each of the first targeted chromosome and the second targeted chromosome may further include a selection marker gene and/or a transposon ITR sequence.

The bringing of the first targeted cell and one or more microcell into contact with each other may be to locate the targeted cell and the one or more microcell in the same medium or buffer.

The positively-charged surface active material may be PEG.

In the step of treating the first targeted cell and the microcell with a positively-charged surface active material, the cells may be further treated with a mitogen.

The mitogen may be PHA-P.

In an example for producing the fusion cell, cell fusion may be performed by mixing a microcell and a targeted cell in a specific ratio.

Here, the specific ratio (microcell : targeted cell) may be 1:1, 1:2, 1:3, 1:4, 1:5, 1:6 1:7, 1:8, 1:9 or 1:10. Alternatively, the specific ratio may be 2:1, 3:1, 4:1, 5:1, 6:1, 7:1, 8:1, 9:1 or 10:1.

Here, the specific ratio may vary according to purpose. For example, compared to the case of producing a fusion cell having 2n+3 chromosomes, in the production of a fusion cell having 2n+1 chromosomes, the amount of a microcell base on the amount of a targeted cell may be smaller.

Here, the specific ratio may be set to any ratio in consideration of various factors including a purpose, cell fusion time, fusion cell isolation, etc.

The examples described above are merely examples, and each constituent element (targeted cell, microcell, fusion cell etc.) may be variously modified or altered according to purpose.

### iv) Production of cell including artificial recombinant chromosome using fusion cell

According to an aspect disclosed herein, a cell including an artificial recombinant chromosome and a method of producing the same may be provided.

The artificial recombinant chromosome is as described above.

The cell including the artificial recombinant chromosome may be produced using the above-described first fusion cell.

The first fusion cell may include two or more targeted chromosomes.

Here, the two or more targeted chromosomes may be a first targeted chromosome and a second targeted chromosome.

Here, each of the first targeted chromosome and the second targeted chromosome may further include a selection marker gene and/or a transposon ITR sequence.

The artificial recombinant chromosome may be produced by recombination of the first targeted chromosome and the second targeted chromosome.

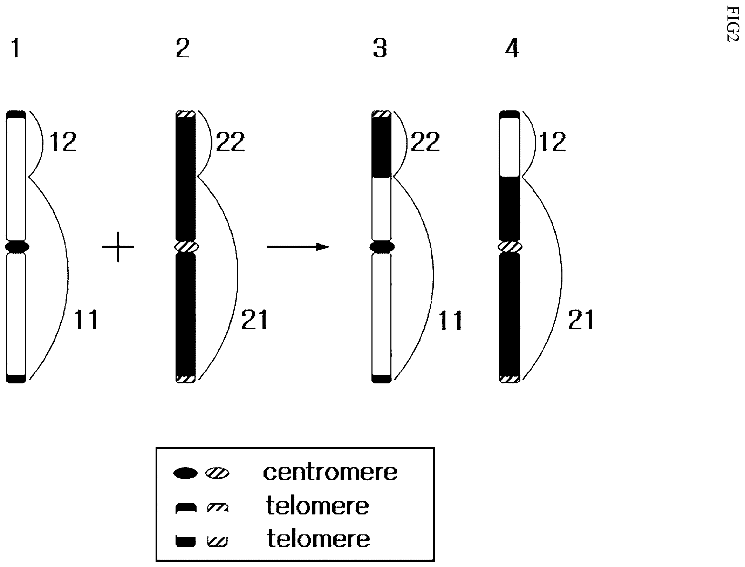

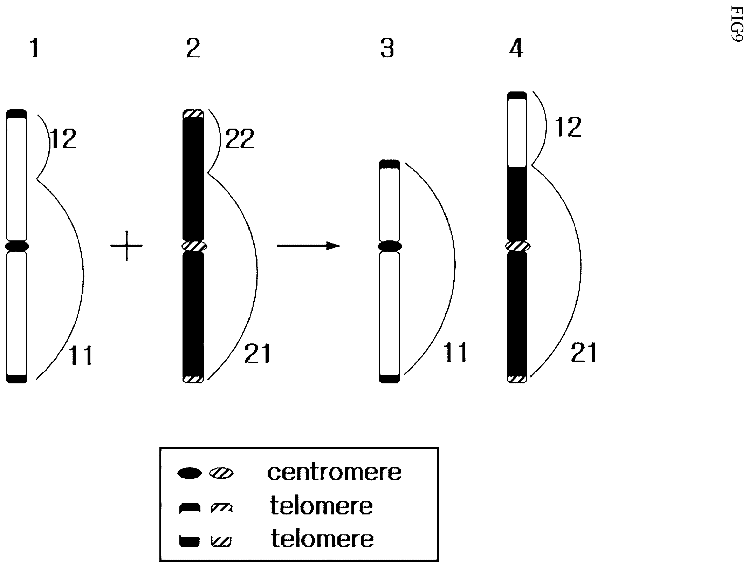

In one example, when the first targeted chromosome (1) includes a first part (11) and a first fragment (12), and the second targeted chromosome (2) includes a second part (21) and a second fragment (22), the artificial recombinant chromosome may be a first artificial recombinant chromosome (3) including the first part (11) and the second fragment (22). Alternatively, the artificial recombinant chromosome may be an artificial recombinant chromosome (4) including the second part (21) and the first fragment (12) (FIGS. 2 and 9).

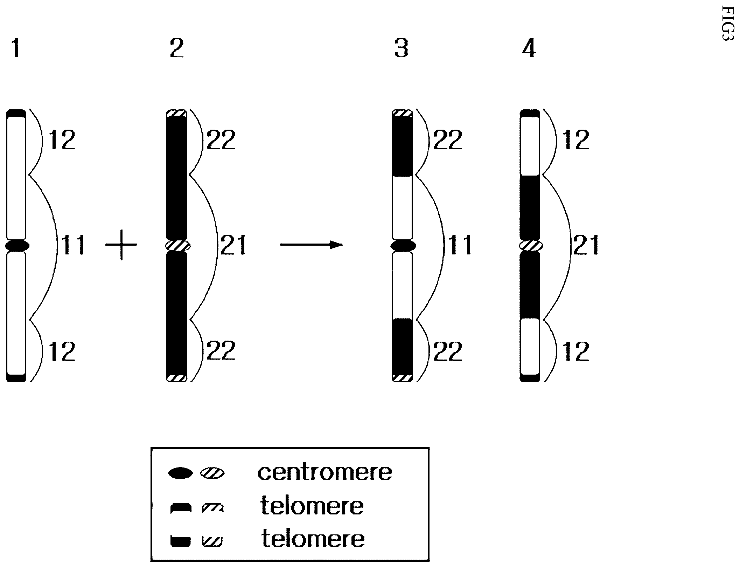

In another example, when the first targeted chromosome (1) includes a first part (11) and fragments (12) at both ends, and the second targeted chromosome (2) includes a second part (21) and fragments (22) at both ends, the artificial recombinant chromosome may be a first artificial recombinant chromosome (3) including the first part (11) and the fragments (22) at both ends of the second targeted chromosome. Alternatively, the artificial recombinant chromosome may include an artificial recombinant chromosome (4) including the second part (21) and the fragments (12) at both ends of the first targeted chromosome (FIG. 3).

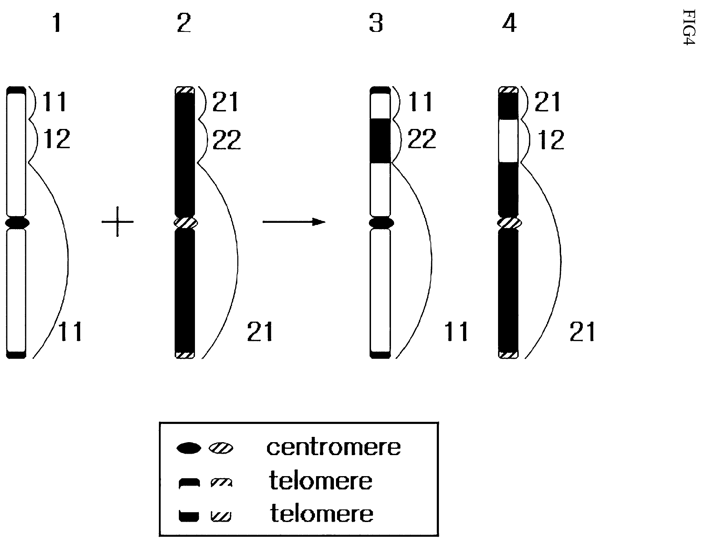

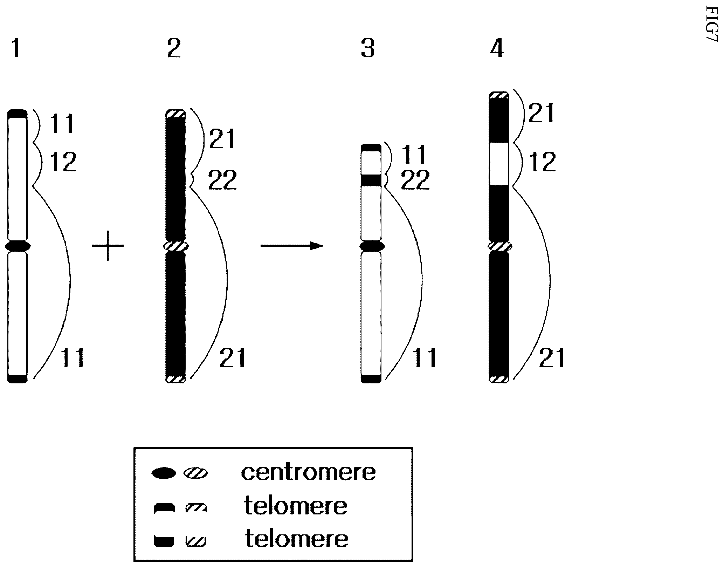

In still another example, when the first targeted chromosome (1) includes apart (11) with both ends and a first fragment (12), and the second targeted chromosome (2) includes a part (21) with both ends and a second fragment (22), the artificial recombinant chromosome may be a first artificial recombinant chromosome (3) including the part (11) with both ends of the first targeted chromosome and the second fragment (22). Alternatively, the artificial recombinant chromosome may include an artificial recombinant chromosome (4) including the part (21) with both ends of the second targeted chromosome and the second fragment (12) (FIGS. 4 and 7).

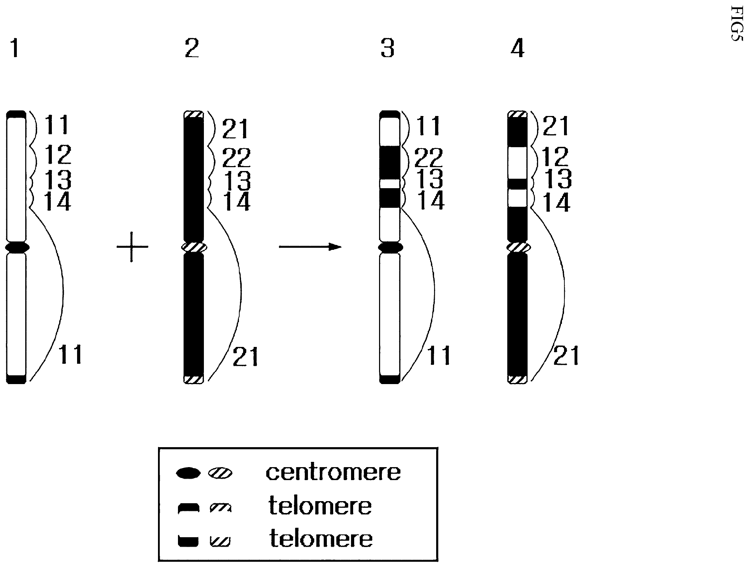

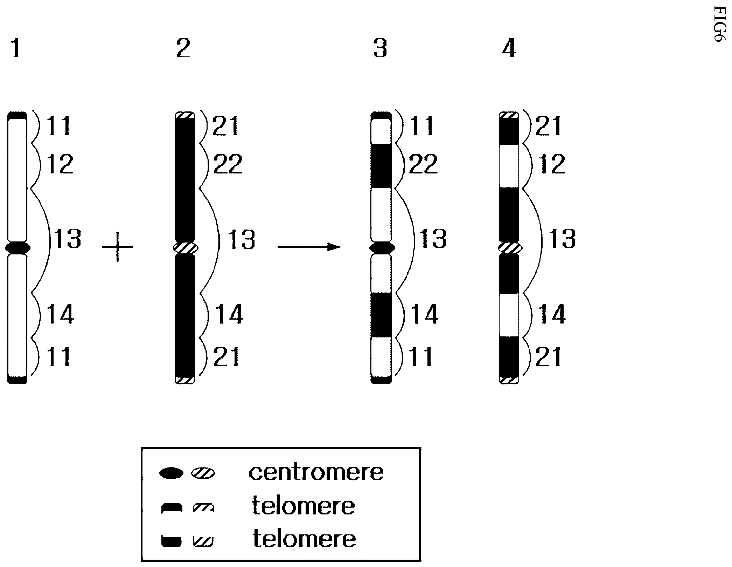

In another example, when the first targeted chromosome (1) includes a part (11) including both ends, a first part (13), a first fragment (12) and a second fragment (14), and the second targeted chromosome (2) includes a part (21) including both ends, a second part (23), a third fragment (22) and a fourth fragment (24), the artificial recombinant chromosome may a first artificial recombinant chromosome (3) including the part (11) including both ends of the first targeted chromosome, the third fragment (22), the first part (13) and the fourth fragment (24). Alternatively, the artificial recombinant chromosome may be an artificial recombinant chromosome (4) including the part (21) including both ends of the second targeted chromosome, the first fragment (12), the second part (23) and the second fragment (14) (FIGS. 5 and 6).

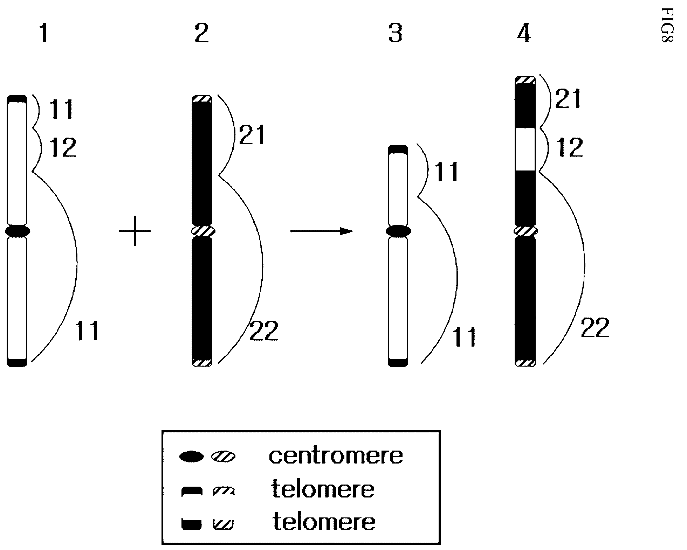

In still another example, when the first targeted chromosome (1) includes a part (11) including both ends and a first fragment (12), and the second targeted chromosome (2) includes a first part (21) and a second part (22), the artificial recombinant chromosome may be an artificial recombinant chromosome (4) including the first part (21), the first fragment (12) and the second part (22) (FIG. 8).

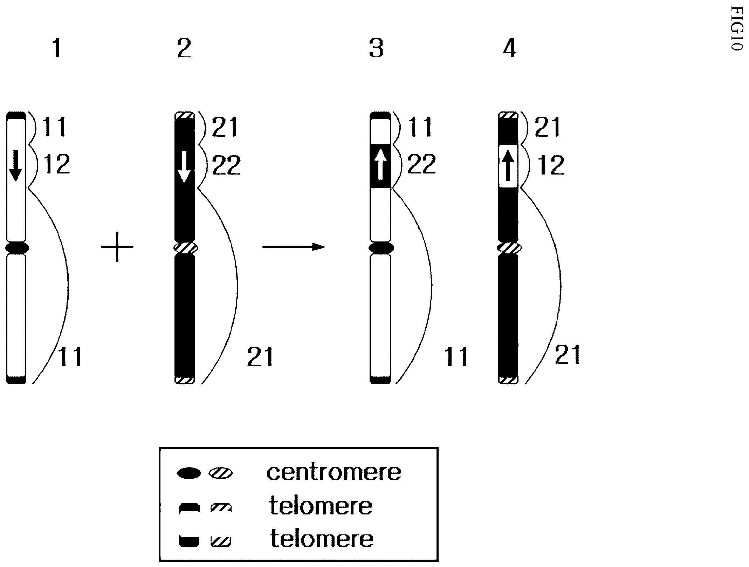

In another example, when the first targeted chromosome (1) includes a part (11) including both ends and a first fragment (12), the second targeted chromosome (2) includes a part (21) including both ends and a second fragment (22), the artificial recombinant chromosome may be a first artificial recombinant chromosome (3) including a part (11) including both ends of the first targeted chromosome and an inverted second fragment (22). Here, the inverted second fragment may be obtained by inversion of the second fragment (22) present in the second targeted chromosome (2). In this case, in a cell including the first artificial recombinant chromosome, a gene included in the inverted second fragment may not be expressed as a protein. Alternatively, a cell including the first artificial recombinant chromosome may have a different expression pattern of a gene included in the second fragment, compared to the first fusion cell including the second targeted chromosome. Alternatively, the artificial recombinant chromosome may be an artificial recombinant chromosome (4) including a part (21) including both ends of the second targeted chromosome and an inverted first fragment (12). Here, the inverted first fragment may be obtained by inversion of the first fragment (12) present in the first targeted chromosome (1). In this case, in a cell including the second artificial recombinant chromosome, a gene included in the inverted first fragment may not be expressed as a protein. Alternatively, a cell including the second artificial recombinant chromosome may have a different expression pattern of a gene included in the first fragment, compared to the first fusion cell (FIG. 10).

The artificial recombinant chromosome may further include an RRS and/or an ASCE.

The artificial recombinant chromosome may further include a selection marker gene and/or a transposon ITR sequence.

### iv-1) RRS-SSR mediated chromosome exchange

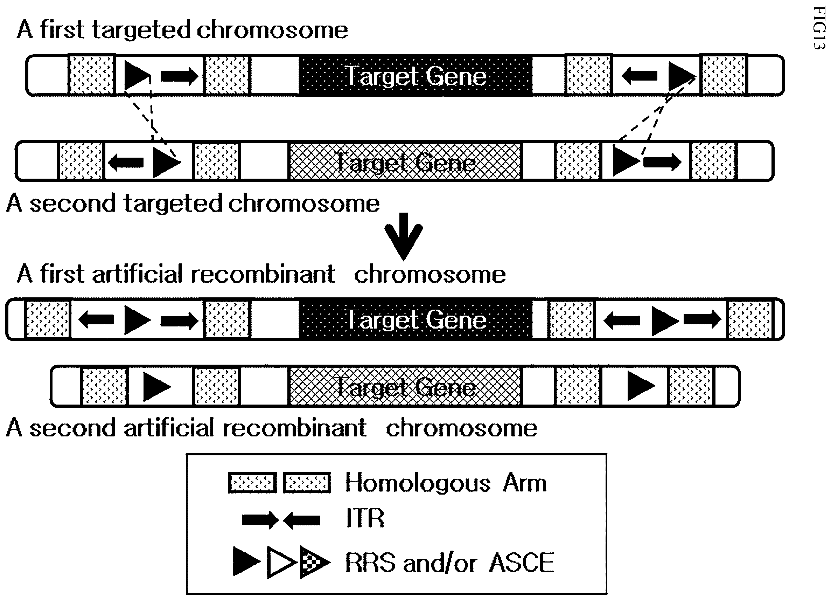

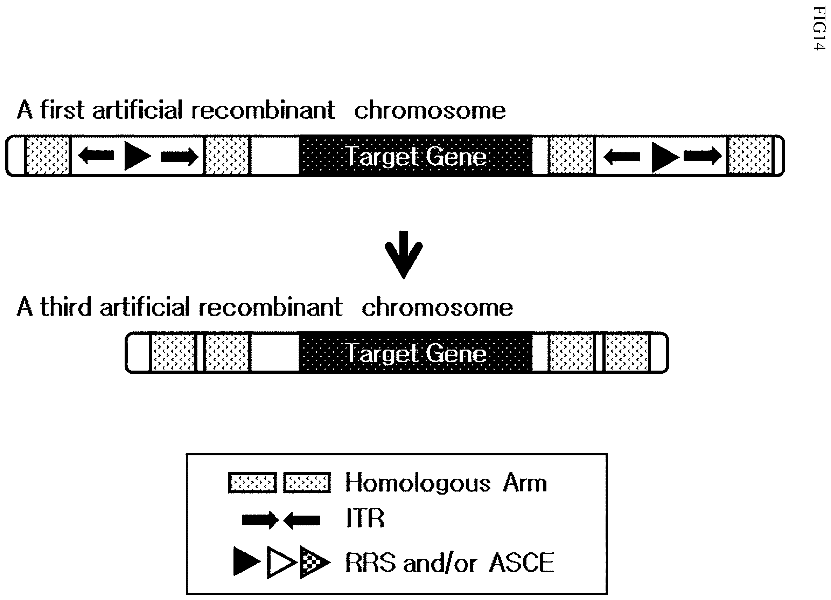

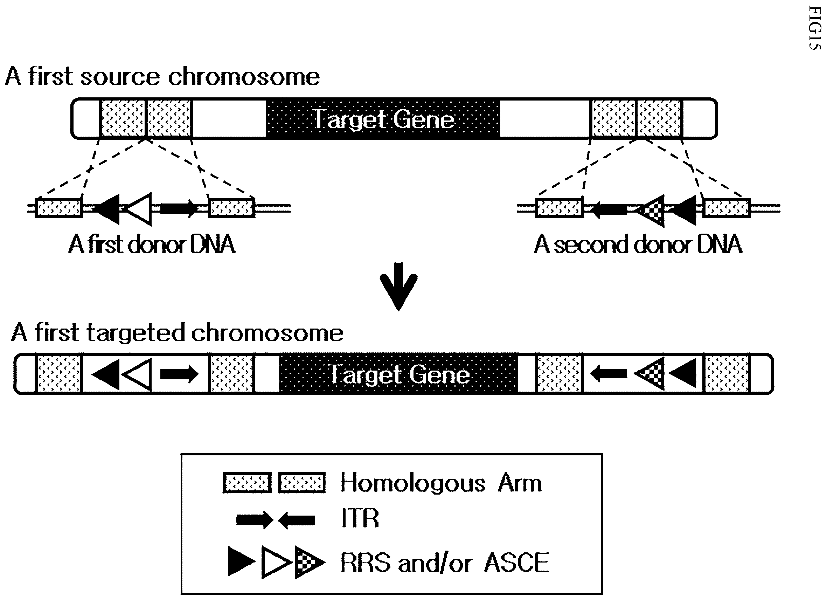

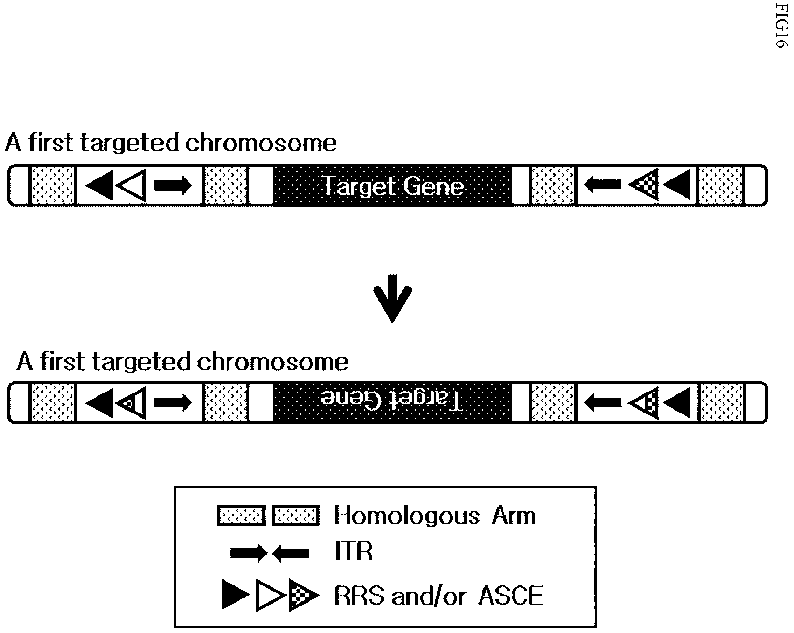

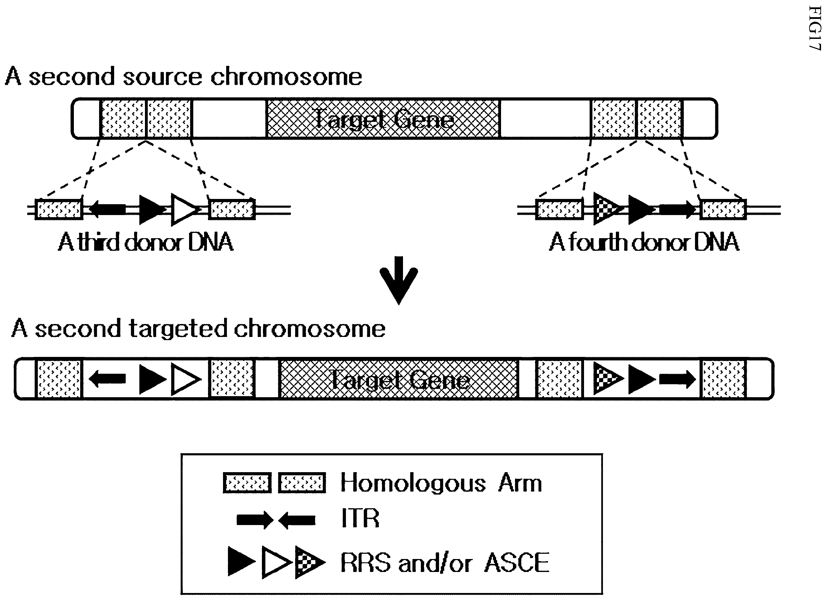

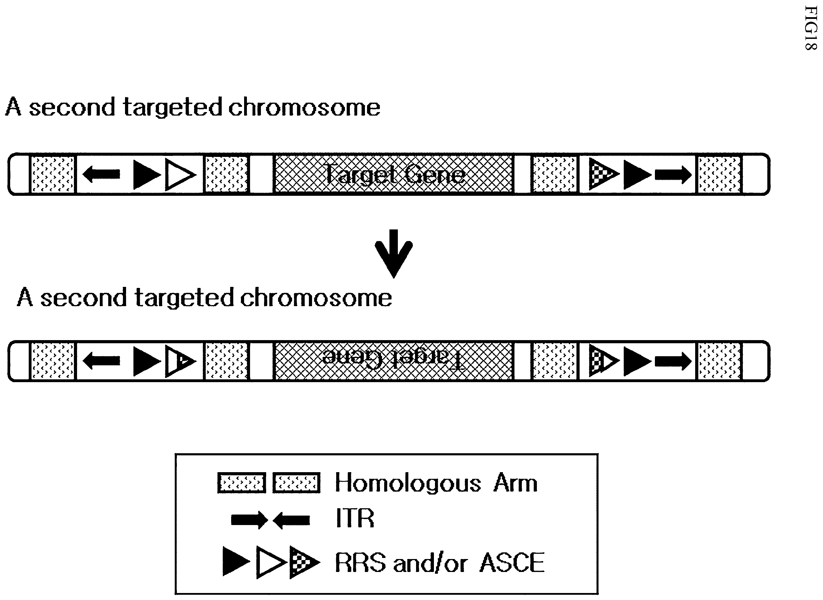

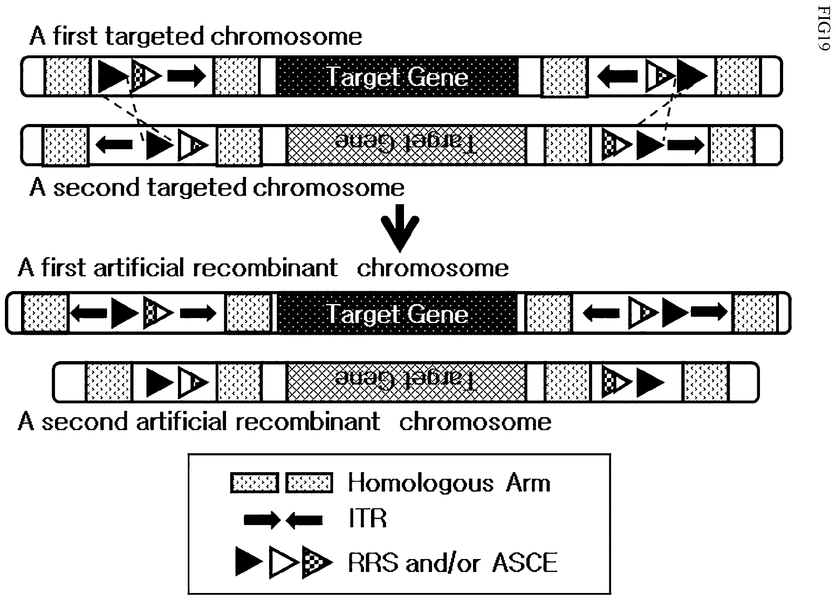

According to an exemplary embodiment disclosed herein, a cell including an artificial recombinant chromosome using an RRS-SSR-mediated chromosome exchange method and a method of producing the same may be provided (FIGS. 13 to 19).

The cell including the artificial recombinant chromosome may be produced using the above-described first fusion cell.

The first fusion cell may include two or more targeted chromosomes.

The two or more targeted chromosomes may include a first targeted chromosome and a second targeted chromosome.

Here, the first targeted chromosome may include one or more RRSs.

Here, the second targeted chromosome may include one or more RRSs.

Here, the one or more RRSs included in the first targeted chromosome may be the same as or different from the one or more RRSs included in the second targeted chromosome.

Here, the one or more RRSs included in the first targeted chromosome may be paired with the one or more RRSs included in the second targeted chromosome.

Here, the RRS may be a known sequence. In one example, the RRS may be one selected from the LoxP variants listed in Table 1.

Here, the pairing may be a pair of two or more LoxP variants, and the pairing may be recognized by an SSR.

In one example, the pair of two or more LoxP variants may include Lox 71 and Lox 66.

In one example, the pair of the two or more LoxP variants may include Lox m2/71 and Lox m2/66.

In one example, the pairing of the Lox m2/71 and the Lox m2/66 may be recognized by an SSR.

In one example, the pairing of the Lox 71 and the Lox 66 may be recognized by an SSR.

The first targeted chromosome and the second targeted chromosome may further include a selection marker gene and/or a transposon ITR sequence.

The artificial recombinant chromosome may include one or more RRSs.

The artificial recombinant chromosome may further include a selection marker gene and/or a transposon ITR sequence.

In one exemplary embodiment, the method of producing a cell including an artificial recombinant chromosome may include treating a first fusion cell with an SSR.

The description of the first fusion cell is as described above.

The first fusion cell may include two or more targeted chromosomes.

The two or more targeted chromosomes may include a first targeted chromosome and a second targeted chromosome.

Here, the first targeted chromosome may include one or more RRSs (a first RRS), a first fragment and a first part.

Here, the second targeted chromosome may include one or more RRSs (a second RRS), a second fragment and a second part.

In one example, the first RRS may be one of Lox 71 and Lox 66. Here, the second RRS may be the other of Lox 71 and Lox 66. For example, when the first RRS is Lox 71, the second RRS may be Lox 66.

In another example, the first RRS may be one of Lox m2/71 and Lox m2/66. Here, the second RRS may be the other of Lox m2/71 and Lox m2/66. For example, when the first RRS is Lox m2/71, the second RRS may be Lox m2/66.

Here, the first RRS may be paired with the second RRS.

Here, a site where the first RRS and the second RRS are paired may be recognized by an SSR.

Each of the first targeted chromosome and the second targeted chromosome may further include a selection marker gene and/or a transposon ITR sequence.

The treating the first fusion cell with an SSR may be to introduce or deliver the SSR to the first fusion cell in the form of a protein.

Here, the introduction or delivery in the form of a protein may be performed by electroporation, microinjection, transient cell compression or squeezing (e.g., described in [Lee, et al, (2012) Nano Lett., 12, 6322-6327]), lipid-mediated transfection, nanoparticles, liposomes, peptide-mediated delivery or a combination thereof.

Alternatively, the treating the first fusion cell with an SSR may be to introduce or deliver a vector having a nucleic acid sequence encoding the SSR to the first fusion cell.

Here, the vector may be a viral vector or a recombinant viral vector. The virus may be a retrovirus, a lentivirus, an adenovirus, an adeno-associated virus (AAV), a vaccinia virus, a poxvirus or a herpes simplex virus, but the present invention is not limited thereto.

Here, the introduction or delivery of the vector may be to provide the vector to the non-target source cell using a known transfection method. For example, the transfection method may use a viral transfection method, a reagent transfection method, or a physical transfection method. The viral transfection method may use, for example, a lentivirus. The reagent transfection method may use, for example, calcium phosphate, cation lipid, DEAE-dextran, or polyethylenimine (PEI). The physical transfection method may use, for example, electroporation. In addition, the transfection may use a liposome, but the present invention is not limited thereto.

The SSR may induce chromosome exchange.

In one example, the SSR may be Cre recombinase. The amino acid sequence of the Cre recombinase is disclosed in Table 3 below. However, the Cre recombinase disclosed in Table 3 below is an example of Cre recombinase for Lox 71 and Lox 66, but the present invention is not limited thereto. However, the Cre recombinase is disclosed in Table 3 below is an example of Cre recombinase for Lox m2/71 and Lox m2/66, but the present invention is not limited thereto.

**[Table 3]**

| | Amino acid sequences of Cre recombinases | |
|---|---|---|
| No. | Target | Amino acid sequence of Cre recombinase |
| 1 | Lox 71, Lox 66 | SNLLTVHQNLPALPVDATSDEVRKNLMDMFRDRQAFSEHTWKMLLSVCRS WAAWCKLNNRKWFPAEPEDVRDYLLYLQARGLAVKTIQQHLGQLNMLHR RSGLPRPSDSNAVSLVMRRIRKENVDAGERAKQALAFERTDFDQVRSLMEN SDRCQDIRNLAFLGIAYNTLLRIAEIARIRVKDISRTDGGRMLIHIGRTKTLVS TAGVEKALSLGVTKLVERWISVSGVADDPNNYLFCRVRKNGVAAPSATSQ LSTRALEGIFEATHRLIYGAKDDSGQRYLAWSGHSARVGAARDMARAGVSI PEIMQAGGWTNVNIVMNYIRNLDSETGAMVRLLEDGD (SEQ ID NO: 32) |
| 2 | Lox m2/71, Lox m2/66 | SNLLTVHQNLPALPVDATSDEVRKNLMDMFRDRQAFSEHTWKMLLSVCRS WAAWCKLNNRKWFPAEPEDVRDYLLYLQARGLAVKTIQQHLGQLNMLHR RSGLPRPSDSNAVSLVMRRIRKENVDAGERAKQALAFERTDFDQVRSLMEN SDRCQDIRNLAFLGIAYNTLLRIAEIARIRVKDISRTDGGRMLIHIGRTKTLVS TAGVEKALSLGVTKLVERWISVSGVADDPNNYLFCRVRKNGVAAPSATSQ LSTRALEGIFEATHRLIYGAKDDSGQRYLAWSGHSARVGAARDMARAGVSI PEIMQAGGWTNVNIVMNYIRNLDSETGAMVRLLEDGD (SEQ ID NO: 32) |

The cell including an artificial recombinant chromosome produced by the above-described method may include at least one or more artificial recombinant chromosomes.

Here, the one or more artificial recombinant chromosomes may be a first artificial recombinant chromosome including the first fragment and the second part. Here, the first artificial recombinant chromosome may further include the first RRS and/or the second RRS. Alternatively, the first artificial recombinant chromosome may include a third RRS. The third RRS may be an RRS produced by recombination of the first RRS and the second RRS.

Alternatively, the one or more artificial recombinant chromosomes may be a second artificial recombinant chromosome including the second fragment and the first part. Here, the second artificial recombinant chromosome may further include the first RRS and/or the second RRS. Alternatively, the second artificial recombinant chromosome may include a third RRS. The third RRS may be an RRS produced by recombination of the first RRS and the second RRS.

Here, the one or more artificial recombinant chromosomes may further include a selection marker gene and/or a transposon ITR sequence.

In another exemplary embodiment, the method of producing a cell including an artificial recombinant chromosome may include treating a first fusion cell with an SSR.

The description of the first fusion cell is as described above.

The first fusion cell may include two or more targeted chromosomes.

The two or more targeted chromosomes may include a first targeted chromosome and a second targeted chromosome.

Here, the first targeted chromosome may include two or more RRSs (a first RRS and a second RRS), a first fragment, a first part and a second part. In one example, the first targeted chromosome may consist of [first part]-[first RRS]-[first fragment]-[second RRS]-[second part].

Here, the second targeted chromosome may include two or more RRSs (a third RRS and a fourth RRS), a second fragment, a third part and a fourth part. In one example, the second targeted chromosome may consist of [third part]-[third RRS]-[second fragment]-[fourth RRS]-[fourth part].

In one example, the first RRS may be one of Lox 71 and Lox 66. Here, the third RRS or the fourth RRS may be the other of Lox 71 and Lox 66. For example, when the first RRS is Lox 71, the third RRS may be Lox 66. Alternatively, when the first RRS is Lox 71, the fourth RRS may be Lox 66.

In another example, the second RRS may be one of Lox m2/71 and Lox m2/66. Here, the third RRS or the fourth RRS may be the other of Lox m2/71 and Lox m2/66. For example, when the second RRS is Lox m2/71, the third RRS may be Lox m2/66. Alternatively, when the second RRS is Lox m2/71, the fourth RRS may be Lox m2/66.

Here, the first RRS may be paired with the third RRS or the fourth RRS.

Here, the second RRS may be paired with the third RRS or the fourth RRS.

For example, the first RRS may be paired with the third RRS, and the second RRS may be paired with the fourth RRS. Alternatively, the first RRS may be paired with the fourth RRS, and the second RRS may be paired with the third RRS.

Here, a site where the first RRS is paired with the third RRS or the fourth RRS may be recognized by an SSR.

Here, a site where the second RRS is paired with the third RRS or the fourth RRS may be recognized by an SSR.

Each of the first targeted chromosome and the second targeted chromosome may further include a selection marker gene and/or a transposon ITR sequence.

The treating the first fusion cell with an SSR may be to introduce or deliver the SSR to the first fusion cell in the form of a protein or a nucleic acid sequence encoding the SSR to the first fusion cell. Here, the description of the introduction or delivery is as described above.

The SSR may induce chromosome exchange.

In one example, the SSR may be Cre recombinase.

The cell including an artificial recombinant chromosome produced by the above-described method may include at least one or more artificial recombinant chromosomes.

Here, the one or more artificial recombinant chromosomes may be a first artificial recombinant chromosome including the third part, the first fragment and the fourth part. Here, the first artificial recombinant chromosome may further include the first RRS, the second RRS, the third RRS and/or the fourth RRS. Alternatively, the first artificial recombinant chromosome may include a fifth RRS. The fifth RRS may be an RRS produced by recombination of two paired RRSs of the first RRS, the second RRS, the third RRS and the fourth RRS.

Alternatively, the one or more artificial recombinant chromosomes may be a second artificial recombinant chromosome including the first part, the second fragment and the second part. Here, the second artificial recombinant chromosome may further include the first RRS, the second RRS, the third RRS and/or the fourth RRS. Alternatively, the second artificial recombinant chromosome may include a fifth RRS. The fifth RRS may be RRS produced by recombination of two paired RRSs of the first RRS, the second RRS, the third RRS and the fourth RRS.

Here, the one or more artificial recombinant chromosomes may further include a selection marker gene and/or a transposon ITR sequence.

In still another exemplary embodiment, the method of producing a cell including an artificial recombinant chromosome may include treating a first fusion cell with an SSR.

The description of the first fusion cell is as described above.

The first fusion cell may include two or more targeted chromosomes.

The two or more targeted chromosomes may include a first targeted chromosome and a second targeted chromosome.

Here, the first targeted chromosome may include two or more RRSs (a first RRS and a second RRS), a first fragment, a first part and a second part. In one example, the first targeted chromosome may consist of [first part]-[first RRS]-[first fragment]-[second RRS]-[second part].

Here, the first fragment may further include a third RRS and a fourth RRS. In this case, the third RRS may be paired with the fourth RRS.

Here, the second targeted chromosome may include two or more RRSs (a fifth RRS and a sixth RRS), a second fragment, a third part and a fourth part. In one example, the second targeted chromosome may consist of [third part]-[fifth RRS]-[second fragment]-[sixth RRS]-[fourth part].

Here, the second fragment may further include a seventh RRS and an eighth RRS. In this case, the seventh RRS may be paired with the eighth RRS.

In one example, the first RRS may be one of Lox 71 and Lox 66. Here, the sixth RRS may be the other of Lox 71 and Lox 66. For example, when the first RRS is Lox 71, the sixth RRS may be Lox 66.

In another example, the second RRS may be one of Lox m2/71 and Lox m2/66. Here, the fifth RRS may be the other of Lox m2/71 and Lox m2/66. For example, when the second RRS is Lox m2/71, the fifth RRS may be Lox m2/66.

Here, the first RRS may be paired with the sixth RRS.

Here, the second RRS may be paired with the fifth RRS.

Here, a site where the first RRS and the sixth RRS are paired may be recognized by an SSR.

Here, a site where the second RRS and the fifth RRS are paired may be recognized by an SSR.

Each of the first targeted chromosome and the second targeted chromosome may further include a selection marker gene and/or a transposon ITR sequence.

The treating a first fusion cell with an SSR may be to introduce or deliver the SSR to the first fusion cell in the form of a protein or a vector including a nucleic acid sequence encoding the same. Here, the description of introduction or delivery is as described above.

The SSR may induce chromosome exchange.

In one example, the SSR may be Cre recombinase.

The cell including an artificial recombinant chromosome produced by the above-described method may include at least one or more artificial recombinant chromosomes.

Here, the one or more artificial recombinant chromosomes may be a first artificial recombinant chromosome including the third part, an inverted first fragment and the fourth part. Here, the inverted first fragment may be obtained by inversion of the first fragment included in the first targeted chromosome. Here, the first artificial recombinant chromosome may further include the first RRS, the second RRS, the fifth RRS and/or the sixth RRS. Alternatively, the first artificial recombinant chromosome may further include a ninth RRS. The ninth RRS may be RRS produced by recombination of two paired RRSs of the first RRS, the second RRS, the fifth RRS and the sixth RRS.

Alternatively, here, the one or more artificial recombinant chromosomes may be a second artificial recombinant chromosome including the first part, an inverted second fragment and the second part. Here, the inverted second fragment may be obtained by inversion of the second fragment included in the second targeted chromosome. Here, the second artificial recombinant chromosome may further include the first RRS, the second RRS, the fifth RRS and/or the sixth RRS. Alternatively, the second artificial recombinant chromosome may further include a ninth RRS. The ninth RRS may be RRS produced by recombination of two paired RRSs of the first RRS, the second RRS, the fifth RRS and the sixth RRS.

Here, the one or more artificial recombinant chromosomes may further include a selection marker gene and/or a transposon ITR sequence.

In the cell including the first artificial recombinant chromosome, a gene included in the inverted first fragment may not be expressed as a protein. Alternatively, the cell including the first artificial recombinant chromosome may have a different expression pattern of the gene included in the first fragment, compared to the first fusion cell including the first targeted chromosome.

In the cell including the second artificial recombinant chromosome, a gene included in the inverted second fragment may not be expressed as a protein. Alternatively, the cell included in the second artificial recombinant chromosome may have a different expression pattern of the gene included in the second fragment, compared to the first fusion cell including the second targeted chromosome.

The cell including an artificial recombinant chromosome produced by the above-described method may be controlled in the expression of a specific gene under a specific condition.

The specific condition may be to treat the cell including an artificial recombinant chromosome with an SSR. Here, the specific gene may be a gene included in the artificial recombinant chromosome.

In one example, in the cell including the first artificial recombinant chromosome, the specific gene may be present in the inverted first fragment of a first artificial recombinant chromosome. Here, the specific gene may be present in an inverted form. In this case, the cell including the first artificial recombinant chromosome may be treated with an SSR. In the SSR-treated cell including the first artificial recombinant chromosome, the inverted first fragment may be reinverted. The reinversion may be caused by pairing of the third RRS and the fourth RRS included in the first fragment and an SSR recognizing the same. The cell including the first artificial recombinant chromosome including the reinverted first fragment may allow a specific gene to express a protein.

In another example, in the cell including the second artificial recombinant chromosome, the specific gene may be present in the inverted second fragment of the second artificial recombinant chromosome. Here, the specific gene may be present in an inverted form. In this case, the cell including the second artificial recombinant chromosome may be treated with an SSR. In the SSR-treated cell including the second artificial recombinant chromosome, the inverted second fragment may be reinverted. The reinversion may be caused by pairing of the seventh RRS and the eighth RRS included in the second fragment and an SSR recognizing the same. The cell including the second artificial recombinant chromosome including the reinverted second fragment may allow a specific gene to express a protein.

In addition, an animal including the cell including the artificial recombinant chromosome produced by the above-described method may regulate the expression of a specific gene. Here, the specific condition may be introduction or delivery of an SSR to the animal.

In addition, an animal produced using the cell including the artificial recombinant chromosome produced by the above-described method may regulate the expression of a specific gene. Here, the specific condition may be introduction or delivery of an SSR to the animal.

### iv-2) ASCE-HR mediated chromosome exchange

According to an exemplary embodiment disclosed herein, a cell including an artificial recombinant chromosome using an ASCE-HR-mediated chromosome exchange method and a method of producing the same may be provided.

The cell including the artificial recombinant chromosome may be produced using the above-described first fusion cell.

The first fusion cell may include two or more targeted chromosomes.

The two or more targeted chromosomes may include a first targeted chromosome and a second targeted chromosome.

Here, the first targeted chromosome may include one or more ASCEs.

Here, the second targeted chromosome may include one or more ASCEs.

Here, the one or more ASCEs included in the first targeted chromosome may be the same as or different from those included in the second targeted chromosome.

Here, the one or more ASCEs included in the first targeted chromosome may form homologous bonds with those included in the second targeted chromosome.

Here, the one or more ASCEs included in the first targeted chromosome and one or more ASCEs included in the second targeted chromosome may be nucleic acids including nucleotides with at least 80% or more homology.

Here, each of the first targeted chromosome and the second targeted chromosome may further include a selection marker gene and/or a transposon ITR sequence.

In one exemplary embodiment, the method of producing a cell including an artificial recombinant chromosome may include treating a first fusion cell with a factor inducing homologous recombination.

The description of the first fusion cell is as described above.

The first fusion cell may include two or more targeted chromosomes.

The two or more targeted chromosomes may be a first targeted chromosome and a second targeted chromosome.

Here, the first targeted chromosome may include one or more ASCEs (a first ASCE), a first fragment and a first part.

Here, the second targeted chromosome may include one or more ASCEs (a second ASCE), a second fragment and a second part.

Here, the first ASCE may form homologous bonds with the second ASCE.

Here, the first ASCE and the second ASCE may be nucleic acids including nucleotides with at least 80% or more homology.

Here, each of the first targeted chromosome and the second targeted chromosome may further include a selection marker gene and/or a transposon ITR sequence.

The factor inducing the homologous recombination may be a factor inducing double strand breaking (DSB) of the first targeted chromosome and/or the second targeted chromosome.

The factor inducing the homologous recombination may be a factor inducing single strand breaking (SSB) of the first targeted chromosome and/or the second targeted chromosome.

Here, the factor inducing homologous recombination may be clastogen (material that induces a chromosomal abnormality). The clastogen may be an ionizing radiation, a UV, X-rays, γ-rays, reactive oxygen species or a specific chemical. The specific chemical may be, for example, bleomycin, hydroxyurea, camptothecin, 4-nitroquinoline 1-oxide (4-NQO), cisplatin, or a methylating agent such as EMS or MMS, but the present invention is not limited thereto.

Here, the factor inducing homologous recombination may be engineered nucleases. The engineered nucleases may be Zinc-finger nucleases (ZFNs), transcription activator-like effector nucleases (TALENs) or clustered regularly interspaced short palindromic repeats/CRISPR associated protein (CRISPR/Cas).

Here, the engineered nucleases may be introduced or delivered to the first fusion cell in the form of a protein or a vector including a nucleic acid sequence encoding the same. The introduction or delivery in the form of a protein may be performed by electroporation, microinjection, transient cell compression or squeezing (e.g., described in [Lee, et al, (2012) Nano Lett., 12, 6322-6327]), lipid-mediated transfection, nanoparticles, liposomes, peptide-mediated delivery or a combination thereof. The vector may be a viral vector or a recombinant viral vector. The virus may be a retrovirus, a lentivirus, an adenovirus, an adeno-associated virus (AAV), a vaccinia virus, a poxvirus or a herpes simplex virus, but the present invention is not limited thereto. Here, the introduction or delivery in the form of a vector may be provided to the non-target source cell using a known transfection method. For example, the transfection method may be a viral transfection method, a reagent transfection method, or a physical transfection method. The viral transfection method may use, for example, a lentivirus. The reagent transfection method may use, for example, calcium phosphate, cation lipid, DEAE-dextran, or PEI. The physical transfection method may use, for example, electroporation. In addition, the transfection may use a liposome, but the present invention is not limited thereto.

The homologous recombination using DSB or SSB may induce chromosome exchange.

The cell including an artificial recombinant chromosome produced by the above-described method may include at least one or more artificial recombinant chromosomes.

Here, the one or more artificial recombinant chromosomes may be a first artificial recombinant chromosome including the first fragment and a second part. Here, the first artificial recombinant chromosome may further include the first ASCE and/or the second ASCE. Alternatively, the first artificial recombinant chromosome may include a third ASCE. The third ASCE may be an ASCE produced by recombination of the first ASCE and the second ASCE.

Alternatively, here, the one or more artificial recombinant chromosomes may be a second artificial recombinant chromosome including the second fragment and a first part. Here, the second artificial recombinant chromosome may further include the first ASCE and/or the second ASCE. Alternatively, the second artificial recombinant chromosome may include a third ASCE. The third ASCE may be an ASCE produced by recombination of the first ASCE and the second ASCE.

Here, the one or more artificial recombinant chromosomes may further include a selection marker gene and/or a transposon ITR sequence.

In another exemplary embodiment, the method of producing a cell including an artificial recombinant chromosome may include treating a first fusion cell with a factor inducing homologous recombination.

The description of the first fusion cell is as described above.

The first fusion cell may include two or more targeted chromosomes.

The two or more targeted chromosomes may include a first targeted chromosome and a second targeted chromosome.

Here, the first targeted chromosome may include two or more ASCEs (a first ASCE and a second ASCE), a first fragment, a first part and a second part. In one example, the first targeted chromosome may consist of [first part]-[first ASCE]-[first fragment]-[second ASCE]-[second part].

Here, the second targeted chromosome may include two or more ASCEs (a third ASCE and a fourth ASCE), a second fragment, a third part and a fourth part. In one example, the second targeted chromosome may consist of [third part]-[third ASCE]-[second fragment]-[fourth ASCE]-[fourth part].

Here, the first ASCE may form homologous bonds with the third ASCE or the fourth ASCE. In this case, the first ASCE may be a nucleic acid including nucleotides with at least 80% or more homology with the third ASCE or the fourth ASCE.

Here, the second ASCE may form homologous bonds with the third ASCE or the fourth ASCE. In this case, the second ASCE may include a nucleic acid including nucleotides with at least 80% or more homology with the third ASCE or the fourth ASCE.

Here, each of the first targeted chromosome and the second targeted chromosome may further include a selection marker gene and/or a transposon ITR sequence.

The factor inducing the homologous recombination may be a factor inducing DSB of the first targeted chromosome and/or the second targeted chromosome.

The factor inducing the homologous recombination may be a factor inducing SSB of the first targeted chromosome and/or the second targeted chromosome.

Here, the factor inducing homologous recombination may be clastogen (material that induces a chromosomal abnormality). The clastogen may be an ionizing radiation, a UV, X-rays, γ-rays, reactive oxygen species or a specific chemical. The specific chemical may be, for example, bleomycin, hydroxyurea, camptothecin, 4-nitroquinoline 1-oxide (4-NQO), cisplatin, or a methylating agent such as EMS or MMS, but the present invention is not limited thereto.

Here, the factor inducing homologous recombination may be engineered nucleases. The engineered nucleases may be ZFNs, TALENs or CRISPR/Cas.

Here, the engineered nucleases may be introduced or delivered to the first fusion cell in the form of a protein or a vector including a nucleic acid sequence encoding the same. The description of the induction or delivery is as described above.

The homologous recombination using DSB or SSB may induce chromosome exchange.

The cell including an artificial recombinant chromosome produced by the above-described method may include at least one or more artificial recombinant chromosomes.

Here, the one or more artificial recombinant chromosomes may be a first artificial recombinant chromosome including the third part, the first fragment and the fourth part. Here, the first artificial recombinant chromosome may further include the first ASCE, the second ASCE, the third ASCE and/or the fourth ASCE. Alternatively, the first artificial recombinant chromosome may include a fifth ASCE. The fifth ASCE may be an ASCE produced by recombination of two homologically bonded ASCEs of the first ASCE, the second ASCE, the third ASCE and the fourth ASCE.

Alternatively, here, the one or more artificial recombinant chromosomes may be a second artificial recombinant chromosome including the first part, the second fragment and the second part. Here, the second artificial recombinant chromosome may further include the first ASCE, the second ASCE, the third ASCE and/or the fourth ASCE. Alternatively, the second artificial recombinant chromosome may include a fifth ASCE. The fifth ASCE may be ASCE produced by recombination of two homologically bonded ASCEs of the first ASCE, the second ASCE, the third ASCE and the fourth ASCE.

Here, the one or more artificial recombinant chromosomes may further include a selection marker gene and/or a transposon ITR sequence.

In yet another exemplary embodiment, the method of producing a cell including an artificial recombinant chromosome may include treating a first fusion cell with a factor inducing homologous recombination.

The description of the first fusion cell is as described above.

The first fusion cell may include two or more targeted chromosomes.

The two or more targeted chromosomes may include a first targeted chromosome and a second targeted chromosome.

Here, the first targeted chromosome may include two or more ASCEs (a first ASCE and a second ASCE), a first fragment, a first part and a second part. In one example, the first targeted chromosome may consist of [first part]-[first ASCE]-[first fragment]-[second ASCE]-[second part].

Here, the first fragment may further include a third ASCE and a fourth ASCE. In this case, the third ASCE may form homologous bonds with the fourth ASCE.

Here, the second targeted chromosome may include two or more ASCEs (a fifth ASCE and a sixth ASCE), a second fragment, a third part and a fourth part. In one example, the second targeted chromosome may consist of [third part]-[fifth ASCE]-[second fragment]-[sixth ASCE]-[fourth part].

Here, the second fragment may further include a seventh ASCE and an eighth ASCE. In this case, the seventh ASCE may form homologous bonds with the eighth ASCE.

The first ASCE may form homologous bonds with the sixth ASCE. In this case, the first ASCE may be a nucleic acid having nucleotides with at least 80% or more homology with the sixth ASCE.

The second ASCE may form homologous bonds with the fifth ASCE. In this case, the second ASCE may be a nucleic acid having nucleotides with at least 80% or more homology with the fifth ASCE.

Each of the first targeted chromosome and the second targeted chromosome may further include a selection marker gene and/or a transposon ITR sequence.

The factor inducing the homologous recombination may be a factor inducing DSB of the first targeted chromosome and/or the second targeted chromosome.

The factor inducing the homologous recombination may be a factor inducing SSB of the first targeted chromosome and/or the second targeted chromosome.

Here, the factor inducing homologous recombination may be clastogen (the material that induces a chromosomal abnormality). The clastogen may be an ionizing radiation, a UV, X-rays, γ-rays, reactive oxygen species or a specific chemical. The specific chemical may be, for example, bleomycin, hydroxyurea, camptothecin, 4-nitroquinoline 1-oxide (4-NQO), cisplatin, or a methylating agent such as EMS or MMS, but the present invention is not limited thereto.

Here, the factor inducing homologous recombination may be engineered nucleases. The engineered nucleases may be ZFNs, TALENs or CRISPR/Cas.

Here, the engineered nucleases may be introduced or delivered to the first fusion cell in the form of a protein or a vector including a nucleic acid sequence encoding the same. The description of the induction or delivery is as described above.

The homologous recombination using DSB or SSB may induce chromosome exchange.

The cell including an artificial recombinant chromosome produced by the above-described method may include at least one or more artificial recombinant chromosomes.

Here, the one or more artificial recombinant chromosomes may be a first artificial recombinant chromosome including the third part, an inverted first fragment and the fourth part. Here, the inverted first fragment may be obtained by inversion of the first fragment included in the first targeted chromosome. Here, the first artificial recombinant chromosome may further include the first ASCE, the second ASCE, the fifth ASCE and/or the sixth ASCE. Alternatively, the first artificial recombinant chromosome may further include a ninth ASCE. The ninth ASCE may be ASCE produced by recombination of two paired ASCEs of the first ASCE, the second ASCE, the fifth ASCE and the sixth ASCE.

Alternatively, here, the one or more artificial recombinant chromosomes may be a second artificial recombinant chromosome including the first part, an inverted second fragment and the second part. Here, the inverted second fragment may be obtained by inversion of the second fragment included in the second targeted chromosome. Here, the second artificial recombinant chromosome may further include the first ASCE, the second ASCE, the fifth ASCE and/or the sixth ASCE. Alternatively, the second artificial recombinant chromosome may further include a ninth ASCE. The ninth ASCE may be ASCE produced by recombination of two paired ASCEs of the first ASCE, the second ASCE, the fifth ASCE and the sixth ASCE.

Here, the one or more artificial recombinant chromosomes may further include a selection marker gene and/or a transposon ITR sequence.

In the cell including the first artificial recombinant chromosome, a gene included in the inverted first fragment may not be expressed as a protein. Alternatively, the cell including the first artificial recombinant chromosome may have a different expression pattern of a gene included in the first fragment, compared to the first fusion cell including the first targeted chromosome.

In the cell including the second artificial recombinant chromosome, a gene included in the inverted second fragment may not be expressed as a protein. Alternatively, a cell including the second artificial recombinant chromosome may have a different expression pattern of a gene included in the second fragment, compared to the first fusion cell including the second targeted chromosome.

The cell including an artificial recombinant chromosome produced by the above-described method may be controlled in the expression of a specific gene under a specific condition.

The specific condition may be to treat the cell including an artificial recombinant chromosome with a factor inducing homologous recombination. Here, the specific gene may be a gene included in the artificial recombinant chromosome.

In one example, in the cell including the first artificial recombinant chromosome, the specific gene may be present in the inverted first fragment of the first artificial recombinant chromosome. Here, the specific gene may be present in an inverted form. In this case, the cell including the first artificial recombinant chromosome may be treated with a factor inducing homologous recombination. In the cell including the first artificial recombinant chromosome treated with the factor inducing homologous recombination, the inverted first fragment may be reinverted. The reinversion may be caused by homologous binding of third ASCE and fourth ASCE included in the first fragment. In the cell including the first artificial recombinant chromosome including the reinverted first fragment, the specific gene may be expressed to produce a protein.

In another example, in the cell including the second artificial recombinant chromosome, the specific gene may be present in the inverted second fragment of the second artificial recombinant chromosome. Here, the specific gene may be present in an inverted form. In this case, the cell including the second artificial recombinant chromosome may be treated with a factor inducing homologous recombination. In the cell including the second artificial recombinant chromosome treated with the factor inducing the homologous recombination, the inverted second fragment may be reinverted. The reinversion may be caused by homologous binding of seventh ASCE and eighth ASCE included in the second fragment. In the cell including the second artificial recombinant chromosome including the reinverted second fragment, the specific gene may be expressed to produce a protein.

In addition, an animal including the cell including an artificial recombinant chromosome produced by the above-described method may be controlled in the expression of a specific gene. Here, the specific condition may be to introduce or deliver a factor inducing homologous recombination to the animal.

In addition, an animal produced using the cell including an artificial recombinant chromosome produced by the above-described method may be controlled in the expression of a specific gene. Here, the specific condition may be to induce or deliver a factor inducing homologous recombination to the animal.

A method of producing an artificial recombinant chromosome disclosed herein may be referred to as artificial interspecies chromosome segment exchange (AiCE) technology. The AiCE is to exchange a first fragment of a first targeted chromosome with a second fragment of a second targeted chromosome to form an artificial recombinant chromosome. The AiCE is to exchange a first fragment of a first targeted chromosome with a third fragment of a second targeted chromosome, and exchange a second fragment of the first targeted chromosome with a fourth fragment of a third targeted chromosome to form an artificial recombinant chromosome. Here, the production of the artificial recombinant chromosome is not limited to the number of fragments. For example, a first fragment of a first targeted chromosome, a second fragment of a second targeted chromosome and a third fragment of a third targeted chromosome may be used to form an artificial recombinant chromosome. For example, a first fragment of a first targeted chromosome, a second fragment of a second targeted chromosome, a third fragment of a third targeted chromosome, and a n^{th} fragment of a n^{th} targeted chromosome may be used to form an artificial recombinant chromosome.

### iv-3) Removal of constituent element for recombination

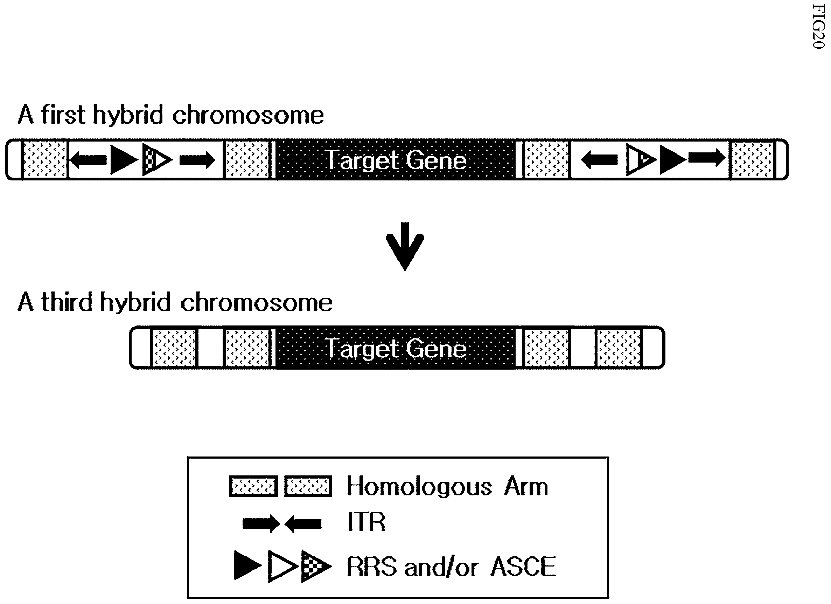

As another aspect disclosed herein, the cell including an artificial recombinant chromosome and the method of producing the same may further include removing a constituent element for recombination (FIG. 20).

The method of producing a cell including an artificial recombinant chromosome may use an RRS-SSR-mediated chromosome exchange method or an ASCE-HR-mediated chromosome exchange method.

The descriptions of the RRS-SSR-mediated chromosome exchange method and the ASCE-HR-mediated chromosome exchange method are as described above.

The method of producing a cell including an artificial recombinant chromosome may further include removing a constituent element for recombination.

Here, the constituent element for recombination may be an RRS or an ASCE.

Here, the constituent element for recombination may be included in an artificial recombinant chromosome.

The removing a constituent element for recombination may use a transposon system. The transposon system may use a method known in the art. As a known method, Fraser, MJ et al. ("Acquisition of Host Cell DNA Sequences by Baculoviruses: Relationship Between Host DNA Insertions and FP Mutants of Autographa californica and Galleria mellonella Nuclear Polyhedrosis Viruses," 1983, Journal of Virology. 47 (2): 287-300.); Sarkar, A. et al. ("Molecular evolutionary analysis of the widespread piggyBac transposon family and related "domesticated" sequences," 2003, Molecular Genetics and Genomics. 270 (2): 173-180); Bouallègue, M et al. ("Molecular Evolution of piggyBac Superfamily: From Selfishness to Domestication," 2017, Genome Biology and Evolution. 9 (2): 323-339); Grabundzija I et al. ("Comparative analysis of transposable element vector systems in human cells," 2010, Mol. Ther. 18 (6): 1200-1209); Cadiñanos, J and Bradley, A ("Generation of an inducible and optimized piggyBac transposon system," 2007, Nucleic Acids Research. 35 (12): e87); Izsvák Z and Ivics Z ("Sleeping beauty transposition: biology and applications for molecular therapy," 2004, Mol. Ther. 9 (2): 147-156); Mates L et al. ("Molecular evolution of a novel hyperactive Sleeping Beauty transposase enables robust stable gene transfer in vertebrates," 2009, Nat. Genet. 41 (6): 753-761); and Yusa, K. et al. ("A hyperactive piggyBac transposase for mammalian applications," 2011, Proceedings of the National Academy of Sciences. 108 (4): 1531-1536) may be referenced, but the present invention is not limited thereto.

The cell including an artificial recombinant chromosome produced by the above-described method (method including removing a constituent for recombination) may include at least one or more artificial recombinant chromosomes which do not include a constituent element for recombination.

Here, the cell including an artificial recombinant chromosome may include at least one or more artificial recombinant chromosomes which do not include an RRS.

Here, the cell including an artificial recombinant chromosome may include at least one or more artificial recombinant chromosomes which do not include an ASCE.

Hereinafter, for convenience of description, the artificial recombinant chromosome which does not include a constituent element for recombination is described as a final artificial recombinant chromosome.

In one exemplary embodiment, the method of producing a cell including the artificial recombinant chromosome may include:
a) treating a first fusion cell with an SSR; and
b) treating the cell (a second fusion cell) formed in a) with a transposase or a nucleic acid encoding the same.

In another exemplary embodiment, the method of producing a cell including the artificial recombinant chromosome may include:
a) treating a first fusion cell with a factor inducing homologous recombination; and
b) treating the cell (second fusion cell) formed in a) with a transposase or a nucleic acid encoding the same.

The description of a) treating a first fusion cell with an SSR and a) treating the first fusion cell formed in a) with a factor inducing homologous recombination is as described in the iv-1 and the iv-2.

The cell (second fusion cell) produced in a) may be a cell including an artificial recombinant chromosome.

Here, the artificial recombinant chromosome may be an artificial recombinant chromosome including at least one or more RRSs or ASCEs.

Here, the artificial recombinant chromosome may include at least one or more transposon ITR sequences.

Here, the artificial recombinant chromosome may further include a selection marker gene.

The second fusion cell may include one or more artificial recombinant chromosomes including at least one or more RRSs or ASCEs.

The second fusion cell may include one or more artificial recombinant chromosomes including at least one or more transposon ITR sequences.

The transposase may be Piggy Bac transposase (PB transposase) or Sleeping Beauty transposase (SB transposase).

Here, the amino acid sequence of the PB transposase is disclosed in Table 4 below. However, the PB transposase disclosed in Table 4 below may be an example, but the present invention is not limited thereto.

**[Table 4]**

| | Amino acid sequences of Piggy Bac transposases |
|---|---|
| No. | Amino acid sequence of Piggy Bac Transposase |
| 1 | MGSSLDDEHILSALLQSDDELVGEDSDSEISDHVSEDDVQSDTEEAFIDEVHEVQPTSS GSEILDEQNVIEQPGSSLASNRILTLPQRTIRGKNKHCWSTSKSTRRSRVSALNIVRSQR GPTRMCRNIYDPLLCFKLFFTDEIISEIVKWTNAEISLKRRESMTGATFRDTNEDEIYAF FGILVMTAVRKDNHMSTDDLFDRSLSMVYVSVMSRDRFDFLIRCLRMDDKSIRPTLRE NDVFTPVRKIWDLFIHQCIQNYTPGAHLTIDEQLLGFRGRCPFRMYIPNKPSKYGIKILM MCDSGTKYMINGMPYLGRGTQTNGVPLGEYYVKELSKPVHGSCRNITCDNWFTSIPL AKNLLQEPYKLTIVGTVRSNKREIPEVLKNSRSRPVGTSMFCFDGPLTLVSYKPKPAK MVYLLSSCDEDASINESTGKPQMVMYYNQTKGGVDTLDQMCSVMTCSRKTNRWPM ALLYGMINIACINSFIIYSHNVSSKGEKVQSRKKFMRNLYMSLTSSFMRKRLEAPTLKR YLRDNISNILPNEVPGTSDDSTEEPVMKKRTYCTYCPSKIRRKANASCKKCKKVICREH NIDMCQSCF(SEQ ID NO: 33) |

The treatment with the transposase or nucleic acid encoding the same may be to introduction or delivery in the form of a protein or a vector including the nucleic acid sequence encoding the same. Here, the description of introduction or delivery is as described above.

The cell including an artificial recombinant chromosome produced by the above-described method may include at least one or more final artificial recombinant chromosomes.

Here, the one or more final artificial recombinant chromosomes may be artificial recombinant chromosomes which do not include an RRS.

Here, the one or more final artificial recombinant chromosomes may be artificial recombinant chromosomes which do not include an ASCE.

Hereinafter, for convenience of description, the cell including one or more final artificial recombinant chromosomes is described as a final recombinant cell.

As an example of the method of producing a cell including an artificial recombinant chromosome using a fusion cell, a cell including an artificial recombinant chromosome may be produced by treating a fusion cell produced by cell fusion of a targeted cell including a first targeted chromosome and a microcell including a second targeted chromosome with a Cre recombinase.

Here, the fusion cell may include the first targeted chromosome and the second targeted chromosome.

Here, the first targeted chromosome may include a first RRS and a second RRS. Here, one or more genes (described as gene A below for convenience of description) may be included between the first RRS and the second RRS.

Here, the second targeted chromosome may include a third RRS and a fourth RRS. Here, one or more genes (described as gene B below for convenience of description) may be included between the third RRS and the fourth RRS.

Here, the gene A included in the first targeted chromosome may be a gene the same as or different from gene B included in the second targeted chromosome.

The first RRS present in the first targeted chromosome may be paired with the third RRS present in the second targeted chromosome, and the second RRS present in the first targeted chromosome may be paired with the fourth RRS present in the second targeted chromosome.

Alternatively, the first RRS present in the first targeted chromosome may be paired with the fourth RRS present in the second targeted chromosome, and the second RRS present in the first targeted chromosome may be paired with the third RRS present in the second targeted chromosome.

The pairing may be recognized by the Cre recombinase. As a result, recombination may be induced.

By the recombination using the pairing and the Cre recombinase, an artificial recombinant chromosome may be produced.

The artificial recombinant chromosome may be produced by exchanging the gene A of the first targeted chromosome with the gene B.

Alternatively, the artificial recombinant chromosome may be produced by exchanging the gene B of the second targeted chromosome with the gene A.

The cell including the artificial recombinant chromosome may be further treated with transposase.

Here, the transposase treatment may be to remove the RRS present in the artificial recombinant chromosome.

The examples described above are merely examples, and each constituent element (targeted chromosome, targeted cell, microcell, fusion cell, artificial recombinant chromosome, recombinase, cell having an artificial recombinant chromosome, etc.) may be modified or altered in various ways according to purpose.

### v) Selection methods

According to an aspect disclosed herein, the method of producing a cell including one or more artificial recombinant chromosomes may further include a method of selecting a specific cell.

The specific cell may be a targeted cell, a microcell, a first fusion cell, a second fusion cell and/or a final recombinant cell.

The descriptions of the targeted cell, the microcell, the first fusion cell, the second fusion cell and the final recombinant cell are as described above.

The selection method may be further added to the above-described i) to iv).

The selection method may be to select a specific cell using an inverted gene.

Here, the inverted gene may be an inverted selection marker gene. The inverted selection marker gene may be obtained by inversion of the selection marker gene. The description of the selection marker gene is as described above.

Here, the specific cell may be a targeted cell, a microcell, a first fusion cell, a second fusion cell and/or a final recombinant cell.

According to an exemplary embodiment, the specific cell may be selected by a reinverted antibiotic-resistant gene.

The antibiotic-resistant gene may be any one or more from a hygromycin-resistant gene, a neomycin-resistant gene, a kanamycin-resistant gene, a blasticidin-resistant gene, a zeocin-resistant gene and a puroΔTK gene, but the present invention is not limited thereto.

For example, when a targeted cell including a donor DNA-inserted targeted chromosome is selected, the donor DNA may include an inverted antibiotic-resistant gene and an FRT. When the targeted cell including the donor DNA-inserted targeted chromosome is treated with a recombinase, that is, flippase (FLP), the inverted antibiotic-resistant gene may be reinverted. As a result, the reinverted antibiotic-resistant gene can be normally expressed, and the targeted cell including the donor DNA-inserted targeted chromosome may be selected from the FLP-treated cells through antibiotic treatment.

According to another exemplary embodiment, the specific cell may be selected by a reinverted fluorescent protein gene.

The fluorescent protein gene may be any one or more from a GFP gene, an YFP gene, an RFP gene and an mCherry gene, but the present invention is not limited thereto.

For example, when the targeted cell including the donor DNA-inserted targeted chromosome is selected, the donor DNA may include an inverted GFP gene and an FRT. When the targeted cell including the donor DNA-inserted targeted chromosome is treated with a recombinase, that is, flippase (FLP), the inverted GFP gene may be reinverted. As a result, the reinverted GFP gene can be normally expressed, and the targeted cell including the donor DNA-inserted targeted chromosome may be selected from the FLP-treated cells through the detection of a fluorescent protein.

According to still another exemplary embodiment, the specific cell may be selected by an inverted fluorescent protein-encoding gene.

The fluorescent protein-encoding gene may be any one or more from a GFP gene, an YFP gene, an RFP gene and an mCherry gene, but the present invention is not limited thereto.

For example, when the targeted cell including the donor DNA-inserted targeted chromosome is selected, the donor DNA may include an mCherry gene and an FRT. When the targeted cell including the donor DNA-inserted targeted chromosome is treated with a recombinase, that is, flippase (FLP), the mCherry gene may be inverted. As a result, the inverted mCherry gene cannot be normally expressed, and the targeted cell including the donor DNA-inserted targeted chromosome may be selected from the FLP-treated cells through the detection of a fluorescent protein.

The selection method may be to select a specific cell using a selection marker.

Here, the selection marker may be a selection marker gene. The description of the selection marker gene is as described above.

Here, the specific cell may be a targeted cell, a microcell, a first fusion cell, a second fusion cell and/or a final recombinant cell.

According to an exemplary embodiment, the specific cell may be selected by detection of a fluorescent protein.

For the fluorescent protein detection, at least one or more chromosomes included in the specific cell may include a fluorescent protein-encoding gene.

Here, the at least one or more chromosomes may be a targeted chromosome, an artificial recombinant chromosome and/or a final recombinant chromosome. The descriptions of the targeted chromosome, the artificial recombinant chromosome and the final recombinant chromosome are as described above.

The fluorescent protein-encoding gene may be any one or more from a GFP gene, an YFP gene, an RFP gene or an mCherry gene, but the present invention is not limited thereto.

According to an exemplary embodiment, from media in which specific cells and non-target source cells are mixed, the specific cells may be selected by the detection of a fluorescent protein.

For example, when the specific cell is a targeted cell, the targeted cell may include a targeted chromosome into which a GFP gene is inserted. In this case, the specific cell, that is, the targeted cell may be selected from the non-specific source cell through the detection of green fluorescence.

From media in which two or more specific cells are mixed, one specific cell of interest may be selected by the detection of a fluorescent protein.

Here, the two or more cells may be two or more selected from a targeted cell, a microcell, a first fusion cell, a second fusion cell and a final recombinant cell.

For example, when the two or more specific cells include a first fusion cell and a second fusion cell, the second fusion cell may include an artificial recombinant chromosome into which an mCherry gene is inserted. Here, one specific cell of interest, that is, a second fusion cell may be selected from the first fusion cell through the detection of mCherry fluorescence.

According to another exemplary embodiment, the specific cell may be selected by antibiotic resistance.

For the antibiotic resistance detection, at least one or more chromosomes included in the specific cell may include an antibiotic-resistant gene.

Here, the at least one or more chromosomes may include a targeted chromosome, an artificial recombinant chromosome and/or a final recombinant chromosome. The descriptions of the targeted chromosome, the artificial recombinant chromosome and the final recombinant chromosome are as described above.

The antibiotic-resistant gene may include any one or more from a hygromycin-resistant gene, a neomycin-resistant gene, a kanamycin-resistant gene, a blasticidin-resistant gene, a zeocin-resistant gene and a puroΔTK gene, but the present invention is not limited thereto.

According to an exemplary embodiment, from media in which specific cells and non-target source cells are mixed, the specific cells may be selected by the detection of antibiotic resistance.

For example, when the specific cell is a targeted cell, the targeted cell may include a specific chromosome into which a hygromycin-resistant gene is inserted. Here, a specific cell, that is, a targeted cell may be selected from a non-specific source cell through the detection of antibiotic resistance to hygromycin.

From media in which two or more specific cells are mixed, one specific cell of interest may be selected by the detection of antibiotic resistance.

Here, the two or more specific cells may be two or more selected from a targeted cell, a microcell, a first fusion cell, a second fusion cell and a final recombinant cell.

For example, when the two or more specific cells include a first fusion cell and a second fusion cell, the second fusion cell may include an artificial recombinant chromosome into which a neomycin-resistant gene is inserted. Here, one specific cell of interest, that is, the second fusion cell may be selected from the first fusion cell through the detection of antibiotic resistance to neomycin.

According to still another exemplary embodiment, the specific cell may be selected by deadCas9-reporter (dCas9-Reporter). The reporter may include a marker. The reporter may include, for example, a fluorescent marker. The reporter may further include an aptamer and/or an agent. The aptamer and/or agent may provide binding affinity to a specific material.

The aptamer may have, for example, the binding affinity to dCas9.

The agent may have, for example, binding affinity to dCas9. The agent may be, for example, an anti-dCas9 antibody or an antibody variant. The antibody variant may include, for example, any one or more from an antigen-binding fragment (Fab), F(ab)'2, monospecific Fab2, bispecific Fab2, trispecific Fab2, monovalent Ig, a single-chain variable fragment (scFv), a bispecific diabody, single-chain variable fragment-fragment crystallizable (scFv-Fc), a minibody, an immunoglobulin new antigen receptor (IgNAR), a variable-new antigen receptor (V-NAR), heavy chain Immunoglobulin G (hcIgG) and a variable domain of a heavy chain antibody (VhH), but the present invention is not limited thereto.

For the detection of dCas9-reporter, the specific cell may be treated with gRNA or a nucleic acid encoding the same.

The gRNA may bind to a gRNA target sequence. The gRNA target sequence may include a sense sequence for gRNA. The gRNA target sequence may include an antisense sequence for gRNA. The gRNA target sequence may not be present in a non-target source chromosome, but may be selected from sequences present in a targeted chromosome and an artificial recombinant chromosome. The gRNA target sequence may be inserted into the target sequence of the non-target source chromosome by donor DNA. The non-target source chromosome and the target sequence were described above.

A candidate group of the gRNA target sequence and/or gRNA may be extracted through *in silico* design.

According to an exemplary embodiment, from media in which a specific cell and a non-target source cell are mixed, the specific cell may be selected by the detection of dCas9-reporter (dCas9-Reporter).

For example, the specific cell may include a targeted chromosome or artificial recombinant chromosome, which includes a gRNA target sequence. Here, the specific cell may be selected from non-target source cells through treatment with gRNA, dCas9 and a reporter aptamer and detection of a marker included in the reporter aptamer.

From media in which two or more specific cells mixed, one specific cell of interest may be selected by the detection of dCas9-reporter.

For example, when the two or more specific cells are the group of microcells, and the one specific cell of interest is a microcell including a targeted chromosome, the targeted chromosome may include a gRNA target sequence. Here, the microcell including a targeted chromosome may be selected from the group of microcells through treatment with gRNA, dCas9 and a reporter aptamer and detection of a marker included in the reporter aptamer.

According to another exemplary embodiment, the specific cell may be selected by fluorescence in situ hybridization (FISH). As an example of the FISH, an antibody-reporter may be used. The antibody-reporter may be used to detect a target sequence using a chromosome-specific condensation structure and/or the binding affinity of an antibody to a specific sequence of a chromosome. The FISH using the antibody-reporter may use a known method.

The specific cell may include a chromosome including an antibody target sequence.

Here, the chromosome including the antibody target sequence may include a targeted chromosome, an artificial recombinant chromosome and/or a final recombinant chromosome. The descriptions of the targeted chromosome, the artificial recombinant chromosome and the final recombinant chromosome are as described above.

For the FISH detection, the specific cell may be treated with the antibody-reporter. The antibody-reporter may mean a construct in which a primary antibody and a reporter are connected. In addition, the antibody-reporter may mean a construct in which a secondary antibody having binding affinity to one region of the primary antibody and a reporter are connected. In this case, the treatment with the antibody-reporter may include providing a secondary antibody-reporter construct in time series after the primary antibody treatment.

The antibody may bind to an antibody target sequence on the chromosome.

The candidate group of the antibody target sequences and/or antibodies may be extracted through *in silico* design.

According to an exemplary embodiment, from media in which two or more specific cells are mixed, one specific cell of interest may be selected by FISH detection.

Here, the two or more specific cells may be two or more selected from a targeted cell, a microcell, a first fusion cell, a second fusion cell and a final recombinant cell.

For example, when the two or more specific cells are a first fusion cell and a second fusion cell, the second fusion cell may include an artificial recombinant chromosome including an antibody target sequence. Here, a second fusion cell may be selected from a first fusion cell through treatment of antibody-reporter and detection of a marker included in the reporter. The antibody may bind to an antibody target sequence. The antibody target sequence is one region of the artificial recombinant chromosome.

### One aspect of the disclosure in the specification relates to a method of producing an animal using a cell including one or more artificial recombinant chromosomes.

The method of producing an animal may use a cell including one or more artificial recombinant chromosomes.

The animal may be a non-human animal.

Here, the non-human animal may be a mouse, a rat, a rabbit, a goat, sheep, a pig, a cow, a horse or a monkey, but the present invention is not limited thereto.

The animal may be a transgenic (transformed) non-human animal.

Here, the transformation may be caused by one or more artificial recombinant chromosomes.

Here, the transgenic (transformed) non-human animal may have one or more different phenotypes, compared to a wild-type non-human animal. The one or more different phenotypes may be caused by the one or more artificial recombinant chromosomes.

The description of the cell including the one or more artificial recombinant chromosomes is as described above.

The cell including the one or more artificial recombinant chromosomes may be a second fusion cell or a final recombinant cell.

The description of the second fusion cell and the final recombinant cell is as described above.

### Somatic cell nuclear transfer (SCNT)

According to an exemplary embodiment disclosed herein, an offspring produced from the second fusion cell or the final recombinant cell is provided.

According to another exemplary embodiment disclosed herein, a method of producing an offspring from the second fusion cell or the final recombinant cell is provided.

The offspring produced from the second fusion cell or the final recombinant cell may be produced by SCNT. The SCNT may be used to obtain a donor nucleus from the second fusion cell or the final recombinant cell, to produce a cloned egg by transplanting the donor nucleus into a enucleated egg, and to generate an offspring by transplanting the cloned egg into the uterus of a surrogate, and the SCNT may use a known method. As a known method, Campbell KH et al. ("Sheep cloned by nuclear transfer from a cultured cell line," 1996, Nature. 380 (6569): 64-6); Gupta, M. K et al. ("Transgenic Chicken, Mice, Cattle, and Pig Embryos by Somatic Cell Nuclear Transfer into Pig Oocytes," 2013, Cellular Reprogramming. 15 (4): 322-328); or Li, J et al. ("Human embryos derived by somatic cell nuclear transfer using an alternative enucleation approach," 2009, Cloning and Stem Cells. 11 (1): 39-50) may be referenced, but the present invention is not limited thereto.

### Development from embryo

According to an exemplary embodiment disclosed herein, an offspring produced from the second fusion cell or the final recombinant cell is provided.

According to another exemplary embodiment disclosed herein, a method of producing an offspring from the second fusion cell or the final recombinant cell is provided.

The offspring from the second fusion cell or the final recombinant cell may be produced through embryonic development. The embryonic development is to develop an offspring from an embryo by implanting the embryo in the uterus of a surrogate, and may be used by a known method.

Here, the embryo may be the second fusion cell or the final recombinant cell.

### Blastocyst injection

According to an exemplary embodiment disclosed herein, an offspring produced from the second fusion cell or the final recombinant cell is provided.

According to another exemplary embodiment disclosed herein, a method of producing an offspring from the second fusion cell or the final recombinant cell is provided.

The offspring produced from the second fusion cell or the final recombinant cell may be produced by blastocyst injection. The blastocyst injection may be used to develop an offspring by transplanting a gene-manipulated embryonic stem cell (ES cell) in a blastula stage, thereby obtaining a chimeric blastocyst, and implanting the chimeric blastocyst in the uterus of a surrogate, and may use a known method.

Here, the gene-manipulated ES cell may be the second fusion cell or the final recombinant cell.

### [Examples]

Hereinafter, the present invention will be described in further detail with reference to examples.

The examples are merely provided to more fully describe the present invention, and it will be obvious to those of ordinary skill in the art that the scope of the present invention is not limited to the following examples.

### Example 1. Production of artificial recombinant chromosome using single RRS and transgenic animal using the same

This example is a specific experimental example for proving a transformation-introducing method using a chromosome disclosed herein, and relates to a cell having an artificial recombinant chromosome in which a target gene is inserted into a specific site through the recombination between chromosomes and a method of producing a transgenic mouse using the same. The following description provides overall examples regarding a cell in which a fluorescent protein-encoding gene is inserted into the end of the variable region of an immunoglobulin heavy (IgH) locus using a single RRS and the production of a transgenic mouse using the same, which are merely examples using an artificial recombinant chromosome, but the present invention is not limited thereto. The artificial recombinant chromosome of interest may be produced by modifying examples to be described below in various ways, or adding various methods other than the examples to be described below.

### Example 1-1. Vector construction for producing targeted cell

A first DNA donor (a first vector) was designed to produce a mouse embryonic stem cell (mESC) as a targeted mESC, and a second DNA donor (a second vector) was designed to produce a human fibroblast as a targeted human fibroblast.

A taq used in a PCR reaction was a general PCR tag, which is GoTaq G2 green (Promega, USA), and PrimeSTAR (Takara, Japan) was used as a tag for blunt-end production, and SimpliAmp (Thermo Fisher Scientific, USA) was used as a thermocycler. A T-blunt vector (Solgent, Korea) was used as a T-vector used in clone production and DNA sequencing, and a HIT competent cell (RBC Bioscience, USA) was used as a competent cell. All restriction enzymes used in DNA recombination were purchased from New England Biolabs (NEB), and a ligase used in DNA ligation was a T4 DNA ligase (Takara, Japan).

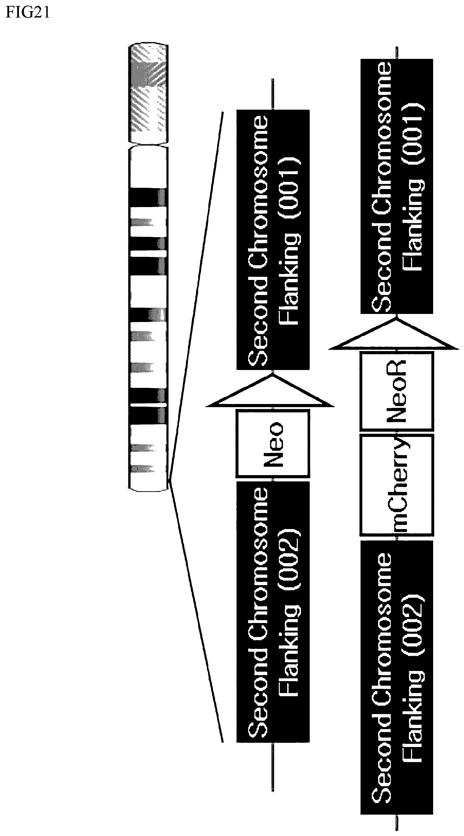

The first DNA donor (first vector) to be used in mouse ESC targeting consists of a flanking region sequence (M002) to be used in 5' end targeting of the variable region of a mouse IgH locus (IgHV), a neomycin-resistant gene (NeoR), loxp, a flanking region sequence (M001) to be used in the 5' end targeting of mouse IgHV, a TK gene used in negative selection, an ampicillin-resistant gene (AmpR) to be used in bacteria-positive selection and a replication origin. In the case of NeoR, a pCMV6-AC-GFP vector was amplified as a template by an overhang-PCR method using a SV40 promoter forward gene specific primer (GSP) including an EcoRI site (Table 5. SEQ ID NO: 1), and bridge including a loxP sequence and a SalI site, reverse primers (Table 5. SEQ ID NOs: 2, 3). PCR amplification was performed with J1 mouse genomic DNA as a template with respect to M001 and M002 using GSP having SalI and XhoI sites (Table 5. SEQ ID NOs: 4 and 5) and GSP having BamHI and EcoRI sites (Table 5, SEQ ID NOs: 6, 7). All PCR products were cloned after ligation to a T-blunt vector, and then their DNA base sequences were confirmed. Plasmids obtained from all clones were cleaved using restriction enzymes acting on restriction sites at both ends, and ligated to a BamHI and XhoI-treated pOSdupdel vector using a T4 DNA ligase (FIG. 21).

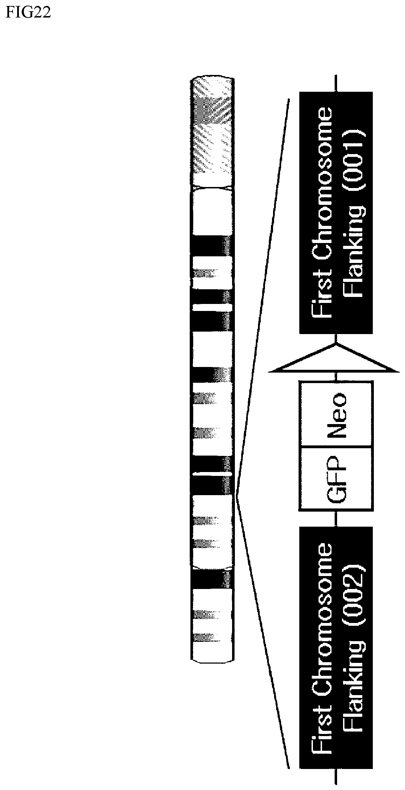

The second DNA donor (second vector) to be used in human fibroblast targeting consists of a flanking region sequence (H002) to be used in the 5' end targeting of the variable region of a human IgH locus (IgHV), turboGFP-NeoR, loxP, a flanking region sequence (H001) to be used in 5' end targeting of human IgHV, a TK gene to be used in negative selection, AmpR to be used in bacterial positive selection and a replication origin. In the case of turboGFP-NeoR, to remove a multi cloning site (MCS) of a template vector (pCMV6-AC-GFP), a CMV promoter and PCR products of the turboGFP-NeoR were subjected to blunt end ligation. The CMV promoter was subjected to overhang-PCR using forward GSP (Table 5. SEQ ID NO: 8) having an HindIII site and PrimeSTAR as reverse GSP (Table 5. SEQ ID NO: 9), and the turboGFP-NeoR was subjected to overhang-PCR using forward GSP (Table 5. SEQ ID NO: 10) and PrimeSTAR as reverse primers (Table 5. SEQ ID NO: 2, 3) having a loxP sequence and a SalI site. PCR amplification was performed with human fibroblast genomic DNA as a template with respect to H001 and H002 using GSP (Table 5. SEQ ID NOs: 11, 12) having SalI and XhoI sites and GSP (Table 5. SEQ ID NO: 13, 14) having BamHI and HindIII sites. All PCR products were cloned after ligation to a T-blunt vector, and then their DNA base sequences were confirmed. Plasmids obtained from all clones were cleaved using restriction enzymes acting on restriction sites at both ends, and ligated to a BamHI and XhoI-treated pOSdupdel vector using a T4 DNA ligase (FIG. 22).

**[Table 5]**

| | Primers used in vector construction and DNA sequences thereof | |
|---|---|---|
| SEQ ID NO: | Primer | DNA sequence |
| 1 | Neo^{R}-EcoRI-forward | gaattcGGCTGTGGAATGTGTGTCAGTTAGGGTG |
| 2 | Neo^{R}-loxp-bridge | CTTATCATGTCTGTATACCGTCGcgccaccataacttcgtatagcatacattatac gaagttatcggtcgacgtcgg |
| 3 | Neo^{R}-SalI-reverse | CCGACGTCGACCGATAACTT |
| 4 | M001-Sa1I-forward | ACGCgtcgacAGGATTTGGACCTGAGCATACT |
| 5 | M001-XhoI-reverse | CCGctcgagGAGGCCAAGAGAGGCTAAAGCC |
| 6 | M002-BamHI-forward | CGCggatccCATTCTCCCATCTCCAATTTAT |
| 7 | M002-EcoRI-reverse | GgaattcTTTTGTAACCCCTAGACAGATG |
| 8 | CMV-HindIII-forward | aagcttCCGCCATGTTGACATTG |
| 9 | CMV-reverse | CGGCCGCCCTATAGTG (5' -phosphorylated) |
| 10 | GFP-Neo-forward | AGATGGAGAGCGACGAGAGCGGCCT (5'-phosphorylated) |
| 11 | H001-Sa1I-forward | ACGCgtcgacTGCGTGAGATCTTTTCTTGGGG |
| 12 | H001-XhoI-reverse | CCGctcgagTCCACACACCCAAGTCATTCGA |
| 13 | H002-BamHI-forward | CGCggatccCTGAAGCCAACCAAGTTTAGGA |
| 14 | H002-HindIII-reverse | CCCaagcttCACATGGTGAACCCAAACACTC |
| 15 | CMV-XhoI-forward | ATCctcgagGACATTGATTATTGACTAG |
| 16 | CMV-KpnI-reverse | ATTggtaccCTCGGCCGCCCTATAG |
| 17 | Hygro-loxm2/71-KpnI-forward | ATAggtaccTACCGTTCGTATATGGTTTCTTATACGAAGTTAT GAATTCCACCATGAAAAAGCCTGAACTCAC |
| 18 | Hygro-lox66-SalI - reverse | ATCgtcgacTACCGTTCGTATAATGTATGCTATACGAAGTTAT GGATCCTAAGATACATTGATG |
| 19 | PuroΔTK-lox71-XhoI-forward | ATTctcgagATAACTTCGTATAATGTATGCTATACGAACGGT AATCGATCCCCAGCATGCCTGCTATTGTCTTC |
| 20 | PuroΔTK-loxm2/66-HindIII-reverse | CTCaagcttATAACTTCGTATATGGTTTCTTATACGAACGGT ACTTAAGCACCATGGGGACCGAGTACAAGCCCAC |
| 21 | Neo-HindIII-forward | CGCaagcttGTGTGTCAGTTAGGGTGTG |
| 22 | Neo-SalI-reverse | ATCgtcgacTAAGATACATTGATGAGTTTG |

### Example 1-2. Production of cell including single RRS-inserted chromosome

### Example 1-2-1. Production of targeted human cell including loxP-inserted human chromosome

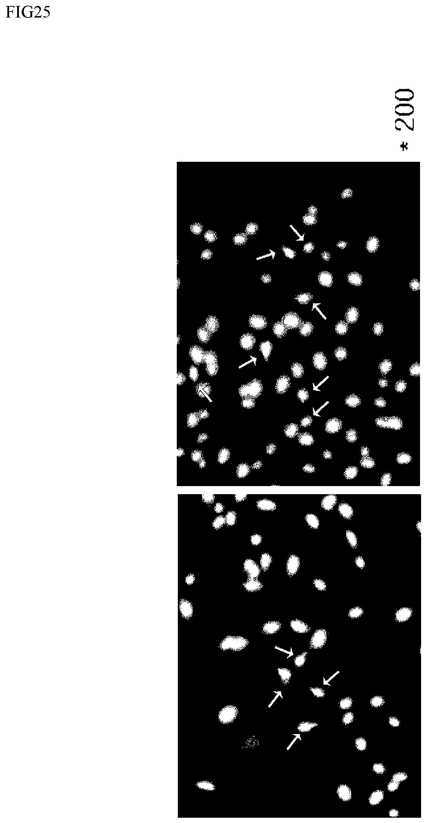

Human dermal fibroblasts (hDFs) used herein were purchased from Cell Engineering for Origin (CEFO; Seoul, Korea). As a basic medium for cell proliferation and maintenance, a Dulbecco's Modified Eagle's Medium (DMEM; Corning, Mannasas, VA, USA) supplemented with a 10% fetal bovine serum (FBS; Corning, Mannasas, VA, USA) and 1% penicillin-streptomycin (Corning, Mannasas, VA, USA) was used, and the cells were incubated in an incubator maintained at 5% CO₂, 95% humidity and 37 °C. Transient transfection was performed using Lipofectamine 3000 (Invitrogen, Carlsbad, CA, USA). The transfection was performed by adding 1x10⁶ cells to FBS and antibiotic-free DMEM, adding 10 µg of the second DNA donor (second vector) single-cut with a NotI enzyme (New England Biolabs, Ipswich, MA, USA) thereto, mixing a Lipofectamine 3000 reagent therewith, and incubating the cells at room temperature for 5 minutes. The cells were incubated for 24 to 48 hours in an incubator maintained at 5% CO₂, 95% humidity and 37 °C (FIG. 25).

To confirm whether the second DNA donor (second vector) is inserted into the human DF, a cell was selected using an antibiotic-resistant gene present in a second DNA donor (second vector). A cell was selected through the expression of a neomycin-resistant gene present in the second DNA donor (second vector) inserted into the human DF. The antibiotic used herein was G418 (Life Technologies, NY, USA), and a cell selection concentration of G418 was measured using a cell counting kit-8 (CCK-8; Dogindo, Kumamoto, Japan). The hDF was transfected with the second DNA donor (second vector), and 48 hours later, treated with 400 µg/ml of G418. The cell selection process was performed for 4 to 6 weeks, and the formed loxP-inserted clone was incubated by fulling. The selected hDF was confirmed through GFP expression using a fluorescence microscope (Olympus Corporation, Tokyo, Japan) (FIG. 25).

As a result, the expression of GFP tagged to the second DNA donor (second vector) in the selected human dermal fibroblast was confirmed. Based on this, it can be confirmed that the second DNA donor (second vector) was inserted into the human dermal fibroblast.

### Example 1-2-2. Production of targeted mouse cell including loxP-inserted mouse chromosome

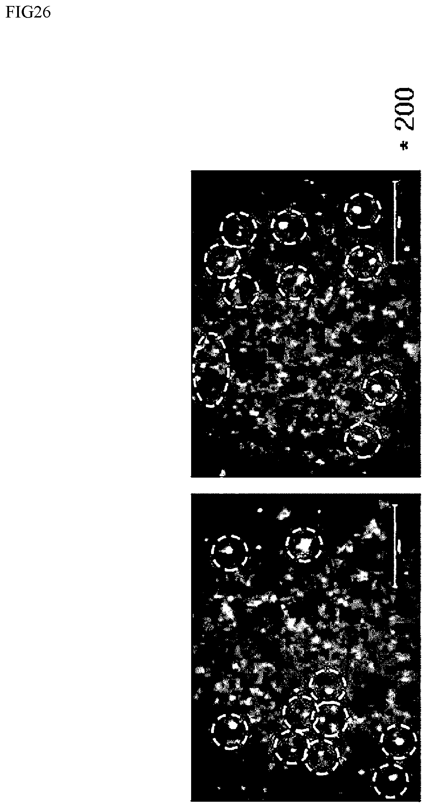

J1 mouse embryonic stem cells (J1 mESCs) used herein were donated by Macrogen (Seoul, Korea). The culture of the J1 mESCs used a 0.1% gelatin-coated dish, and a 2i medium was used as a basic medium for cell proliferation and maintenance. The culture medium was prepared by supplementing an FBS-free N2B27 medium with MEK inhibitor PD0325901 (1 µM) and GSK3 inhibitor CHIR99021 (3 µM) (both from Sigma Aldrich, St. Louis, MO, USA) and 1,000 U/ml LIF (Millipore, Billerica, MA, USA). The cells were incubated in an incubator maintained at 5% CO₂, 95% humidity and 37 °C. Transient transfection was performed using Lipofectamine 3000. The transfection was performed by adding 1x10⁶ cells to FBS and antibiotic-free Opti-MEM (Thermo Scientific, Waltham, MA, USA), adding 10 µg of the first DNA donor (first vector) single-cut with NotI thereto, mixing a Lipofectamine 3000 reagent therewith and incubating the cells at room temperature for 5 minutes. The cells were incubated for 24 to 48 hours in an incubator at 5% CO₂ 95% humidity and 37 °C (FIG. 26).

To confirm whether the first DNA donor (first vector) was inserted into mESC, the cell was selected through the expression of a neomycin-resistant gene present in the first DNA donor (first vector). An antibiotic used herein was G418 (Life Technologies, Grand Island, NY, USA), and a cell selection concentration of G418 was measured using a cell counting kit-8 (CCK-8; Dogindo, Kumamoto, JAPAN). The mESCs were transfected with the first DNA donor (first vector), and 48 hours later, treated with 150 µg/ml of G418. The cell selection process was performed for 4 to 6 weeks, and the formed loxP inserted clone was cultured by fulling. The selected mESC was confirmed by mCherry expression using a fluorescence microscope (FIG. 26).

As a result, the expression of mCherry tagged to the first DNA donor (first vector) in the selected mESC was confirmed. Based on this, it can be confirmed that the first DNA donor (first vector) was inserted into the mESC.

### Example 1-3. Production of microcell using targeted human cell

Micronucleation was performed using colcemid (Life Technologies, Grand Island, NY, USA). One day after 1x10⁶ of the human dermal fibroblasts which were selected using G418 were put into a 100 mm dish, the medium was exchanged with 20% FBS-containing DMEM, the fibroblasts were treated with 0.1 µg/ml of colcemid and incubated for 48 hours in an incubator maintained at 5% CO₂, 95% humidity and 37 °C. The micronucleation-induced human cells were detached using tryLE (Life Technologies, Grand Island, NY, USA), washed with serum-free DMEM, and subjected to centifugation (LABOGENE CO., Ltd, KOREA) at 1000 rpm for 5 minutes. The cells subjected to centrifugation were suspended in pre-warmed serum-free DMEM:Percoll ((Sigma Aldrich, St. Louis, MO, USA) (1:1 (v:v)), and treated with cytochalsin B (Sigma Aldrich, St. Louis, MO, USA) so that a final concentration became 10 µg/ml. The whole cells and the microcells were isolated by centrifugation (LABOGENE) at 16000g and 34 to 37 °C for 1 to 1.5 hours. The isolated whole cells and the microcells were transferred to a 50 ml tube, and serum-free DMEM was added thereto, followed by centrifugation at 500g for 10 minutes. The supernatant was gently removed, 10 ml of serum-free DMEM was added to the pellet attached to the tube surface, and microcells with a size of 8 µm or less were isolated using a 8 µm filter (GE Healthcare, CHICAGO, IL, USA). The supernatant containing the isolated microcells was centrifuged again at 400g for 10 minutes. The centrifuged microcells were isolated using a 5 µm filter in the same manner as the method using an 8 µm filter. The finally isolated microcells were counted using a Nicon eclipse TS100 optical microscope (Nicon Instruments, Melville, NY, USA).

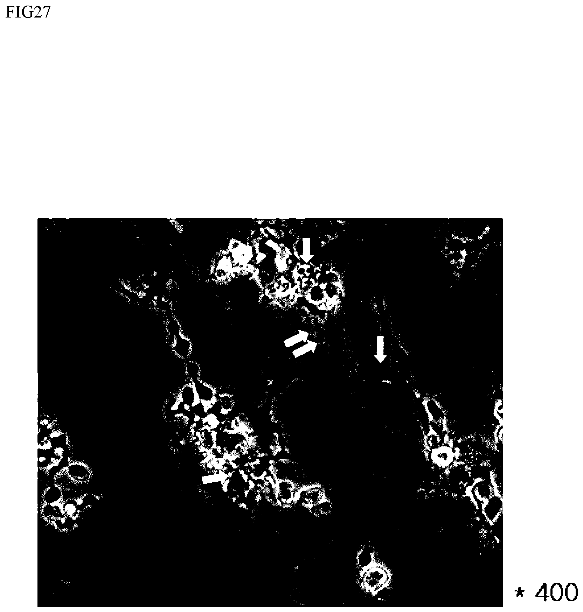

As a result, an optical microscope was used to confirm that the human dermal fibroblasts were treated with colcemid, and then micronuclei were formed, which is the same as the morphology disclosed in a previous paper. In addition, it was confirmed through optical microscopy that the microcells were isolated by size in the process of enucleation of the formed micronuclei with cytochalasin B and the isolation of microcells by size. Therefore, it was demonstrated that the microcells were well produced and isolated from the targeted human cells, that is, the human dermal fibroblasts (FIG. 27).

### Example 1-4. Production of fusion cell of microcell-targeted mouse cell

The isolated microcell (human microcell) and the mESC to be used as a recipient cell were prepared. The mESC was treated with TryLE and centrifuged. The centrifuged mESC was washed with 1xDPBS (Welgene, Korea), and a cell count was calculated using a hemacytometer. The fusion of the human microcell and the mESC was performed using a HVJ-E protein (Cosmo Bio Co., Ltd., Tokyo, Japan) by a suspension method. The calculated ratio of the human microcells to the mESCs was 1:4. Each of the prepared human microcells and mESCs were washed with 500 µl of a cold 1x cell fusion buffer. The solution of the human microcells and the mESCs in the buffer was centrifuged at 300g for 5 minutes at 4 °C. 25 µl of the 1x cell fusion buffer was added per 2x10⁵ mESCs, and the same volume of a 1x cell fusion buffer was added to the human microcells. The mESCs and the human microcells were mixed together, and 5 to 10 µl of a HVJ-E protein was added to the cells, and then the cells were left on ice for 5 minutes. The mixture was left in a 37 °C water bath for 15 minutes. Here, the mixture was tapped every 5 minutes. After the cell fusion of the human microcells and the mESCs, the mixture was centrifuged at 300g for 5 minutes to remove the remaining HVJ-E protein. The fused cells were added to an mESC culture medium-containing dish, and then incubated for 48 hours in an incubator maintained at 5% CO₂, 95% humidity and 37 °C.

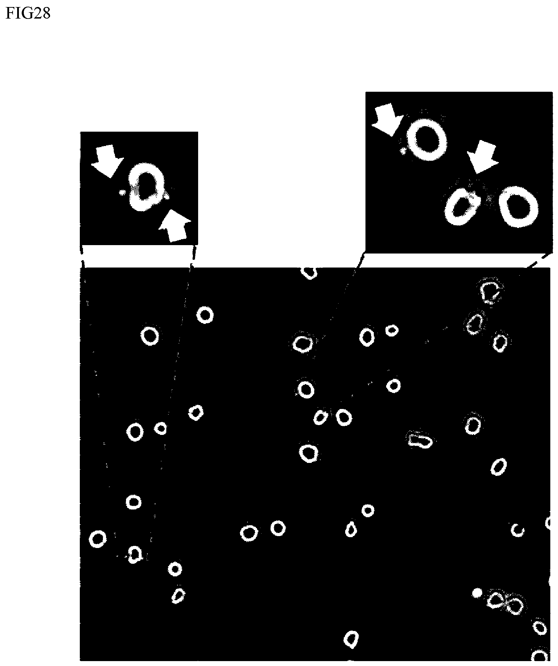

As a result, after the cell fusion of the human microcells and the mESCs, the mixture was added to a culture medium, and observed using a microscope to show that the mESCs and the microcells were fused. Since the method using the HVJ-E protein is a suspension method (performed to carry out an experiment with single cells because the morphology of mESC proliferation is a colony form), a real-time fusion process cannot be confirmed, but indirectly confirmed (FIG. 28).

### Example 1-5. Production of cell including artificial recombinant chromosome using fusion cell

### Example 1-5-1. Production of cell including artificial recombinant chromosome using fusion cell

To produce an artificial recombinant chromosome in the fusion cell formed in Example 1-4, 1x10⁶ of the fusion cells added to 100 µl of an FBS and antibiotic-free Opti-MEM. Here, to express Cre recombinase, 10 µg of a pCMV-Cre vector (System Biosciences, LLC, Palo Alto, CA, USA) was added, followed by transfection at 125V and 5 ms with dual pulses. After 300 µl of a 2i medium was added and mixed well with the cells, the cells were added to a 100 mm dish, and incubated for 48 hours in an incubator maintained at 5% CO₂, 95% humidity and 37 °C to induce the recombination between chromosomes.

Forty-eight hours later, both of a group in which a fusion cell was not transfected with a pCMV-Cre vector (-Cre) and a group in which a fusion cell was transfected with a pCMV-Cre vector (+Cre) were treated with 150 µg/ml of G418. The cells were selected for 10 to 14 days, and treated also with 150 µg/ml of G418 every 2 to 3 days in medium exchange. The observation of the selected cells was confirmed using a fluorescence microscope (Olympus).

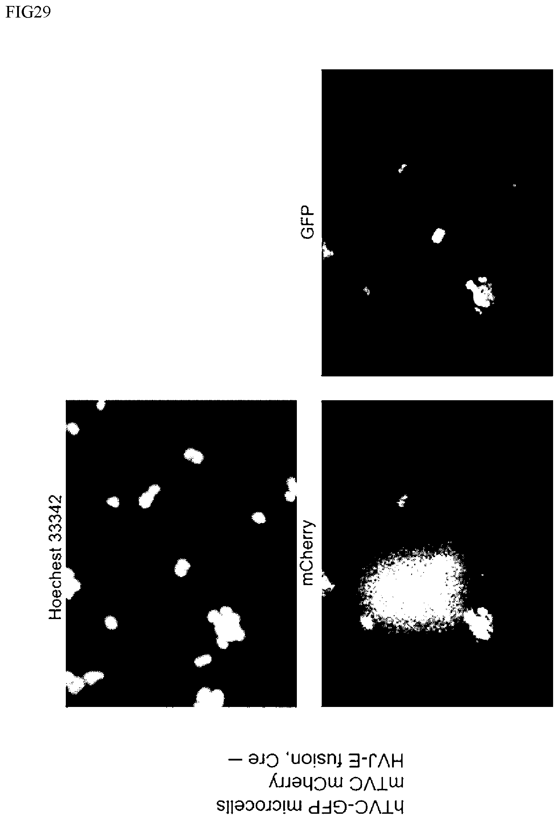

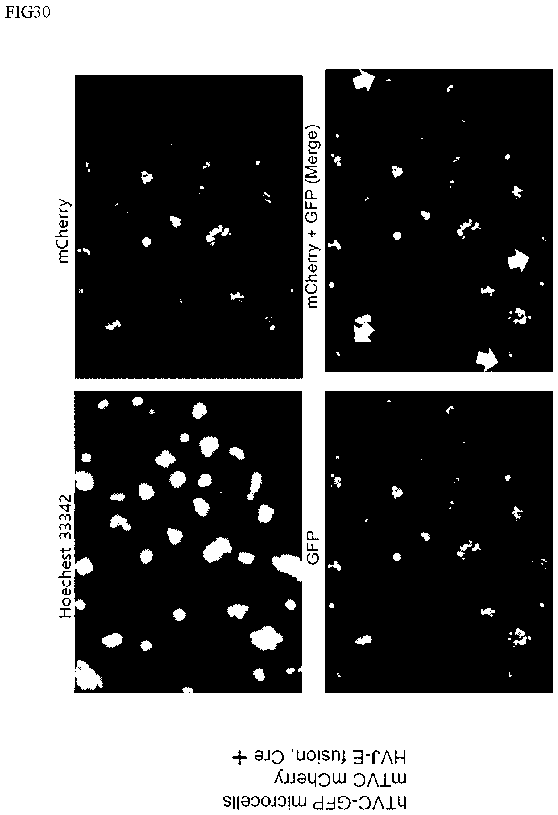

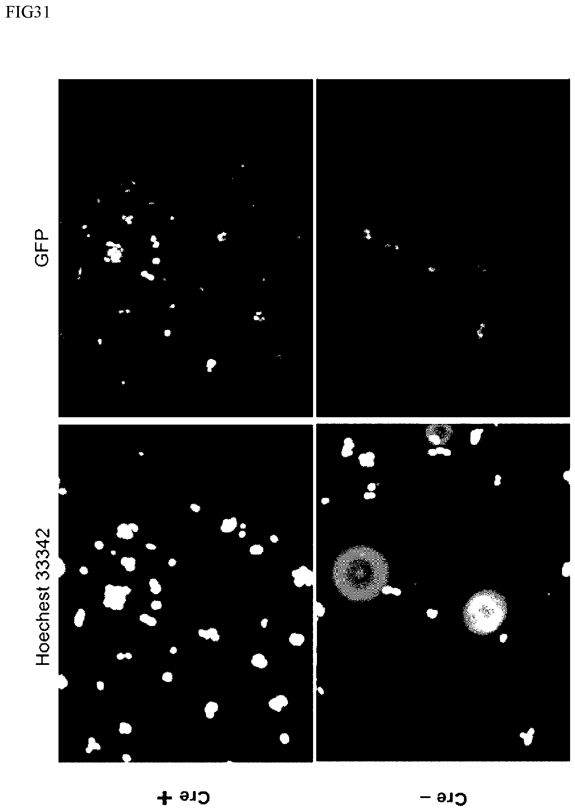

As a result, it was confirmed that specific translocation occurs at a loxP site by Cre recombinase in the fusion cells. That is, a hDF chromosome (chromosome including a GFP gene inserted by the second DNA donor) was transferred into an mESC using the human microcells, and an artificial recombinant chromosome recombined by pairing of loxP located on a hDF chromosome (chromosome including a GFP gene inserted by the second DNA donor) and loxP located on an mESC chromosome (chromosomes including an mCherry gene inserted by the first DNA donor) and Cre recombinase was produced. The produced artificial recombinant chromosome was identified through only GFP expression occurring in the mESC chromosome in which mCherry expression occurred. Therefore, it was confirmed that the chromosome transfer via a human microcell and the recombination between chromosomes using Cre-loxP were possible (FIGS. 29 to 31).

### Example 1-5-2. Confirmation of artificial recombinant chromosome using Fluorescence In Situ Hybridization (FISH)

### FISH process

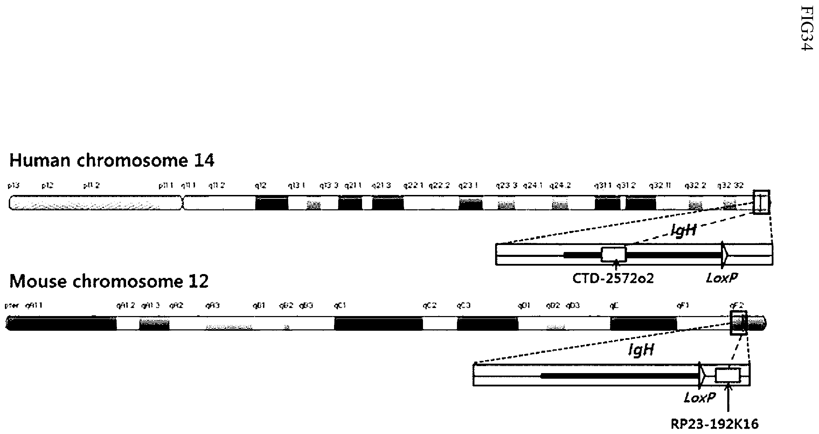

To construct a mouse BAC probe and a human BAC probe, RP23-192K16 was used as a mouse sample and CTD-2572o2 was used as a human sample. BAC DNA was prepared using a Plasmid Maxi kit (Qiagen, Germany), and a BAC probe was constructed using a Tag FISH Tag^{™} DNA Multicolor Kit (Thermo Fisher, USA). The mouse BAC probe was labeled with an Alexa 488 fluorescent dye, and the human BAC probe was labeled with an Alexa 555 fluorescent dye (FIG. 34).

A slide used in FISH was treated with colcemid (Life Technologies, Grand Island, NY, USA) and a hypotonic solution (75Mm KCl) for the diffusion of a metaphase chromosome of a cell, and produced by a basic fixing method with methanol:acetic acid (3:1).

The FISH experiment was performed according to the basic instructions for a FISH Tag^{™} DNA Multicolor Kit (Thermo Fisher, USA). The slide was permeabilized in a 0.05% pepsin (Sigma Aldrich, St. Louis, MO, USA)/10 mM HCl solution at 37 °C for 10 minutes. The slide was dehydrated with an ethanol series (70%, 85% and 100%) for one minute at room temperature, and then air-dried. For hybridization, the slide was denatured with 70% formamide (Sigma Aldrich) and 2x SSC (Sigma Aldrich) at pH 7.0 and 72 °C for 2 minutes, dehydrated with an ethanol series (70%, 80% and 95%) at -20 °C for 2 minutes, and then allowed to air dry. The final concentration of each of the human BAC probe and the mouse BAC probe was 4 ng/µl. 2.5 µl of each DNA probe, 65% formamide and 2x SSC were denatured at 72 °C for 5 minutes and cooled on ice, and each slide was treated with 10 µl of the resulting solution and covered with a glass coverslip, and then four sides of the slide were sealed with rubber cement. Hybridization was performed in a chamber maintained under a wet condition at 37 °C overnight. Afterward, the slide was immersed in 2x SSC to remove a coverslip, equilibrated with 0.4x SSC at room temperature, placed in 0.4x SSC at 73 °C for 2 minutes and then washed by adding phosphate buffered saline (PBS) at room temperature. Nuclear staining was performed using a Vectashield mounting medium and DAPI (Vector Laboratories, Burlingame, CA, USA).

The slide was observed under an LSM 800 confocal microscope (Carl Zeiss, Germany) using Airyscan. The slide was observed using a 40x/1.2 Plan-Apochromat objective lens and a 63x/1.4 NA Plan-Apochromat oil objective lens, and a confocal microscope image was analyzed using Zeiss Zen Blue software.

### FISH result

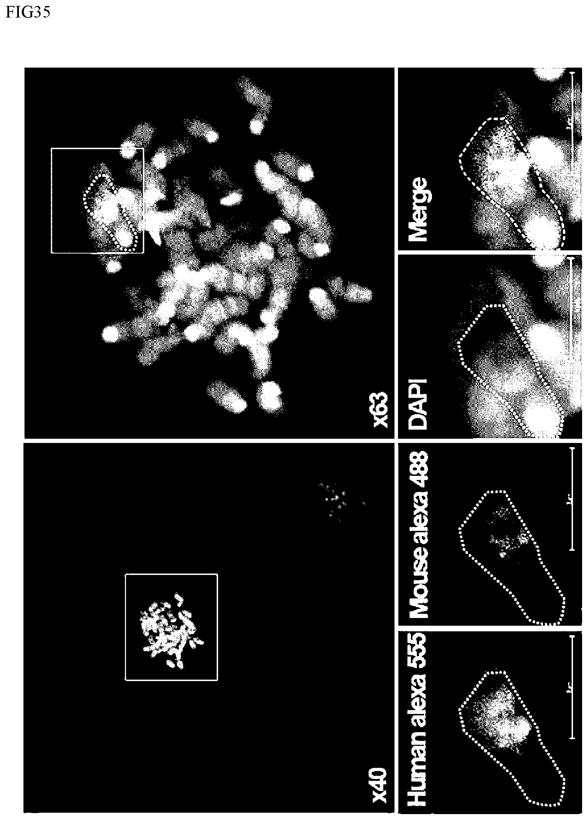

After a cell including an artificial recombinant chromosome was selected, a FISH experiment was performed using a human chromosome 14-specific human BAC probe (Alexa 555) and a mouse chromosome 15-specific mouse BAC probe (Alexa 488). The chromosome of the cell was confirmed by DAPI staining (40x), and using a 63x oil objective lens. The fluorescence of the human BAC probe (Alexa 555) and the mouse BAC probe (Alexa 488) was emitted at the same site in a fusion cell having an artificial recombinant chromosome, confirming that the end of the 4.1 Mb mouse chromosome 12 was transferred to the end of the human chromosome 14 (FIGS. 34 and 35).

### Example 1-6. Production of transgenic animal using cell including artificial recombinant chromosome

To produce a transgenic animal using a cell having an artificial recombinant chromosome, the cell having the artificial recombinant chromosome obtained in Example 1-5 is treated with FIAU. The cell obtained after the treatment is implanted into a blastula through blastocyst injection, thereby producing a chimeric blastocyst. The produced chimeric blastocyst is implanted in the uterus of the surrogate, thereby producing a mouse offspring. The produced mouse offspring is a chimeric-transgenic mouse, and a heterozygous transgenic mouse or homozygous transgenic mouse is produced by breeding the chimeric-transgenic mice.

The produced transgenic mouse is a mouse having a GFP gene at the 5' end of IgHV on the mouse genome. Here, the transgenic mouse can be produced by various methods other than blastocyst injection.

### Example 2. Production of artificial recombinant chromosome using two RRSs and production of transgenic animal using the same

This example is an experimental example for proving a method of introducing transformation using a chromosome disclosed herein, and relates to a cell including an artificial recombinant chromosome into which a gene of interest is inserted at a specific position by recombination between chromosomes and a method of producing a transgenic mouse using the same. The following description provides overall examples regarding a cell in which an antibiotic-resistant gene is inserted at the end of the variable region of the IgH locus using two RRSs and the production of a transgenic mouse using the same, which are merely examples using an artificial recombinant chromosome, but the present invention is not limited thereto. The artificial recombinant chromosome of interest may be produced by modifying examples to be described below in various ways, or by adding various methods, other than the examples to be described below.

### Example 2-1. Vector construction for producing targeted cell

A first DNA donor (a first vector) was designed to produce a mouse embryonic stem cell (mESC) as a targeted mESC, and a second DNA donor (a second vector) was designed to produce a human fibroblast as a targeted human fibroblast.

A taq used in a PCR reaction was a general PCR tag, which is GoTaq G2 green (Promega, USA), and PrimeSTAR (Takara, Japan) was used as a tag for blunt-end production, and SimpliAmp (Thermo Fisher Scientific, USA) was used as a thermocycler. A T-blunt vector (Solgent, Korea) was used as a T-vector used in clone production and DNA sequencing, and a HIT competent cell (RBC Bioscience, USA) was used as a competent cell. All restriction enzymes used in DNA recombination were purchased from New England Biolabs (NEB), and a ligase used in DNA ligation was a T4 DNA ligase (Takara, Japan).

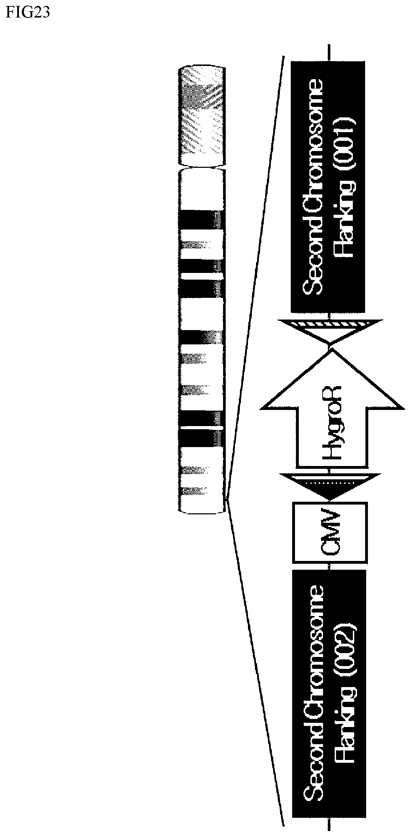

The first DNA donor (first vector) to be used in mouse ESC targeting consists of M002, a CMV promoter, Loxm2/71, a hygromycin-resistant gene (HygroR), lox66, M001, a TK gene to be used in negative selection, AmpR to be used in bacterial positive selection, and a replication origin. In the case of the CMV promoter, overhang-PCR was performed with a pCMV6-AC-GFP vector as a template using forward GSP having an XhoI site (Table 5. SEQ ID NO: 15) and reverse GSP having a KpnI site (Table 5. SEQ ID NO: 16). In the case of HygroR, overhang-PCR was performed with a pSecTag2-hygroA vector as a template using forward GSP having a KpnI site and loxm2/71 (Table 5. SEQ ID NO: 17) and reverse GSP having lox66 and a SalI site (Table 5. SEQ ID NO: 18). The CMV promoter and the HygroR PCR product were ligated to a T-blunt vector and then cloned, followed by confirming DNA base sequences. Plasmids obtained from two clones were cleaved using a restriction enzyme acting on restriction sites at both ends, and then ligated to the first vector of Example 1-1, which was treated with XhoI and SalI, using a T4 DNA ligase (FIG. 23).

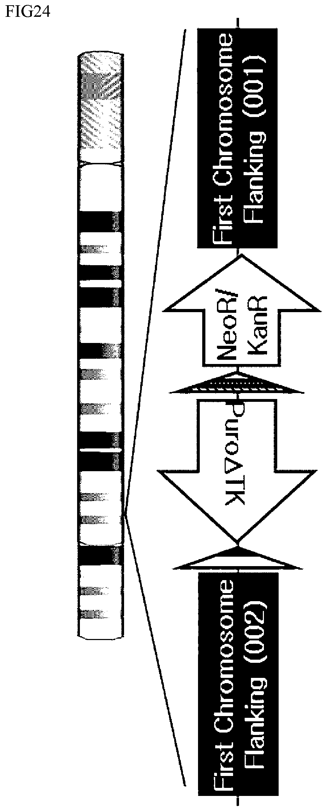

The second DNA donor (second vector) to be used in human fibroblast targeting consists of H002, lox71, inverted puroΔTK, loxm2/66, NeoR, H001, a TK gene to be used in negative selection, AmpR to be used in bacterial positive selection and a replication origin. In the case of the inverted puroΔTK, overhang-PCR was performed with synthetic DNA as a template using forward GSP having an XhoI site and lox71 (Table 5. SEQ ID NO: 19) and reverse GSP having loxm2/66 and HindIII (Table 5. SEQ ID NO: 20). In the case of NeoR, overhang-PCR was performed with a pCMV6-AC-GFP vector as a template using forward GSP having HindIII (Table 5. SEQ ID NO: 21) and reverse GSP having SalI (Table 5. SEQ ID NO: 22). The Lox71-inverted puroΔTK-loxm2/66 and NeoR PCR products were cloned after the ligation to a T-blunt vector, and their DNA base sequences were confirmed. Plasmids obtained from two clones were cleaved using a restriction enzyme acting on restriction sites at both ends, and then ligated to the XhoI and SalI-treated second vector of Example 1-1 using a T4 DNA ligase (FIG. 24).

### Example 2-2. Production of cell including two RRS-inserted chromosomes

### Example 2-2-1. Production of targeted human cell including two loxP-inserted human chromosomes

A normal human foreskin fibroblast cell line (BJ) used herein was purchased from ATCC (Manassas, VA, USA). As a basic culture medium for proliferating and maintaining cells, a Dulbecco's Modified Eagle's Medium (DMEM; Corning, Mannasas, VA, USA) containing 10% fetal bovine serum (FBS; Corning, Mannasas, VA, USA) and 1% penicillin-streptomycin (Corning, Mannasas, VA, USA) was used, and the cells were incubated in an incubator maintained at 5% CO₂, 95% humidity and 37 °C. Transient transfection was performed using a Nepa21(NEPAGENE Co., Ltd., Chiba, Japan) electroporator. The transfection was performed at 150V and 7.5 ms with dual pulses by adding 1x10⁶ cells to 100 µl of FBS and antibiotic-free Opti-MEM, adding 10 µg of the second DNA donor (second vector) single-cut with NotI thereto. After 300 µl of 10% FBS-containing medium was added and mixed well with the cells, the cells were added to a 100 mm dish, and incubated for 24 to 48 hours in an incubator maintained at 5% CO₂,95% humidity and 37 °C.

To confirm whether the second DNA donor (second vector) was inserted into the human cell line BJ, a cell was selected using an antibiotic-resistant gene present in the second DNA donor (second vector). A cell was selected from the BJ cell line through the expression of a neomycin-resistant gene present in the second DNA donor (second vector). The antibiotic used herein was G418 (Life Technologies, NY, USA), and a cell selection concentration of G418 was measured using a cell counting kit-8 (CCK-8; Dogindo, Kumamoto, JAPAN). The BJ cells were transfected with the second DNA donor (second vector), and 48 hours later, treated with 300 µg/ml of G418. The cell selection process was performed for 4 to 6 weeks, and the formed loxP-inserted clone was incubated by fulling.

As a result, by confirming the expression of the antibiotic-resistant gene inserted into the second DNA donor (second vector), it was shown that all of the cells died during the selection period in a control group, but colonies were identified during the selection period in a group into which the second DNA donor (second vector) was inserted. As the proliferation and maintenance of cells continued even with the continuous addition of antibiotics, it can be seen that the expression of the second DNA donor (second vector) continuously occurs.

### Example 2-2-2. Production of targeted mouse cell including two loxP-inserted mouse chromosomes

J1 mouse embryonic stem cells (J1 mESCs) used herein were donated by Macrogen (Seoul, Korea). The J1 mESCs were plated in a 0.1% gelatin-coated dish, and as a basic culture medium for proliferating and maintaining cells, a 2i medium was used, and prepared by adding MEK inhibitor PD0325901 (1 µM) and GSK3 inhibitor CHIR99021 (3 µM) (both from Sigma Aldrich, St. Louis, MO, USA), and 1,000 U/ml LIF (Millipore, Billerica, MA, USA) to an FBS-free N2B27 medium. The cells were incubated in an incubator maintained at 5% CO₂, 95% humidity and 37 °C. Transient transfection was performed using a Nepa21(NEPAGENE Co., Ltd., Chiba, Japan) electroporator. The transfection was performed at 125V and 5 ms with dual pulses by adding 1x10⁶ cells to 100 µl of FBS and antibiotic-free Opti-MEM and adding 10 µg of the first DNA donor (first vector) single-cut with NotI thereto. After 300 µl of a 2i medium was added and mixed well with the cells, the cells were seeded in a 100 mm dish, and incubated for 24 to 48 hours in an incubator maintained at 5% CO₂, 95% humidity and 37 °C.

To confirm whether the first DNA donor (first vector) was inserted into the mESCs, a cell was selected through the expression of a hygromycin-resistant gene present in the first DNA donor (first vector). The antibiotic used herein was hygromycin B (Fujifilm Wako Pure Chemical Corporation, Osaka, JAPAN), and a cell selection concentration of hygromycin was measured using a cell counting kit-8 (CCK-8; Dogindo, Kumamoto, JAPAN). The mESCs were transfected with the first DNA donor (first vector), and 48 hours later, treated with 32 µg/ml of hygromycin. The cell selection process was performed for 4 to 6 weeks, and the formed loxP inserted clone was cultured by fulling.

As a result, by confirming the expression of the antibiotic-resistant gene inserted into the first DNA donor (first vector), it was shown that all of the cells died during the selection period in a control group, but colonies were identified during the selection period in a group into which the first DNA donor (first vector) was inserted. As the proliferation and maintenance of cells continued even with the continuous addition of antibiotics, it can be seen that the expression of a targeting vector continuously occurred.

### Example 2-3. Production of microcell using targeted human cell

Micronucleation was performed using colcemid (Life Technologies, Grand Island, NY, USA). One day after 1x10⁶ cells of the normal human foreskin fibroblast cell line (BJ) selected using G418 were seeded in a 100 mm dish, the medium was exchanged with 20% FBS-containing DMEM, and the cells were treated with 0.1 µg/ml of colcemid and incubated for 48 hours in an incubator maintained at 5% CO₂, 95% humidity and 37 °C. The micronucleation-induced human cells were detached using tryLE (Life Technologies, Grand Island, NY, USA), washed with serum-free DMEM, and subjected to centrifugation (LABOGENE CO., Ltd, KOREA) at 1000 rpm for 5 minutes. The cells subjected to centrifugation were suspended in pre-warmed serum-free DMEM:Percoll ((Sigma Aldrich, St. Louis, MO, USA) (1:1 (v:v)), and treated with cytochalsin B (Sigma Aldrich, St. Louis, MO, USA) so that a final concentration became 10 µg/ml. The whole cells and the microcells were isolated by centrifugation (LABOGENE) at 16000g at 34 to 37 °C for 1 to 1.5 hours. The isolated whole cells and the microcells were transferred to a 50 ml tube, and serum-free DMEM was added thereto, followed by centrifugation at 500g for 10 minutes. The supernatant was gently removed, 10 ml of serum-free DMEM was added to the pellet attached to the tube surface, and microcells with a size of 8 µm or less were isolated using a 8 µm filter (GE Healthcare, CHICAGO, IL, USA). The supernatant containing the isolated microcells was centrifuged again at 400g for 10 minutes. The centrifuged microcells were isolated using a 5 µm filter in the same manner as the method using an 8 µm filter. The finally isolated microcells were counted using a Nicon eclipse TS100 optical microscope (Nicon Instruments, Melville, NY, USA).

As a result, an optical microscope was used to confirm that the normal human foreskin fibroblast cell line (BJ) was treated with colcemid, and then micronuclei were formed, which is the same as the morphology disclosed in a previous paper. In addition, it was confirmed through optical microscopy that the microcells were isolated by size in the process of enucleation of the formed micronuclei with cytochalasin B and isolation of microcells by size. Therefore, it was demonstrated that the microcells were well produced and isolated from the targeted human cells, that is, the normal human foreskin fibroblast cell line (BJ).

### Example 2-4. Production of fusion cell of microcell-targeted mouse cell

The isolated microcells (human microcells) and the mESCs to be used as recipient cells were prepared. The mESCs were treated with TryLE and centrifuged. The centrifuged mESCs were washed with 1xDPBS (Welgene, Korea), and a cell count was calculated using a hemacytometer. The fusion of the human microcells and the mESCs was performed using a HVJ-E protein (Cosmo Bio Co., Ltd., Tokyo, Japan) by a suspension method. The calculated ratio of the human microcells to the mESCs was 1:4. Each of the prepared human microcells and mESCs were washed with 500 µl of a cold 1x cell fusion buffer. Centrifugation was performed with solutions of the human microcells and the mESCs contained in the buffer at 300g for 5 minutes at 4 °C. 25 µl of a 1x cell fusion buffer was added per 2x10⁵ of mESCs, and the same volume of the 1x cell fusion buffer was added to the human microcells. The mESCs and the human microcells were mixed together, and 5 to 10 µl of the HVJ-E protein was added thereto, and then the cells were left on ice for 5 minutes. The mixture was left in a 37 °C water bath for 15 minutes. Here, the mixture was tapped every 5 minutes. After the cell fusion of the human microcells and the mESCs was completed, the remaining HVJ-E protein was removed by centrifugation at 300g for 5 minutes. The fused cells were put into a dish containing an mESC culture medium, and then incubated for 48 hours in an incubator maintained at 5% CO₂, 95% humidity and 37 °C.

As a result, after the cell fusion of the human microcells and the mESCs, the mixture was added to a culture medium, and observed using a microscope to show that the mESCs and the microcells were fused. Since the method using the HVJ-E protein is a suspension method (performed to carry out an experiment with single cells because the morphology of mESC proliferation is a colony form), a real-time fusion process cannot be confirmed, but indirectly confirmed.

### Example 2-5. Production of cell including artificial recombinant chromosome using fusion cell

To produce an artificial recombinant chromosome in the fusion cell formed in Example 2-4, 1x10⁶ of the fusion cells were added to 100 µl of an FBS and antibiotic-free Opti-MEM medium. Here, to express Cre recombinase, 10 µg of a pCMV-Cre vector (System Biosciences, LLC, Palo Alto, CA, USA) was added, followed by transfection at 125V and 5 ms with dual pulses. After 300 µl of a 2i medium was added and mixed well with the cells, the cells were added to a 100 mm dish, and incubated for 48 hours in an incubator maintained at 5% CO₂, 95% humidity and 37 °C.

Forty-eight hours later, 5x10⁴ of the fusion cells were seeded in a 6-well plate, and treated with 0.6 µg/ml of puromycin. The puromycin concentration was determined as an appropriate concentration using a cell counting kit-8 (CCK-8; Dogindo, Kumamoto, Japan). The cells were grown for one week, and treated with a fresh medium and puromycin every 2 or 3 days. For the fixation and staining of the cells, crystal violet (Sigma Aldrich, St. Louis, MO, USA) was used, and colonies of mESCs with a size of approximately 70 µm were counted. A graph was plotted using GraphPad PRISM (version 5.01), and statistical significance between 3 groups was estimated by one-way ANOVA.

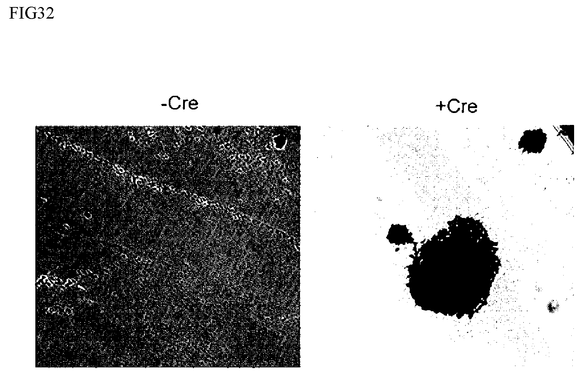

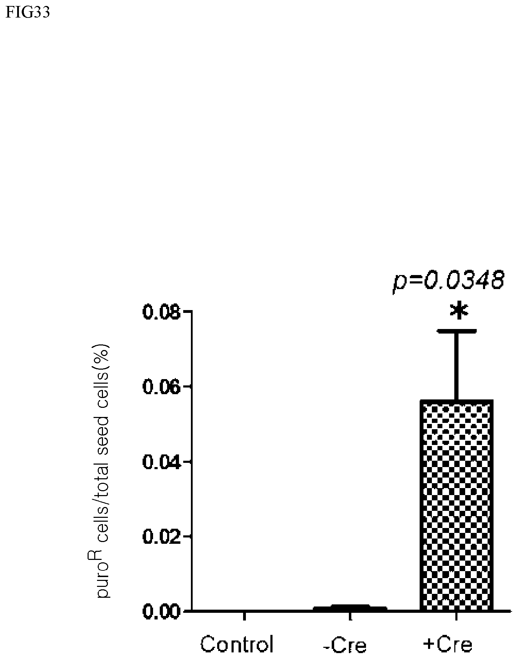

As a result, the process of selecting a cell including an artificial recombinant chromosome was also described in Example 2-1, but the cell was selected by normal expression of a puromycin-resistance gene inverted by Cre recombinase treated to the fusion cell. That is, the chromosome (chromosome including an inverted puroΔTK gene inserted by a second DNA donor) of a human cell line BJ was transferred into an mESC via a human microcell, and an artificial recombinant chromosome recombined by pairing (the pairing of first loxP and fourth loxP; and the pairing of second loxP and third loxP) of two loxPs (the first loxP and the second loxP) located on the BJ chromosome (the chromosome including an inverted puroΔTK gene inserted by the second DNA donor) and two loxPs (third loxP and fourth loxP) located on the mESC chromosome (the chromosome including a hygromycin-resistant gene inserted by the first DNA donor) and Cre recombinase was produced. The produced artificial recombinant chromosome included a reinverted puroΔTK gene, and the survival of a cell was confirmed by treatment of each group with puromycin. As a result, it was confirmed that mESC proliferation occurs in a group subjected to the expression of Cre recombinase. Therefore, it can be seen that the puroΔTK gene was normally expressed by the production of an artificial recombinant chromosome (FIGS. 32 and 33).

### Example 2-6. Production of transgenic animal using cell including artificial recombinant chromosome

To produce a transgenic animal using a cell including an artificial recombinant chromosome, the cell including the artificial recombinant chromosome obtained in Example 2-5 is treated with FIAU. The cells obtained after the treatment are implanted into blastulas through blastocyst injection, thereby constructing chimeric blastocysts. The constructed chimeric blastocysts are implanted in the uterus of a surrogate to produce a mouse offspring. The produced mouse offspring is a chimeric-transgenic mouse, and a heterozygous transgenic mouse or homozygous transgenic mouse is produced by breeding of the chimeric-transgenic mice.

The produced transgenic mouse is a mouse having a puroΔTK gene on the mouse genome. Here, the transgenic mouse can be produced by various methods, other than blastocyst injection.

### Example 3. Production of transgenic animal using artificial recombinant chromosome

This example relates to a method of producing a humanized mouse, and particularly, to a transgenic mouse having a chromosome with a humanized specific gene, that is, an artificial recombinant chromosome. The following description relates to overall examples for producing a transgenic mouse in which the variable region of an IgH locus is humanized, which is merely an example using an artificial recombinant chromosome, but the present invention is not limited thereto. An artificial recombinant chromosome of interest may be produced by modifying examples described below in various ways, and by adding various methods other than the examples described below.

### Example 3-1. Vector construction for producing targeted cell

To produce targeted mESCs from mouse embryonic stem cells (mESCs), a first DNA donor (a first vector) and a second DNA donor (a second vector) are prepared.

The first vector consists of a first homologous arm to be used in targeting of the 5' end of the variable region (all of V segments, D segments and J segments) of a mouse IgH locus, piggyBac terminal repeat (PB-TR), a promoter, loxm2/66 (first RRS), a blasticidin-resistant gene, a promoter, an FRT, and a second homologous arm to be used in targeting of the 5' end of the variable region of the mouse IgH locus.

The second vector consists of a third homologous arm to be used in targeting of the 3' end of the variable region (all of V segments, D segments and J segments) of the mouse IgH locus, an inverted hygromycin-resistant gene, an FRT, lox71 (second RRS), a promoter, a neomycin-resistant gene (NeoR), a piggyBac terminal repeat (PB-TR), and a fourth homologous arm to be used in targeting of the 3' end of the variable region of the mouse IgH locus.

To produce a human fibroblast to be a targeted human fibroblast, a third DNA donor (a third vector) and a fourth DNA donor (a fourth vector) are used.

The third vector consists of a fifth homologous arm to be used in targeting of the 5' end of the variable region (all of V segments, D segments and J segments) of a human IgH locus, a promoter, a blasticidin-resistant gene, lox66 (third RRS), a promoter, an FRT, and a sixth homologous arm to be used in targeting of the 5' end of the variable region of the human IgH locus.

The fourth vector consists of a seventh homologous arm to be used in targeting of the 3' end of the variable region (all of V segments, D segments and J segments) of the human IgH locus, a piggyBac terminal repeat (PB-TR), an inverted hygromycin-resistant gene, an FRT, an inverted puroΔTK gene, loxm2/71 (fourth RRS), a promoter, a neomycin-resistant gene (NeoR), and an eighth homologous arm to be used in targeting of the 3' end of the variable region of the human IgH locus.

Here, the DNA donors may be designed in various forms according to purpose, and the design can be modified to further include various elements for the selection process.

### Example 3-2. Production of cell including RRS-inserted chromosome

### Example 3-2-1. Production of targeted human cell including RRS-inserted human chromosome

Human fibroblasts used herein are cultured for proliferation and maintenance in a media containing a Dulbecco's Modified Eagle's Medium (DMEM; Corning, Mannasas, VA, USA) containing a 10% fetal bovine serum (FBS; Corning, Mannasas, VA, USA) and 1% penicillin-streptomycin (Corning, Mannasas, VA, USA) and a incubator maintained at 5% CO₂, 95% humidity and 37 °C. Transient transfection is performed using Lipofectamine 3000 (Invitrogen, Carlsbad, CA, USA) or a Nepa21(NEPAGENE Co., Ltd., Chiba, Japan) electroporator. The transient transfection is performed by seeding 1x10⁶ cells in an FBS and antibiotic-free medium, adding 10 µg of the third vector thereto, mixing a Lipofectamine 3000 reagent therewith, and incubating the cells at room temperature for 5 minutes. Alternatively, 10 µg of the third vector is added to perform transfection at 150V and 7.5 ms with dual pulses. The transfected cells are incubated for 24 to 48 hours in an incubator maintained at 5% CO₂, 95% humidity and 37 °C.

To confirm whether the third vector is inserted at the 5' end of the variable region of the human IgH locus, the insertion of the third vector is confirmed according to the survival of a cell confirmed by treating the transfected cells with blasticidin. After the blasticidin treatment, surviving cells are obtained, and transfected with the fourth vector by the same method as used for the third vector. The transfected cell is incubated for 24 to 48 hours in an incubator maintained at 5% CO₂, 95% humidity and 37 °C. To confirm whether the fourth vector is inserted at the 3' end of the variable region of the human IgH locus, the insertion of the fourth vector is confirmed by treating the transfected cell with G418 (Life Technologies, NY, USA). After the G418 treatment, the surviving cells are obtained.

To confirm whether the third vector and the fourth vector are inserted into the same chromosome (chromosome having a human IgH locus), the cells obtained after G418 treatment are treated with a recombinase flippase (FLP). When the third vector and the fourth vector are inserted into the same chromosome, the variable region of the human IgH locus is inverted by inducing recombination by the FRT present in the two vectors and the treated FLP. To confirm this, the cells in which the FRT-FLP recombination is induced are treated with zeocin, and then according to the survival of the cells, the insertion of the third vector and the fourth vector into the same chromosome is confirmed. After the zeocin treatment, surviving cells are obtained. The obtained cells are cells including a chromosome in which lox66 (third RRS) and loxm2/71 (fourth RRS) are inserted at both ends of the variable region of the human IgH locus, respectively. Since a somatic cell generally has two alleles, such a selection process usually performs for exclusion a case in which only one of the third vector and the fourth vector is inserted into two alleles.

Here, when there are several vectors, the vector introduction can be sequentially, randomly or simultaneously performed. The process of selecting the vector-introduced cell can be modified in various ways according to an element inserted into the vector.

### Example 3-2-2. Production of targeted mouse cell including RRS-inserted mouse chromosome

Mouse embryonic stem cells (mESCs) used herein are cultured in a basic medium, that is, a 2i medium, which is prepared by supplementing a FBS-free N2B27 medium with MEK inhibitor PD0325901 (1 µM) and GSK3 inhibitor CHIR99021 (3 µM) (both from Sigma Aldrich, St. Louis, MO, USA) and 1,000 U/ml LIF (Millipore, Billerica, MA, USA), in an incubator maintained at 5% CO₂, 95% humidity and 37 °C for proliferation and maintenance. Transient transfection is performed using Lipofectamine 3000 or a Nepa21 (NEPAGENE Co., Ltd., Chiba, Japan) electroporator. The transient transfection is performed by seeding 1x10⁶ cells in an FBS and antibiotic-free medium, adding 10 µg of the first vector thereto, mixing a Lipofectamine 3000 reagent therewith, and incubating the cells at room temperature for 5 minutes. Alternatively, 10 µg of the first vector is added to perform transfection at 125V and 5 ms with dual pulses. The transfected cells are incubated for 24 to 48 hours in an incubator maintained at 5% CO₂, 95% humidity and 37 °C.

To confirm whether the first vector is inserted at the 5' end of the variable region of the mouse IgH locus, the insertion of the first vector is confirmed according to the survival of a cell after the transfected cell is treated with blasticidin. After the blasticidin treatment, the surviving cells are obtained, and then transfected with a second vector by the same method as for the first vector. The transfected cell is incubated for 24 to 48 hours in an incubator maintained at 5% CO₂, 95% humidity and 37 °C. To confirm whether the second vector is inserted at the 3' end of the variable region of the mouse IgH locus, the insertion of the second vector is confirmed according to the survival of a cell after the transfected cell is treated with G418 (Life Technologies, NY, USA). After the G418 treatment, the surviving cells are obtained.

To confirm whether the first vector and the second vector are inserted into the same chromosome (chromosome having a mouse IgH locus), cells obtained after the G418 treatment are treated with a recombinase, that is, flippase (FLP). When the first vector and the second vector are inserted into the same chromosome, the variable region of the mouse IgH locus is inverted by inducing recombination by the FRT present in the two vectors and the treated FLP. To confirm this, the cells in which the FRT-FLP recombination is induced are treated with zeocin, and then according to the survival of the cells, the insertion of the first vector and the second vector into the same chromosome is confirmed. After the zeocin treatment, surviving cells are obtained. The obtained cells are cells including a chromosome in which loxm2/66 (first RRS) and lox71 (second RRS) are inserted at both ends of the variable region of the mouse IgH locus, respectively. Since a somatic cell generally has two alleles, such a selection process usually excludes a case in which only one of the first vector and the second vector is inserted into two alleles.

Here, when there are several vectors, the vector introduction may be sequentially, randomly or simultaneously performed. The process of selecting the vector-introduced cell can be modified in various ways according to an element inserted into the vector.

### Example 3-3. Production of microcell using targeted human cell

Micronucleation is performed using colcemid (Life Technologies, Grand Island, NY, USA). One day after the selected targeted human cells (cell including a chromosome in which lox66 (third RRS) and loxm2/71 (fourth RRS) are inserted at both ends of the variable region of the human IgH locus, respectively) are grown to be 1x10⁶ cells, the medium is exchanged with a 20% FBS-containing DMEM, and then the cells are treated with 0.1 µg/ml of colcemid and incubated for 48 hours in an incubator maintained at 5% CO₂, 95% humidity and 37 °C. The micronucleation-induced targeted human cells are detached using tryLE (Life Technologies, Grand Island, NY, USA) and washed with a serum-free DMEM, followed by centrifugation (LABOGENE CO., Ltd, KOREA) at 1000 rpm for 5 minutes. The cells subjected to centrifugation are suspended in pre-warmed serum-free DMEM:Percoll ((Sigma Aldrich, St. Louis, MO, USA) (1:1 (v:v)), and treated with cytochalsin B (Sigma Aldrich, St. Louis, MO, USA) so that a final concentration became 10 µg/ml. Whole cells and the microcells are isolated by centrifugation (LABOGENE) at 16000g and 34 to 37 °C for 1 to 1.5 hours. The isolated whole cells and the microcells are transferred to a 50 ml tube, and serum-free DMEM is added thereto, followed by centrifugation at 500g for 10 minutes. The supernatant is gently removed, 10 ml of serum-free DMEM is added to the pellet attached to the tube surface, and microcells with a size of 8 µm or less are isolated using an 8 µm filter (GE Healthcare, CHICAGO, IL, USA). The supernatant containing the isolated microcells is centrifuged again at 400g for 10 minutes. The centrifuged microcells are isolated using a 5 µm filter in the same manner as the method using an 8 µm filter. The finally isolated microcells are counted using a Nicon eclipse TS100 optical microscope (Nicon Instruments, Melville, NY, USA). Therefore, the microcells are obtained from the targeted human cells.

### Example 3-4. Production of fusion cell of microcell-targeted mouse cell

The isolated human microcells and targeted mouse cells (cells including a chromosome in which loxm2/66 (first RRS) and lox71 (second RRS) are inserted at both ends of the variable region of the mouse IgH locus, respectively) to be used as recipient cells are prepared. The targeted mouse cells are treated with TryLE, followed by centrifugation. The centrifuged targeted mouse cells are washed with 1xDPBS, and a cell count is calculated using a hemacytometer. The fusion of the human microcells and the targeted mouse cells is performed using an HVJ-E protein (Cosmo Bio Co., Ltd., Tokyo, Japan) by a suspension method. The calculated ratio of the human microcells to the targeted mouse cells is 1:4. Each of the prepared human microcells and targeted mouse cells are washed with 500 µl of a cold 1x cell fusion buffer. Centrifugation is performed with solutions of the human microcells and targeted mouse cells contained in the buffer at 300g and 4 °C for 5 minutes. 25 µl of a 1x cell fusion buffer is added per 2x10⁵ of targeted mouse cells, and the same volume of the 1x cell fusion buffer is added to the human microcells. The targeted mouse cells and the human microcells are mixed together, and 5 to 10 µl of the HVJ-E protein is added thereto, and then the cells are left on ice for 5 minutes. The mixture is left in a 37 °C water bath for 15 minutes. Here, the mixture is tapped every 5 minutes. After the cell fusion of the human microcells and the targeted mouse cells is completed, the remaining HVJ-E protein is removed by centrifugation at 300g for 5 minutes. The fused cells are put into a dish containing a targeted mouse cell culture medium, and then incubated for 48 hours in an incubator maintained at 5% CO₂, 95% humidity and 37 °C. The produced fusion cell is a cell including a targeted human chromosome (chromosome including the variable region of a human IgH locus into which lox66 (third RRS) and loxm2/71 (fourth RRS) were inserted) and a targeted mouse chromosome (chromosome including the variable region of the mouse IgH locus into which loxm2/66 (first RRS) and lox71 (second RRS) were inserted).

### Example 3-5. Production of cell including artificial recombinant chromosome using fusion cell

Human microcells and targeted mouse cells are fused, and the fusion cells are stabilized for 48 hours. 100 µl of FBS and antibiotic-free Opti-MEM is added to 1x10⁶ of the fusion cells. Here, 10 µg of a pCMV-Cre vector (System Biosciences, LLC, Palo Alto, CA, USA) is added to perform transfection at 125V and 5 ms with dual pulses. 300 µl of a 2i medium is added and mixed well with the cells, and the cells are transferred to a 100 mm dish and incubated for 48 hours in an incubator maintained at 5% CO₂, 95% humidity and 37 °C.

To confirm whether an artificial recombinant chromosome is produced in the Cre recombinase-treated fusion cells, the Cre recombinase-treated fusion cells are treated with antibiotics (Puromycin, G418 and hygromycin). In the Cre recombinase-treated fusion cells, the recombination between a targeted human chromosome (chromosome including the variable region of the human IgH locus into which lox66 (third RRS) and loxm2/71 (fourth RRS) were inserted) and a targeted mouse chromosome (chromosome including the variable region of the mouse IgH locus into which loxm2/66 (first RRS) and lox71 (second RRS) were inserted) is induced by the Cre recombinase. The first RRS in the targeted mouse chromosome is paired with the fourth RRS in the targeted human chromosome, and the second RRS in the targeted mouse chromosome is paired with the third RRS in the targeted human chromosome. The RRS pairings are recognized by the Cre recombinase to induce the recombination.

As a result, a first artificial recombinant chromosome in which the variable region of the mouse IgH locus of the targeted mouse chromosome was replaced with a human IgH variable region and a second artificial recombinant chromosome in which the variable region of the human IgH locus of the targeted human chromosome was replaced with a mouse IgH Variable region are produced. In the first artificial recombinant chromosome, a part excluding the variable region of the human IgH locus has a mouse gene (e.g., the constant region of the mouse IgH locus is a mouse gene). In the second artificial recombinant chromosome, a part excluding the variable region of the mouse IgH locus has a human gene (e.g., the constant region of the human IgH locus is a human gene).

After the antibiotic (Puromycin, G418 and hygromycin) treatment, surviving cells are obtained. The obtained cells are cells including the first artificial recombinant chromosome and the second artificial recombinant chromosome, and cells not including the targeted human chromosome and the targeted mouse chromosome.

The produced first artificial recombinant chromosome may include a fifth RRS and a sixth RRS. The fifth RRS and the sixth RRS are RRSs produced by the recombination between the first RRS and the fourth RRS and the recombination between the second RRS and the third RRS. In addition, the produced second artificial recombinant chromosome may include a seventh RRS and an eighth RRS. The seventh RRS and the eighth RRS are RRSs produced by the recombination between the first RRS and the fourth RRS and the recombination between the second RRS and the third RRS.

The RRS (the fifth RRS, the sixth RRS, the seventh RRS and the eighth RRS), the puroΔTK gene, the neomycin-resistant gene (NeoR), the hygromycin-resistant gene and the FRT included in the first artificial recombinant chromosome and the second artificial recombinant chromosome are removed by treating the obtained cells including the first artificial recombinant chromosome and the second artificial recombinant chromosome with piggyBac transposase. Here, cells including artificial recombinant chromosomes from which RRSs (the fifth RRS, the sixth RRS, the seventh RRS and the eighth RRS), the puroΔTK gene, the neomycin-resistant gene (NeoR), the zeocin-resistant gene and the FRT are removed are selected by treatment with fialuridine (FIAU).

The artificial recombinant chromosome may be recombined in various ways according to the position, direction and pairing of RRSs. To produce an artificial recombinant chromosome of interest, the artificial recombinant chromosome can be produced by modifying the design of a DNA donor as described above.

### Example 3-6. Production of transgenic animal using cell including artificial recombinant chromosome

To produce a transgenic animal using a cell including an artificial recombinant chromosome, the cell including the artificial recombinant chromosome obtained in Example 3-5 is treated with FIAU. The cells obtained after the treatment are implanted in a blastula through blastocyst injection, thereby producing a chimeric blastocyst. The produced chimeric blastocyst is implanted in the uterus of a surrogate, thereby generating a mouse offspring. The produced mouse offspring is a chimeric-transgenic mouse, and a heterozygous transgenic mouse or homozygous transgenic mouse is produced by breeding of the chimeric-transgenic mice.

In the produced transgenic mouse, the variable region of the IgH locus on the genome may be humanized, and the transgenic mouse may be used in production of a humanized antibody and/or fully human antibody.

Here, the transgenic mouse can be produced by various methods other than blastocyst injection.

Although the embodiments have been described with reference to the limited embodiments and the drawings as described above, various modifications and alternations are possible to those of ordinary skill in the art. For example, appropriate results may be achieved even if the described techniques are performed in a different order from the above-described methods, or replaced or substituted with other constituent elements or equivalents. Therefore, other embodiments, examples and equivalents to the claims are within the scope of the claims described below.

### [Sequence Listing Free-text]

SEQ ID NOs: 1 to 22 are primer sequences, SEQ ID NOs: 23 to 31 are RRS sequences, and SEQ ID NOs: 32 and 33 are the amino acid sequences of a recombinase.

## Claims

1. A method for producing a cell including one or more artificial recombinant chromosomes, the method comprising
i) preparing a first targeted cell and a second targeted cell,
wherein the first targeted cell comprises a first targeted chromosome, and the second targeted cell comprises a second targeted chromosome,
herein, the first targeted chromosome includes a first part, a first recombinase recognition sequence (a first RRS) and a first fragment, wherein the first RRS is located between the first part and the first fragment,
herein, the second targeted chromosome includes a second part, a second recombinase recognition sequence (a second RRS) and a second fragment, wherein the second RRS is located between the second part and the second fragment;
ii) producing one or more microcells using the second targeted cell,
wherein the one or more microcells comprise the second targeted chromosome or a fragment thereof,
herein, the fragment of the second targeted chromosome includes the second RRS and the second fragment;
iii) producing a fusion cell using the first targeted cell and the one or more microcells,
wherein the fusion cell comprises the first targeted chromosome and the second targeted chromosome, or the first targeted chromosome and the fragment of the second targeted chromosome; and
iv) producing a cell including an artificial recombinant chromosome by treating the fusion cell with a site specific recombinase (SSR),
wherein the SSR induces a recombination by recognizing a pairing of the first RRS present in the first targeted chromosome and the second RRS present in the second targeted chromosome,
herein, the first fragment present in the first targeted chromosome is exchanged for the second fragment present in the second targeted chromosome by the recombination,
thereby, a first artificial recombinant chromosome with the first part and the second fragment is produced.

2. The method of claim 1,
wherein the cell including one or more artificial recombinant chromosomes further comprises a second artificial recombinant chromosome,
herein, the second artificial recombinant chromosome includes the second part and the first fragment.

3. The method of claim 1,
wherein the first part includes one of telomere ends located on both sides of the first targeted chromosome.

4. The method of claim 3,
wherein the first fragment includes the other one of telomere ends located on both sides of the first targeted chromosome.

5. The method of claim 1,
wherein the second part includes one of telomere ends located on both sides of the second targeted chromosome.

6. The method of claim 5,
wherein the second fragment includes the other one of telomere ends located on both sides of the second targeted chromosome.

7. The method of claim 1,
wherein the first RRS is one selected from a loxP and a loxP variant,
wherein the second RRS is one selected from a loxP and a loxP variant,
here, the first RRS is capable of pairing with the second RRS.

8. The method of claim 7,
wherein the SSR is a Cre recombinase,
herein, the SSR is capable of recognizing the first RRS and the second RRS.

9. The method of claim 1,
wherein the first RRS is one selected from FRT, attP, attB, ITR and variants thereof,
wherein the second RRS is one selected from FRT, attP, attB, ITR and variants thereof,
herein, the first RRS is capable of pairing with the second RRS.

10. The method of claim 9,
wherein the SSR is one selected from a flippase (FLP), an integrase and a transposase
herein, the SSR is capable of recognizing the first RRS and the second RRS.

11. The method of claim 1,
wherein the cell including one or more artificial recombinant chromosomes can undergo mitosis or meiosis.

12. The method of claim 1,
wherein the cell including the first artificial recombinant chromosome does not include the second targeted chromosome.

13. A method for producing a cell including one or more artificial recombinant chromosomes, the method comprising
i) preparing a first targeted cell and a second targeted cell,
wherein the first targeted cell comprises a first targeted chromosome, and the second targeted cell comprises a second targeted chromosome,
wherein the first targeted chromosome includes a first part, a first recombinase recognition sequence (a first RRS), a first fragment, a second recombinase recognition sequence (a second RRS) and a second part,
herein, the first part includes one of telomere ends located on both sides of the first targeted chromosome, and the second part includes the other one of telomere ends located on both sides of the first targeted chromosome,
herein, the first fragment is located between the first RRS and the second RRS,
wherein the second targeted chromosome includes a third part, a third recombinase recognition sequence (a third RRS), a second fragment, a fourth recombinase recognition sequence (a fourth RRS) and a fourth part,
herein, the third part includes one of telomere ends located on both sides of the second targeted chromosome, and the fourth part includes the other one of telomere ends located on both sides of the second targeted chromosome,
herein, the second fragment is located between the third RRS and the fourth RRS;
ii) producing one or more microcells using the second targeted cell,
wherein the one or more microcells comprise the second targeted chromosome or a fragment thereof,
herein, the fragment of the second targeted chromosome includes the third RRS, the second fragment and the fourth RRS;
iii) producing a fusion cell using the first targeted cell and the one or more microcells,
wherein the fusion cell comprises the first targeted chromosome and the second targeted chromosome, or the first targeted chromosome and the fragment of the second targeted chromosome; and
iv) producing a cell including an artificial recombinant chromosome by treating the fusion cell with site specific recombinase (SSR),
wherein the SSR induces a recombination by recognizing a pairing of the first RRS present in the first targeted chromosome and the third RRS present in the second targeted chromosome, and a pairing of the second RRS present in the first targeted chromosome and the fourth RRS present in the second targeted chromosome,
herein, the first fragment present in the first targeted chromosome is exchanged for the second fragment present in the second targeted chromosome by the recombination,
thereby, a first artificial recombinant chromosome including the first part, the second fragment and the second part is produced.

14. The method of claim 13,
wherein the cell including one or more artificial recombinant chromosomes further comprises a second artificial recombinant chromosome,
herein, the second artificial recombinant chromosome includes the third part, the first fragment and the fourth part.

15. The method of claim 13,
wherein the first part includes a centromere of the first targeted chromosome.

16. The method of claim 13,
wherein the third part includes a centromere of the second targeted chromosome.

17. The method of claim 13,
wherein the first fragment includes a centromere of the first targeted chromosome,
wherein the second fragment includes a centromere of the second targeted chromosome.

18. The method of claim 13,
wherein the first RRS is one selected from a loxP and a loxP variant,
wherein the third RRS is one selected from a loxP and a loxP variant,
herein, the first RRS is capable of pairing with the third RRS.

19. The method of claim 13,
wherein the second RRS is one selected from a loxP and a loxP variant,
wherein the fourth RRS is one selected from a loxP and a loxP variant,
herein, the second RRS is capable of pairing with the fourth RRS.

20. The method of claim 18 or claim 19,
wherein the SSR is a Cre recombinase.

21. The method of claim 13,
wherein the first RRS is one selected from FRT, attP, attB, ITR and variants thereof,
wherein the third RRS is one selected from FRT, attP, attB, ITR and variants thereof,
herein, the first RRS is capable of pairing with the third RRS.

22. The method of claim 13,
wherein the second RRS is one selected from FRT, attP, attB, ITR and variants thereof,
wherein the fourth RRS is one selected from FRT, attP, attB, ITR and variants thereof,
herein, the second RRS is capable of pairing with the fourth RRS.

23. The method of claim 21 or claim 22,
wherein the SSR is one selected from a flippase (FLP), an integrase and a transposase.

24. The method of claim 13,
wherein the cell including one or more artificial recombinant chromosome can undergo mitosis or meiosis.

25. The method of claim 13,
wherein the cell including one or more artificial recombinant chromosome expresses a protein encoded by a gene present in the second fragment.

26. A method for making a transgenic non-human animal using a cell including one or more artificial recombinant chromosome, the method comprising
i) preparing a first targeted cell and a second targeted cell,
wherein the first targeted cell is an embryonic stem cell,
wherein the first targeted cell comprises a first targeted chromosome, and the second targeted cell comprises a second targeted chromosome,
herein, the first targeted chromosome includes a first part, a first recombinase recognition sequence (a first RRS) and a first fragment, and said first RRS is located between the first part and the first fragment,
herein, the second targeted chromosome includes a second part, a second recombinase recognition sequence (a second RRS) and a second fragment, and said second RRS is located between the second part and the second fragment;
ii) producing one or more microcells using the second targeted cell,
wherein the one or more microcells comprise the second targeted chromosome or a fragment thereof,
herein, the fragment of the second targeted chromosome includes the second RRS and the second fragment;
iii) producing a fusion cell using the first targeted cell and the one or more microcells,
wherein the fusion cell comprises the first targeted chromosome and the second targeted chromosome, or the first targeted chromosome and the fragment of the second targeted chromosome;
iv) producing a cell including an artificial recombinant chromosome by treating the fusion cell with a site specific recombinase (SSR),
wherein, the SSR induces a recombination by recognizing a pairing of the first RRS present in the first targeted chromosome and the second RRS present in the second targeted chromosome,
herein, the first fragment present in the first targeted chromosome is exchanged for the second fragment present in the second targeted chromosome,
thereby, a first artificial recombinant chromosome including the first part and the second fragment is produced; and
v) implanting a chimeric blastocyst comprising the first artificial recombinant chromosome in a surrogate mother's uterus to produce an offspring,
herein, the chimeric blastocyst is produced by injecting the cell including the first artificial chromosome into a blastocyst.

27. A method for making a transgenic non-human animal using a cell including one or more artificial recombinant chromosome, the method comprising
i) preparing a first targeted cell and a second target cell,
wherein the first targeted cell is an embryonic stem cell,
wherein the first targeted cell comprises a first targeted chromosome, and the second targeted cell comprises a second targeted chromosome,
wherein the first targeted chromosome includes a first part, a first recombinase recognition sequence (a first RRS), a first fragment, a second recombinase recognition sequence (a second RRS) and a second part,
herein, the first part includes one of telomere ends located on both sides of the first targeted chromosome, and the second part includes the other one of telomere ends located on both sides of the first targeted chromosome,
herein, the first fragment is located between the first RRS and the second RRS,
wherein the second targeted chromosome includes a third part, a third recombinase recognition sequence (a third RRS), a second fragment, a fourth recombinase recognition sequence (a fourth RRS) and a fourth part,
herein, the third part includes one of telomere ends located on both sides of the second targeted chromosome, and the fourth part includes the other one of the telomere ends located on both sides of the second targeted chromosome,
herein, the second fragment is located between the third RRS and the fourth RRS;
ii) producing one or more microcells using the second targeted cell,
wherein the one or more microcells comprises the second targeted chromosome or a fragment thereof,
herein, the fragment of the second targeted chromosome includes the third RRS, the second fragment and the fourth RRS;
iii) producing a fusion cell using the first targeted cell and the one or more microcells,
wherein the fusion cell comprises the first targeted chromosome and the second targeted chromosome, or the first targeted chromosome and the fragment of the second targeted chromosome;
iv) producing a cell including an artificial recombinant chromosome by treating the fusion cell with a site specific recombinase (SSR),
wherein the SSR induces a recombination by recognizing a pairing of the first RRS present in the first targeted chromosome and the third RRS present in the second targeted chromosome, and a pairing of the second RRS present in the first targeted chromosome and the fourth RRS present in the second targeted chromosome,
herein, the first fragment present in the first targeted chromosome is exchanged for the second fragment present in the second targeted chromosome,
thereby, a first artificial recombinant chromosome including the first part, the second fragment and the second part is produced; and
v) implanting a chimeric blastocyst comprising the first artificial recombinant chromosome in a surrogate mother's uterus to produce an offspring,
herein, the chimeric blastocyst is produced by injecting the cell including the first artificial recombinant chromosome into a blastocyst.


<110>	Humab Co., Ltd.

<120>	Artificial recombinant chromosome and the use thereof

<130>	OPP19-086-PCT

<150>	US 62/833489
<151>	2019-04-12

<150>	KR 10-2019-0042840
<151>	2019-04-12

<160>	33

<170>	KoPatentIn 3.0

<210>	1
<211>	34
<212>	DNA
<213>	Artificial Sequence

<220>
<223>	Primer


<400>	1
gaattcggct gtggaatgtg tgtcagttag ggtg	34


<210>	2
<211>	77
<212>	DNA
<213>	Artificial Sequence

<220>
<223>	Primer


<400>	2
cttatcatgt ctgtataccg tcgcgccacc ataacttcgt atagcataca ttatacgaag	60

ttatcggtcg acgtcgg	77


<210>	3
<211>	20
<212>	DNA
<213>	Artificial Sequence

<220>
<223>	Primer


<400>	3
ccgacgtcga ccgataactt	20


<210>	4
<211>	32
<212>	DNA
<213>	Artificial Sequence

<220>

<223>    Primer

<400>    4
acgcgtcgac aggatttgga cctgagcata ct                                    32

<210>    5
<211>    31
<212>    DNA
<213>    Artificial Sequence

<220>
<223>    Primer

<400>    5
ccgctcgagg aggccaagag aggctaaagc c                                     31

<210>    6
<211>    31
<212>    DNA
<213>    Artificial Sequence

<220>
<223>    Primer

<400>    6
cgcggatccc attctcccat ctccaattta t                                     31

<210>    7
<211>    29
<212>    DNA
<213>    Artificial Sequence

<220>
<223>    Primer

<400>    7
ggaattcttt tgtaacccct agacagatg                                        29

<210>    8
<211>    23
<212>    DNA
<213>    Artificial Sequence

<220>
<223>    Primer

<400>    8
aagcttccgc catgttgaca ttg                                              23

<210>    9
<211>    16
<212>    DNA
<213>    Artificial Sequence

<220>
<223>    Primer

<400>    9
cggccgccct atagtg                                                    16

<210>    10
<211>    25
<212>    DNA
<213>    Artificial Sequence

<220>
<223>    Primer

<400>    10
agatggagag cgacgagagc ggcct                                          25

<210>    11
<211>    32
<212>    DNA
<213>    Artificial Sequence

<220>
<223>    Primer

<400>    11
acgcgtcgac tgcgtgagat cttttcttgg gg                                  32

<210>    12
<211>    31
<212>    DNA
<213>    Artificial Sequence

<220>
<223>    Primer

<400>    12
ccgctcgagt ccacacaccc aagtcattcg a                                   31

<210>    13
<211>    31
<212>    DNA
<213>    Artificial Sequence

<220>
<223>    Primer

<400>    13
cgcggatccc tgaagccaac caagtttagg a                                   31

<210>    14
<211>    31

<212>    DNA
<213>    Artificial Sequence

<220>
<223>    Primer


<400>    14
cccaagcttc acatggtgaa cccaaacact c                                    31


<210>    15
<211>    28
<212>    DNA
<213>    Artificial Sequence

<220>
<223>    Primer


<400>    15
atcctcgagg acattgatta ttgactag                                        28


<210>    16
<211>    25
<212>    DNA
<213>    Artificial Sequence

<220>
<223>    Primer


<400>    16
attggtaccc tcggccgccc tatag                                           25


<210>    17
<211>    73
<212>    DNA
<213>    Artificial Sequence

<220>
<223>    Primer


<400>    17
ataggtacct accgttcgta tatggtttct tatacgaagt tatgaattcc accatgaaaa     60

agcctgaact cac                                                        73


<210>    18
<211>    64
<212>    DNA
<213>    Artificial Sequence

<220>
<223>    Primer


<400>    18
atcgtcgact accgttcgta taatgtatgc tatacgaagt tatggatcct aagatacatt     60

gatg                                                                    64

<210>    19
<211>    74
<212>    DNA
<213>    Artificial Sequence

<220>
<223>    Primer

<400>    19
attctcgaga taacttcgta taatgtatgc tatacgaacg gtaatcgatc cccagcatgc      60

ctgctattgt cttc                                                         74

<210>    20
<211>    76
<212>    DNA
<213>    Artificial Sequence

<220>
<223>    Primer

<400>    20
ctcaagctta taacttcgta tatggtttct tatacgaacg gtacttaagc accatgggga      60

ccgagtacaa gcccac                                                       76

<210>    21
<211>    28
<212>    DNA
<213>    Artificial Sequence

<220>
<223>    Primer

<400>    21
cgcaagcttg tgtgtcagtt agggtgtg                                          28

<210>    22
<211>    30
<212>    DNA
<213>    Artificial Sequence

<220>
<223>    Primer

<400>    22
atcgtcgact aagatacatt gatgagtttg                                        30

<210>    23
<211>    34
<212>    DNA

<213>    Artificial Sequence

<220>
<223>    loxp variant


<400>    23
taccgttcgt atatggtttc ttatacgaag ttat                                    34


<210>    24
<211>    34
<212>    DNA
<213>    Artificial Sequence

<220>
<223>    loxp variant


<400>    24
ataacttcgt atatggtttc ttatacgaac ggta                                    34


<210>    25
<211>    34
<212>    DNA
<213>    Artificial Sequence

<220>
<223>    loxp variant


<400>    25
taccgttcgt atagcataca ttatacgaag ttat                                    34


<210>    26
<211>    34
<212>    DNA
<213>    Artificial Sequence

<220>
<223>    loxp variant


<400>    26
ataacttcgt atagcataca ttatacgaac ggta                                    34


<210>    27
<211>    48
<212>    DNA
<213>    Artificial Sequence

<220>
<223>    recombinase recognition site


<400>    27
gaagttccta tactttctag agaataggaa cttcggaata ggaacttc                     48


<210>    28

<211>    100
<212>    DNA
<213>    Artificial Sequence

<220>
<223>    recombinase recognition site


<400>    28
cccaggtcag aagcggtttt cgggagtagt gccccaactg gggtaacctt tgagttctct        60

cagttggggg cgtagggtcg ccgacatgac acaaggggtt                              100


<210>    29
<211>    70
<212>    DNA
<213>    Artificial Sequence

<220>
<223>    recombinase recognition site


<400>    29
ctcgaagccg cggtgcgggt gccagggcgt gcccttgggc tccccgggcg cgtactccac        60

ctcacccatc                                                               70


<210>    30
<211>    63
<212>    DNA
<213>    Artificial Sequence

<220>
<223>    recombinase recognition site


<400>    30
ccctagaaag ataatcatat tgtgacgtac gttaaagata atcatgcgta aaattgacgc        60

atg                                                                      63


<210>    31
<211>    35
<212>    DNA
<213>    Artificial Sequence

<220>
<223>    recombinase recognition site


<400>    31
catgcgtcaa ttttacgcag actatctttc taggg                                   35


<210>    32
<211>    342
<212>    PRT
<213>    Artificial Sequence

<220>

<223>    Cre recombinase

<400>    32
Ser Asn Leu Leu Thr Val His Gln Asn Leu Pro Ala Leu Pro Val Asp
1               5                   10                  15

Ala Thr Ser Asp Glu Val Arg Lys Asn Leu Met Asp Met Phe Arg Asp
            20                  25                  30

Arg Gln Ala Phe Ser Glu His Thr Trp Lys Met Leu Leu Ser Val Cys
        35                  40                  45

Arg Ser Trp Ala Ala Trp Cys Lys Leu Asn Asn Arg Lys Trp Phe Pro
    50                  55                  60

Ala Glu Pro Glu Asp Val Arg Asp Tyr Leu Leu Tyr Leu Gln Ala Arg
65                  70                  75                  80

Gly Leu Ala Val Lys Thr Ile Gln Gln His Leu Gly Gln Leu Asn Met
                85                  90                  95

Leu His Arg Arg Ser Gly Leu Pro Arg Pro Ser Asp Ser Asn Ala Val
            100                 105                 110

Ser Leu Val Met Arg Arg Ile Arg Lys Glu Asn Val Asp Ala Gly Glu
        115                 120                 125

Arg Ala Lys Gln Ala Leu Ala Phe Glu Arg Thr Asp Phe Asp Gln Val
    130                 135                 140

Arg Ser Leu Met Glu Asn Ser Asp Arg Cys Gln Asp Ile Arg Asn Leu
145                 150                 155                 160

Ala Phe Leu Gly Ile Ala Tyr Asn Thr Leu Leu Arg Ile Ala Glu Ile
                165                 170                 175

Ala Arg Ile Arg Val Lys Asp Ile Ser Arg Thr Asp Gly Gly Arg Met
            180                 185                 190

Leu Ile His Ile Gly Arg Thr Lys Thr Leu Val Ser Thr Ala Gly Val
        195                 200                 205

Glu Lys Ala Leu Ser Leu Gly Val Thr Lys Leu Val Glu Arg Trp Ile
    210                 215                 220

Ser Val Ser Gly Val Ala Asp Asp Pro Asn Asn Tyr Leu Phe Cys Arg
225                 230                 235                 240

Val Arg Lys Asn Gly Val Ala Ala Pro Ser Ala Thr Ser Gln Leu Ser
                245                 250                 255

Thr Arg Ala Leu Glu Gly Ile Phe Glu Ala Thr His Arg Leu Ile Tyr
            260                 265                 270

Gly Ala Lys Asp Asp Ser Gly Gln Arg Tyr Leu Ala Trp Ser Gly His
        275                 280                 285

Ser Ala Arg Val Gly Ala Ala Arg Asp Met Ala Arg Ala Gly Val Ser
    290                 295                 300

Ile Pro Glu Ile Met Gln Ala Gly Gly Trp Thr Asn Val Asn Ile Val
305                 310                 315                 320

Met Asn Tyr Ile Arg Asn Leu Asp Ser Glu Thr Gly Ala Met Val Arg
                325                 330                 335

Leu Leu Glu Asp Gly Asp
            340

<210>   33
<211>   594
<212>   PRT
<213>   Artificial Sequence

<220>
<223>   Piggy Bac Transposase

<400>   33
Met Gly Ser Ser Leu Asp Asp Glu His Ile Leu Ser Ala Leu Leu Gln
1               5                   10                  15

Ser Asp Asp Glu Leu Val Gly Glu Asp Ser Asp Ser Glu Ile Ser Asp
            20                  25                  30

His Val Ser Glu Asp Asp Val Gln Ser Asp Thr Glu Glu Ala Phe Ile
        35                  40                  45

Asp Glu Val His Glu Val Gln Pro Thr Ser Ser Gly Ser Glu Ile Leu
    50                  55                  60

Asp Glu Gln Asn Val Ile Glu Gln Pro Gly Ser Ser Leu Ala Ser Asn
65                  70                  75                  80

Arg Ile Leu Thr Leu Pro Gln Arg Thr Ile Arg Gly Lys Asn Lys His
                85                  90                  95

Cys Trp Ser Thr Ser Lys Ser Thr Arg Arg Ser Arg Val Ser Ala Leu
            100                 105                 110

Asn Ile Val Arg Ser Gln Arg Gly Pro Thr Arg Met Cys Arg Asn Ile
        115                 120                 125

Tyr Asp Pro Leu Leu Cys Phe Lys Leu Phe Phe Thr Asp Glu Ile Ile
    130                 135                 140

Ser Glu Ile Val Lys Trp Thr Asn Ala Glu Ile Ser Leu Lys Arg Arg
145                 150                 155                 160

Glu Ser Met Thr Gly Ala Thr Phe Arg Asp Thr Asn Glu Asp Glu Ile
                165                 170                 175

Tyr Ala Phe Phe Gly Ile Leu Val Met Thr Ala Val Arg Lys Asp Asn
            180                 185                 190

His Met Ser Thr Asp Asp Leu Phe Asp Arg Ser Leu Ser Met Val Tyr
        195                 200                 205

Val Ser Val Met Ser Arg Asp Arg Phe Asp Phe Leu Ile Arg Cys Leu
    210                 215                 220

Arg Met Asp Asp Lys Ser Ile Arg Pro Thr Leu Arg Glu Asn Asp Val
225                 230                 235                 240

Phe Thr Pro Val Arg Lys Ile Trp Asp Leu Phe Ile His Gln Cys Ile
                245                 250                 255

Gln Asn Tyr Thr Pro Gly Ala His Leu Thr Ile Asp Glu Gln Leu Leu
            260                 265                 270

Gly Phe Arg Gly Arg Cys Pro Phe Arg Met Tyr Ile Pro Asn Lys Pro
        275                 280                 285

Ser Lys Tyr Gly Ile Lys Ile Leu Met Met Cys Asp Ser Gly Thr Lys
    290                 295                 300

Tyr Met Ile Asn Gly Met Pro Tyr Leu Gly Arg Gly Thr Gln Thr Asn
305                 310                 315                 320

Gly Val Pro Leu Gly Glu Tyr Tyr Val Lys Glu Leu Ser Lys Pro Val
                325                 330                 335

His Gly Ser Cys Arg Asn Ile Thr Cys Asp Asn Trp Phe Thr Ser Ile
            340                 345                 350

Pro Leu Ala Lys Asn Leu Leu Gln Glu Pro Tyr Lys Leu Thr Ile Val
        355                 360                 365

Gly Thr Val Arg Ser Asn Lys Arg Glu Ile Pro Glu Val Leu Lys Asn
    370                 375                 380

Ser Arg Ser Arg Pro Val Gly Thr Ser Met Phe Cys Phe Asp Gly Pro
385                 390                 395                 400

Leu Thr Leu Val Ser Tyr Lys Pro Lys Pro Ala Lys Met Val Tyr Leu
                405                 410                 415

Leu Ser Ser Cys Asp Glu Asp Ala Ser Ile Asn Glu Ser Thr Gly Lys
            420                 425                 430

Pro Gln Met Val Met Tyr Tyr Asn Gln Thr Lys Gly Gly Val Asp Thr
        435                 440                 445

Leu Asp Gln Met Cys Ser Val Met Thr Cys Ser Arg Lys Thr Asn Arg
    450                 455                 460

Trp Pro Met Ala Leu Leu Tyr Gly Met Ile Asn Ile Ala Cys Ile Asn
465                 470                 475                 480

Ser Phe Ile Ile Tyr Ser His Asn Val Ser Ser Lys Gly Glu Lys Val
                485                 490                 495

Gln Ser Arg Lys Lys Phe Met Arg Asn Leu Tyr Met Ser Leu Thr Ser
            500                 505                 510

Ser Phe Met Arg Lys Arg Leu Glu Ala Pro Thr Leu Lys Arg Tyr Leu
        515                 520                 525

Arg Asp Asn Ile Ser Asn Ile Leu Pro Asn Glu Val Pro Gly Thr Ser
    530                 535                 540

Asp Asp Ser Thr Glu Glu Pro Val Met Lys Lys Arg Thr Tyr Cys Thr
545                 550                 555                 560

Tyr Cys Pro Ser Lys Ile Arg Arg Lys Ala Asn Ala Ser Cys Lys Lys
                565                 570                 575

Cys Lys Lys Val Ile Cys Arg Glu His Asn Ile Asp Met Cys Gln Ser
            580                 585                 590

Cys Phe